# EUROPEAN PATENT SPECIFICATION

(11) **EP 2 531 614 B1**
(45) Date of publication and mention of the grant of the patent: **18.07.2018**
(21) Application number: 11706065.7
(22) Date of filing: 07.02.2011
(51) Int. Cl.: C12Q 1/6883

(54) **TRANSCRIPTIONAL PROFILING AND BIOMARKER-BASED METHODS FOR IDENTIFYING AND EVALUATING AGENTS FOR ANTIOXIDANT EFFICACY IN COSMETIC SKIN CARE FORMULATIONS**
TRANSKRIPTIONSPROFILIERUNG UND BIOMARKER BASIERTE VERFAHREN ZUR IDENTIFIZIERUNG UND BEURTEILUNG DER ANTIOXIDANZEFFIZIENZ IN KOSMETISCHEN HAUTPLEGEMITTEL.
PROFILAGE TRANSCRIPTIONNEL ET PROCEDE BASE SUR DEs BIOMARQUEURS POUR L'IDENTIFICATION ET EVALUATION DE L'EFFICACITE DES ANTIOXYDANTS DANS DES PRODUITS COSMETIQUES POUR LE SOIN DE LA PEAU

(30) Priority: 05.02.2010 US 301758 P
(43) Date of publication of application: 12.12.2012
(73) Proprietor: The Procter & Gamble Company, Cincinnati, OH 45202 (US)
(72) Inventor: FINLAY, Deborah, Ruth, Cincinnati, OH 45224 (US); BINDER, Robert, Lloyd, Montgomery, OH 45242 (US); ROBINSON, Michael, Keith, West Chester, OH 45069 (US); OSBORNE, Rosemarie, Oxford, OH 45056 (US); MULLINS, Lisa, Ann, West Chester, OH 45069 (US)
(74) Representative: Sauvaître, Thibault Bruno
(86) International application number: PCT/US2011/023918
(87) International publication number: WO 2011/097572

(56) References cited:
- EP-A1- 2 113 242
- WO-A2-02/053773
- WO-A2-2008/065451
- WO-A2-2009/036204
- US-A1- 2006 148 732
- ANONYMOUS: "GeneChip TM Human Genome U95 Set Human Genome U133 Set GeneChip TM Human Genome U133 Plus 2.0 Array", INTERNET CITATION, 1 January 2009 (2009-01-01), page 1, XP007918296, Retrieved from the Internet: URL:https://www.affymetrix.com/analysis/ne taffx/showresults.affx [retrieved on 2011-04-14]
- "Affymetrix Human Genome U133 Plus 2.0 Array", GENE EXPRESSION OMNIBUS, 7 November 2003 (2003-11-07), XP002627319, [retrieved on 2003-11-07]
- "Genomics analysis of the reduced antioxidant capacity of aging skin", JOURNAL OF THE AMERICAN ACADEMY OF DERMATOLOGY, C.V. MOSBY, ST. LOUIS, MO, US, vol. 60, no. 3, 1 March 2009 (2009-03-01), page AB1, XP025963875, ISSN: 0190-9622, DOI: DOI:10.1016/J.JAAD.2008.11.028 [retrieved on 2009-02-24]
- LIU YANXIA ET AL: "A genomic screen for activators of the antioxidant response element", PROCEEDINGS OF THE NATIONAL ACADEMY OF SCIENCES OF THE UNITED STATES OF AMERICA, vol. 104, no. 12, March 2007 (2007-03), pages 5205-5210, XP002636400, ISSN: 0027-8424
- WONDRAK GEORG THOMAS ET AL: "Identification of small molecule Nrf2-activators targeting skin cell photo-oxidative stress", FREE RADICAL BIOLOGY & MEDICINE, vol. 43, no. Suppl. 1, 2007, page S127, XP002636401, 14TH ANNUAL MEETING OF THE SOCIETY-FOR-FREE-RADICAL-BIOLOGY-AND-MEDI CINE; WASHINGTON, DC, USA; NOVEMBER 14 -18, 2007 ISSN: 0891-5849
- WONDRAK G T ET AL: "Cinnamoyl-based Nrf2-activators targeting human skin cell photo-oxidative stress", FREE RADICAL BIOLOGY AND MEDICINE, ELSEVIER SCIENCE, US, vol. 45, no. 4, 15 August 2008 (2008-08-15), pages 385-395, XP022831891, ISSN: 0891-5849, DOI: DOI:10.1016/J.FREERADBIOMED.2008.04.023 [retrieved on 2008-04-26]
- PERRICONE ET AL: "Topical 5% alpha lipoic acid cream in the treatment of cutaneous rhytids", AESTHETIC SURGERY JOURNAL, MOSBY-YEAR BOOK, ST. LOUIS, MO, US, vol. 20, no. 3, 1 May 2000 (2000-05-01), pages 218-222, XP005701142, ISSN: 1090-820X, DOI: 10.1016/S1090-820X(00)70020-3
- PODDA MAURIZO ET AL: "Kinetic study of cutaneous and subcutaneous distribution following topical application of (7,8-14C)rac-alpha-lipoic acid onto hairless", BIOCHEMICAL PHARMACOLOGY, ELSEVIER, US, vol. 52, no. 4, 1 January 1996 (1996-01-01), pages 627-633, XP009100391, ISSN: 0006-2952, DOI: 10.1016/0006-2952(96)00337-1
- WANG Y J ET AL: "Dihydrolipoic acid inhibits tetrachlorohydroquinone-induced tumor promotion through prevention of oxidative damage", FOOD AND CHEMICAL TOXICOLOGY, PERGAMON, GB, vol. 46, no. 12, 1 December 2008 (2008-12-01), pages 3739-3748, XP026700792, ISSN: 0278-6915, DOI: 10.1016/J.FCT.2008.09.064 [retrieved on 2008-12-01]
- FARRIS P: "Idebenone, green tea, and Coffeeberry(R) extract: New and innovative antioxidants", DERMATOLOGIC THERAPY, MUNKSGAARD, COPENHAGEN, DK, vol. 20, no. 5, 1 January 2007 (2007-01-01), pages 322-329, XP009120548, ISSN: 1396-0296

### SEQUENCE LISTING

<110> Finlay, Deborah R
<120> TRANSCRIPTIONAL PROFILING AND BIOMARKER-BASED METHODS FOR IDENTIFYING AND EVALUATING AGENTS FOR ANTIOXIDANT EFFICACY IN COSMETIC SKIN CARE FORMULATIONS
<130> 11584P
<160> 39
<170> PatentIn version 3.5
<210> 1
   <211> 3702
   <212> DNA
   <213> Homo sapiens
<400> 1
   agtcctcccc cggcgcctcc gactggcagt gggactcagc gggcgtggag gtcgcggctg      60
   agcgagcgag ccctgggcga gtgaattgtg gctgtgggtt gacggtggag acaccccccg     120
   gagggaggcg gagggaaggg aggcgaggcc tgcacctgca tgcttcccgc ctcccactcc     180
   ccagcgcccc cggaccgtgc agttctctgc aggaccaggc catggagctc gaagtccggc     240
   gggtccgaca ggcgttcctg tccggccggt cgcgacctct gcggtttcgg ctgcagcagc     300
   tggaggccct gcggaggatg gtgcaggagc gcgagaagga tatcctgacg gccatcgccg     360
   ccgacctgtg caagagtgaa ttcaatgtgt acagtcagga agtcattact gtccttgggg     420
   aaattgattt tatgcttgag aatcttcctg aatgggttac tgctaaacca gttaagaaga     480
   acgtgctcac catgctggat gaggcctata ttcagccaca gcctctggga gtggtgctga     540
   taatcggagc ttggaattac cccttcgttc tcaccattca gccactgata ggagccatcg     600
   ctgcaggaaa tgctgtgatt ataaagcctt ctgaactgag tgaaaataca gccaagatct     660
   tggcaaagct tctccctcag tatttagacc aggatctcta tattgttatt aatggtggtg     720
   ttgaggaaac cacggagctc ctgaagcagc gatttgacca cattttctat acgggaaaca     780
   ctgcggttgg caaaattgtc atggaagctg ctgccaagca tctgacccct gtgactcttg     840
   aactgggagg gaaaagtcca tgttatattg ataaagattg tgacctggac attgtttgca     900
   gacgcataac ctggggaaaa tacatgaatt gtggccaaac ctgcattgca cccgactata     960
   ttctctgtga agcatccctc caaaatcaaa ttgtatggaa gattaaggaa acagtgaagg    1020
   aattttatgg agaaaatata aaagagtctc ctgattatga aaggatcatc aatcttcgtc    1080
   attttaagag gatactaagt ttgcttgaag gacaaaagat agcttttggt ggggagactg    1140
   atgaggccac acgctacata gccccaacag tacttaccga tgttgatcct aaaaccaagg    1200
   tgatgcaaga agaaattttt ggaccaattc ttccaatagt gcctgtgaaa aatgtagatg    1260
   aggccataaa tttcataaat gaacgtgaaa agcctctggc tctttatgta ttttcgcata    1320
   accataagct catcaaacgg atgattgatg agacatccag tggaggtgtc acaggcaatg    1380
   acgtcattat gcacttcacg ctcaactctt tcccatttgg aggagtgggt tccagtggga 1440
   tgggagctta tcacggaaaa catagttttg atactttttc tcatcagcgt ccctgtttat 1500
   taaaaagttt aaagagagaa ggtgctaaca aactcagata tcctcccaac agccagtcaa 1560
   aggtggattg gggaaaattt tttctcttga aacggttcaa caaagaaaaa ctcggtctcc 1620
   tgttgctcac tttcctgggt attgtagccg ctgtgcttgt caaggcagaa tattactgaa 1680
   gaatgatcct gttcaacctc ctagtgcctc tactgaatta ttcctctttt aaatggttaa 1740
   tgaaccaata atttttaaat cataccaaaa atagtaagaa aatatgcaaa cactctgtga 1800
   tcaaacttaa aagtcattgc cattcatcat taataaaagt tgccatttca actacgtccc 1860
   aacattccct aatagggtat tcagggaacc tgtcttaaat tgtgcttatc taaatcttgg 1920
   aactttgagc taggggagga gaatgtatta gactaaatac aaactgcggg gttgtaaggg 1980
   agtctcagaa cctcactgaa tccttcactc cagttaatgg cactgctcac ttcctgcctc 2040
   tgctgccacc atcactgtgt gaagctttca agagcttggt acttcccagg gctaccggca 2100
   gtcctctgta gtccagagag gtgagattag atcttcttgg ttccctgtga ggtttcaggc 2160
   actaaaactc tatgtgggga agggaggggt tactcctcct ccaatgggac tcaaggactt 2220
   gacctccagg agtaggcccc tggtcagaag tgccatctca ccagtggtct tcattcttcc 2280
   tcattcattc tttatcatcc tgtgttctgt ttagttgcaa caatctcttg tgactaatgt 2340
   cactcaaagc atcttgtaaa tcctagggct tcctggaagt tagttgccaa agtcatgcaa 2400
   gcatcacctg tcattcttgt gttggagtta tagaattcta catcttataa aacctaactg 2460
   gcatttaaaa aatactgtgg ccgggcgtgg tggctcatgc ctgtaatccc agcactttgg 2520
   gaggccgagg tgggaggatt gcttgagtcc aggaatttga gaccagcctg gacaacacag 2580
   tgagacctca tctctatcaa aaaataaaaa ttagctagat gtggtggcat gagcctgtgt 2640
   tcccagctgc ttaggaggct gaagcaggag gattgattga gcctgcgagg ccaaggctgc 2700
   agcaggctgt gattgcacca ctgcacttca gcttgggcaa cagagcaaga ccctgtctcc 2760
   gaaacaaata aaaaatactg taataaaagt acttataaac atactaatcc tctttcagga 2820
   ccctaaagtt gcaggttagt aggtcttcaa ggacaaatct gtaagtttct tatttctgta 2880
   gtgcaagtaa aatttcactt tttgaaacta tagagagatc cctttctgat tagcctacag 2940
   aacttaaagt gagggaacca tttcctctca cagacaaaga ggcctgggat attaggactt 3000
   tggggtttga gagcatcatg gggcagacag atggtggatg gtctggacaa gaagcgagta 3060
   agccactgcg gttggtcata ctgaagggaa ttgatggcaa gaggatcccc tgagcaagtc 3120
   agaagttact ctcatcagtc gttcatggtc acaacctgag gtactctgct gagtgggcaa 3180
   ggctgaagaa gaggcctgtg gaatgcagca ttacctgctg gacagagcag ggcaggcagt 3240
   tctatgcctt ggagctcctg actgcaggga ctctgtcccc acactcaaaa agactcagct 3300
   cactcaatga gagaatgtga tttactttat agaacgtata atcaactttg ttgaataatt     3360
   tgttctatta aggctgtcta aagtatgtga tgtcttcatc atagtatgaa gtgttgaaaa     3420
   ttaataacga gcctagttta ggaaaaagct gcttaaaact gtggctctaa gagagtaatc     3480
   ataaaatacc ttagataaaa ttgcactatg gaattttcat tgagtatgtt taaattattg     3540
   gcttgtctac taatacacat ctgcttcaaa atgaacatat ttcataaaat tggcatcaat     3600
   tttaatgacg ctcctggtat ggaacctcag atatacccta ttggagacaa tcctttgatc     3660
   ataaattctc cccaactata aatcatttta tgtctttatc at                        3702
<210> 2
   <211> 3823
   <212> DNA
   <213> Homo sapiens
<400> 2
   agtcctcccc cggcgcctcc gactggcagt gggactcagc gggcgtggag gtcgcggctg      60
   agcgagcgag ccctgggcga gtgaattgtg gctgtgggtt gacggtggag acaccccccg     120
   gagggaggcg gagggaaggg aggcgaggcc tgcacctgca tgcttcccgc ctcccactcc     180
   ccagcgcccc cggaccgtgc agttctctgc aggaccaggc catggagctc gaagtccggc     240
   gggtccgaca ggcgttcctg tccggccggt cgcgacctct gcggtttcgg ctgcagcagc     300
   tggaggccct gcggaggatg gtgcaggagc gcgagaagga tatcctgacg gccatcgccg     360
   ccgacctgtg caagagtgaa ttcaatgtgt acagtcagga agtcattact gtccttgggg     420
   aaattgattt tatgcttgag aatcttcctg aatgggttac tgctaaacca gttaagaaga     480
   acgtgctcac catgctggat gaggcctata ttcagccaca gcctctggga gtggtgctga     540
   taatcggagc ttggaattac cccttcgttc tcaccattca gccactgata ggagccatcg     600
   ctgcaggaaa tgctgtgatt ataaagcctt ctgaactgag tgaaaataca gccaagatct     660
   tggcaaagct tctccctcag tatttagacc aggatctcta tattgttatt aatggtggtg     720
   ttgaggaaac cacggagctc ctgaagcagc gatttgacca cattttctat acgggaaaca     780
   ctgcggttgg caaaattgtc atggaagctg ctgccaagca tctgacccct gtgactcttg     840
   aactgggagg gaaaagtcca tgttatattg ataaagattg tgacctggac attgtttgca     900
   gacgcataac ctggggaaaa tacatgaatt gtggccaaac ctgcattgca cccgactata     960
   ttctctgtga agcatccctc caaaatcaaa ttgtatggaa gattaaggaa acagtgaagg    1020
   aattttatgg agaaaatata aaagagtctc ctgattatga aaggatcatc aatcttcgtc    1080
   attttaagag gatactaagt ttgcttgaag gacaaaagat agcttttggt ggggagactg    1140
   atgaggccac acgctacata gccccaacag tacttaccga tgttgatcct aaaaccaagg    1200
   tgatgcaaga agaaattttt ggaccaattc ttccaatagt gcctgtgaaa aatgtagatg    1260
   aggccataaa tttcataaat gaacgtgaaa agcctctggc tctttatgta ttttcgcata    1320
   accataagct catcaaacgg atgattgatg agacatccag tggaggtgtc acaggcaatg     1380
   acgtcattat gcacttcacg ctcaactctt tcccatttgg aggagtgggt tccagtggga     1440
   tgggagctta tcacggaaaa catagttttg atactttttc tcatcagcgt ccctgtttat     1500
   taaaaagttt aaagagagaa ggtgctaaca aactcagata tcctcccaac agccagtcaa     1560
   aggtggattg ggaaaaattt tttctcttga aacggttcaa caaagaaaaa ctcggtctcc     1620
   tgttgctcac tttcctgggt attgtagccg ctgtgcttgt caagaaatac caagctgtgc     1680
   tgaggagaaa ggccctgttg atttttctgg tagttcacag actgcgttgg tccagtaagc     1740
   agagatgaac accagatttc aaaacccagc cctgtctgtt aagagtgagg cagaatatta     1800
   ctgaagaatg atcctgttca acctcctagt gcctctactg aattattcct cttttaaatg     1860
   gttaatgaac caataatttt taaatcatac caaaaatagt aagaaaatat gcaaacactc     1920
   tgtgatcaaa cttaaaagtc attgccattc atcattaata aaagttgcca tttcaactac     1980
   gtcccaacat tccctaatag ggtattcagg gaacctgtct taaattgtgc ttatctaaat     2040
   cttggaactt tgagctaggg gaggagaatg tattagacta aatacaaact gcggggttgt     2100
   aagggagtct cagaacctca ctgaatcctt cactccagtt aatggcactg ctcacttcct     2160
   gcctctgctg ccaccatcac tgtgtgaagc tttcaagagc ttggtacttc ccagggctac     2220
   cggcagtcct ctgtagtcca gagaggtgag attagatctt cttggttccc tgtgaggttt     2280
   caggcactaa aactctatgt ggggaaggga ggggttactc ctcctccaat gggactcaag     2340
   gacttgacct ccaggagtag gcccctggtc agaagtgcca tctcaccagt ggtcttcatt     2400
   cttcctcatt cattctttat catcctgtgt tctgtttagt tgcaacaatc tcttgtgact     2460
   aatgtcactc aaagcatctt gtaaatccta gggcttcctg gaagttagtt gccaaagtca     2520
   tgcaagcatc acctgtcatt cttgtgttgg agttatagaa ttctacatct tataaaacct     2580
   aactggcatt taaaaaatac tgtggccggg cgtggtggct catgcctgta atcccagcac     2640
   tttgggaggc cgaggtggga ggattgcttg agtccaggaa tttgagacca gcctggacaa     2700
   cacagtgaga cctcatctct atcaaaaaat aaaaattagc tagatgtggt ggcatgagcc     2760
   tgtgttccca gctgcttagg aggctgaagc aggaggattg attgagcctg cgaggccaag     2820
   gctgcagcag gctgtgattg caccactgca cttcagcttg ggcaacagag caagaccctg     2880
   tctccgaaac aaataaaaaa tactgtaata aaagtactta taaacatact aatcctcttt     2940
   caggacccta aagttgcagg ttagtaggtc ttcaaggaca aatctgtaag tttcttattt     3000
   ctgtagtgca agtaaaattt cactttttga aactatagag agatcccttt ctgattagcc     3060
   tacagaactt aaagtgaggg aaccatttcc tctcacagac aaagaggcct gggatattag     3120
   gactttgggg tttgagagca tcatggggca gacagatggt ggatggtctg gacaagaagc     3180
   gagtaagcca ctgcggttgg tcatactgaa gggaattgat ggcaagagga tcccctgagc     3240
   aagtcagaag ttactctcat cagtcgttca tggtcacaac ctgaggtact ctgctgagtg     3300
   ggcaaggctg aagaagaggc ctgtggaatg cagcattacc tgctggacag agcagggcag 3360
   gcagttctat gccttggagc tcctgactgc agggactctg tccccacact caaaaagact 3420
   cagctcactc aatgagagaa tgtgatttac tttatagaac gtataatcaa ctttgttgaa 3480
   taatttgttc tattaaggct gtctaaagta tgtgatgtct tcatcatagt atgaagtgtt 3540
   gaaaattaat aacgagccta gtttaggaaa aagctgctta aaactgtggc tctaagagag 3600
   taatcataaa ataccttaga taaaattgca ctatggaatt ttcattgagt atgtttaaat 3660
   tattggcttg tctactaata cacatctgct tcaaaatgaa catatttcat aaaattggca 3720
   tcaattttaa tgacgctcct ggtatggaac ctcagatata ccctattgga gacaatcctt 3780
   tgatcataaa ttctccccaa ctataaatca ttttatgtct tta 3823
<210> 3
   <211> 4949
   <212> DNA
   <213> Homo sapiens
<400> 3
   cgccccactc ggcgggtcgg tgccgccggg tcccaggtgc ccgctacttc ccagaacctc      60
   cgcctcccgc tccgggccct cgaaccagcg cggacaccac aatggaccgg gcgtccgagc     120
   tgctcttcta cgtgaacggc cgcaaggtga tagaaaaaaa tgtcgatcct gaaacaatgc     180
   tgttgcctta tttgaggaag aagcttcgac tcacaggaac taagtatggc tgtggaggag     240
   gaggctgtgg tgcttgtaca gtgatgatat cacgatacaa ccccatcacc aagaggataa     300
   ggcatcaccc agccaatgcc tgtctgattc ccatctgttc tctgtatggt gctgccgtca     360
   ccacagtaga aggcatagga agcacccaca ccagaattca tcctgttcag gagaggattg     420
   ccaagtgtca tggcacccag tgtggcttct gcacacctgg gatggtgatg tccatctaca     480
   cgctgctcag gaaccaccca gagcccactc tggatcagtt aactgatgcc cttggtggta     540
   acctgtgccg ttgcactgga tacaggccca taattgatgc atgcaagact ttctgtaaaa     600
   cttcgggctg ctgtcaaagt aaagaaaatg gggtttgctg tttggatcaa ggaatcaatg     660
   gattgccaga atttgaggaa ggaagtaaga caagtccaaa actcttcgca gaagaggagt     720
   ttctgccatt ggatccaacc caggaactga tatttcctcc tgagctaatg ataatggctg     780
   agaaacagtc gcaaaggacc agggtgtttg gcagtgagag aatgatgtgg ttttcccccg     840
   tgaccctgaa ggaactgctg gaatttaaat tcaagtatcc ccaggctcct gttatcatgg     900
   gaaacacctc tgtggggcct gaagtgaaat ttaaaggcgt ctttcaccca gttataattt     960
   ctcctgatag aattgaagaa ctgagtgttg taaaccatgc atataatgga ctcacccttg    1020
   gtgctggtct cagcctagcc caggtgaagg acattttggc tgatgtagtc cagaagcttc    1080
   cagaggagaa gacacagatg taccatgctc tcctgaagca tttgggaact ctggctgggt    1140
   cccagatcag gaacatggct tctttagggg gacacatcat tagcaggcat ccagattcag    1200
   atctgaatcc catcctggct gtgggtaact gtaccctcaa cttgctatca aaagaaggaa 1260
   aacgacagat tcctttaaat gagcaattcc tcagcaagtg ccctaatgca gatcttaagc 1320
   ctcaagaaat cttggtctca gtgaacatcc cctactcaag gaagtgggaa tttgtgtcag 1380
   ccttccgaca agcccagcga caggagaatg cgctagcgat agtcaattca ggaatgagag 1440
   tcttttttgg agaaggggat ggcattatta gagagttatg catctcatat ggaggcgttg 1500
   gtccagccac catctgtgcc aagaattcct gccagaaact cattggaagg cactggaacg 1560
   aacagatgct ggatatagcc tgcaggctta ttctgaatga agtctccctt ttgggctcgg 1620
   cgccaggtgg gaaagtggag ttcaagagga ctctcatcat cagcttcctc ttcaagttct 1680
   acctggaagt gtcacagatt ttgaaaaaga tggatccagt tcactatcct agccttgcag 1740
   acaagtatga aagtgcttta gaagatcttc attccaaaca tcactgcagt acattaaagt 1800
   accagaatat aggcccaaag cagcatcctg aagacccaat tggccacccc atcatgcatc 1860
   tgtctggtgt gaagcatgcc acgggggagg ccatctactg tgatgacatg cctctggtgg 1920
   accaggaact tttcttgact tttgtgacta gttcaagagc tcatgctaag attgtgtcta 1980
   ttgatctgtc agaagctctc agcatgcccg gtgtggtgga catcatgaca gcagaacatc 2040
   ttagtgacgt caactccttc tgctttttta ctgaagctga gaaatttctg gcgacagata 2100
   aggtgttctg tgtgggtcag cttgtctgtg ctgtgcttgc cgattctgag gttcaggcaa 2160
   agcgagctgc taagcgagtg aagattgtct atcaagactt ggagccgctg atactaacaa 2220
   ttgaggaaag tatacaacac aactcctcct tcaagccaga aaggaaactg gaatatggaa 2280
   atgttgacga agcatttaaa gtggttgatc aaattcttga aggtgaaata catatgggag 2340
   gtcaagaaca tttttatatg gaaacccaaa gcatgcttgt cgttcccaag ggagaggatc 2400
   aagaaatgga tgtctacgtg tccacacagt ttcccaaata tatacaggac attgttgcct 2460
   caaccttgaa gctcccagct aacaaggtca tgtgccatgt aaggcgtgtt ggtggagcgt 2520
   ttggagggaa ggtgttaaaa accggaatca ttgcagccgt cactgcattt gccgcaaaca 2580
   aacatggccg tgcagttcgc tgtgttctgg aacgaggaga agacatgtta ataactggag 2640
   gccgccatcc ttaccttgga aagtacaaag ctggattcat gaacgatggc agaatcttgg 2700
   ccctggacat ggagcattac agcaatgcag gcgcctcctt ggatgaatca ttattcgtga 2760
   tagaaatggg acttctgaaa atggacaatg cttacaagtt tcccaatctc cgctgccggg 2820
   gttgggcatg cagaaccaac cttccatcca acacagcttt tcgtgggttt ggctttcctc 2880
   aggcagcgct gatcaccgaa tcttgtatca cggaagttgc agccaaatgt ggactatccc 2940
   ctgagaaggt gcgaatcata aacatgtaca aggaaattga tcaaacaccc tacaaacaag 3000
   agatcaatgc caagaaccta atccagtgtt ggagagaatg tatggccatg tcttcctact 3060
   ccttgaggaa agttgctgtg gaaaagttca atgcagagaa ttattggaag aagaaaggac 3120
   tggccatggt ccccctgaag tttcctgttg gccttggctc acgtgctgct ggtcaggctg 3180
   ctgccttggt tcacatttat cttgatggct ctgtgctggt cactcacggt ggaattgaaa 3240
   tggggcaggg ggtccacact aaaatgattc aggtggtcag ccgtgaatta agaatgccaa 3300
   tgtcgaatgt ccacctgcgt ggaacaagca cagaaactgt ccctaatgca aatatctctg 3360
   gaggttctgt ggtggcagat ctcaacggtt tggcagtaaa ggatgcctgt caaactcttc 3420
   taaaacgcct cgaacccatc atcagcaaga atcctaaagg aacttggaaa gactgggcac 3480
   agactgcttt tgatgaaagc attaaccttt cagctgttgg atacttcaga ggttatgagt 3540
   cagacatgaa ctgggagaaa ggcgaaggcc agcccttcga atactttgtt tatggagctg 3600
   cctgttccga ggttgaaata gactgcctga cgggggatca taagaacatc agaacagaca 3660
   ttgtcatgga tgttggctgc agtataaatc cagccattga cataggccag attgaaggtg 3720
   catttattca aggcatggga ctttatacaa tagaggaact gaattattct ccccagggca 3780
   ttctgcacac tcgtggtcca gaccaatata aaatccctgc catctgtgac atgcccacgg 3840
   agttgcacat tgctttgttg cctccttctc aaaactcaaa tactctttat tcatctaagg 3900
   gtctgggaga gtcgggggtg ttcctggggt gttccgtgtt tttcgctatc catgacgcag 3960
   tgagtgcagc acgacaggag agaggcctgc atggaccctt gacccttaat agtccactga 4020
   ccccggagaa gattaggatg gcctgtgaag acaagttcac aaaaatgatt ccgagagatg 4080
   aacctggatc ctacgttcct tggaatgtac ccatctgaat caaatgcaaa cttctggaga 4140
   aaacagagtg cctcttccca gatggcaatc tgtcctatct ctgtgctgga agatgctaga 4200
   tctgaaagac agagtttcca cagttcagaa atcatcccac agtgttgctt ttctatggag 4260
   ctgatttaaa gtattccatt tagatttgat agatatgctt aagcaatcta taaatcattt 4320
   tcaatgttat aaacactaat tggtttcctc tagggtgata ttcgtcatta ctctgtctct 4380
   tcaatccatc cagctaaatg gaataggtga tgacttgcat gtgactccta cttggcttct 4440
   atccaccaac agaaattata ccatatagtg aaaggcaatt ttctaaataa tttcattact 4500
   aatatgaact gtgaagttgt cattttttca tttgtccttt tctgctatca ccttcctctt 4560
   gtcagaatga atatagacac tgtatctaag tgggaccaaa gaaaaaatag cgaactttca 4620
   ccaaagtttt catgaaaacc caaaagcttt aaaagttact atcaagaaat tgaaaggaaa 4680
   cccacagaat aggataaaat atttgtaaat catatatttg ataaaagtct tgtaaccaga 4740
   tacataaaga gctcttacaa ctcaataaaa ggcaagtaat ttaaaaatag gcaaaagaat 4800
   tgctggatgg tatggtagtt ctatttttag tttttaccct aactactctg acttgatcat 4860
   ttaacattct gtgtatgtaa caaaatatca catgcataaa tattatgtat caataaaatt 4920
   ttttaatggg caaaaaaaaa aaaaaaaaa 4949
<210> 4
   <211> 2300
   <212> DNA
   <213> Homo sapiens
<400> 4
   actcggggca acaggcagat ttgcctgctg agggtggaga cccacgagcc gaggcctcct 60
   gcagtgttct gcacagcaaa ccgcacgcta tggctgacag ccgggatccc gccagcgacc 120
   agatgcagca ctggaaggag cagcgggccg cgcagaaagc tgatgtcctg accactggag 180
   ctggtaaccc agtaggagac aaacttaatg ttattacagt agggccccgt gggccccttc 240
   ttgttcagga tgtggttttc actgatgaaa tggctcattt tgaccgagag agaattcctg 300
   agagagttgt gcatgctaaa ggagcagggg cctttggcta ctttgaggtc acacatgaca 360
   ttaccaaata ctccaaggca aaggtatttg agcatattgg aaagaagact cccatcgcag 420
   ttcggttctc cactgttgct ggagaatcgg gttcagctga cacagttcgg gaccctcgtg 480
   ggtttgcagt gaaattttac acagaagatg gtaactggga tctcgttgga aataacaccc 540
   ccattttctt catcagggat cccatattgt ttccatcttt tatccacagc caaaagagaa 600
   atcctcagac acatctgaag gatccggaca tggtctggga cttctggagc ctacgtcctg 660
   agtctctgca tcaggtttct ttcttgttca gtgatcgggg gattccagat ggacatcgcc 720
   acatgaatgg atatggatca catactttca agctggttaa tgcaaatggg gaggcagttt 780
   attgcaaatt ccattataag actgaccagg gcatcaaaaa cctttctgtt gaagatgcgg 840
   cgagactttc ccaggaagat cctgactatg gcatccggga tctttttaac gccattgcca 900
   caggaaagta cccctcctgg actttttaca tccaggtcat gacatttaat caggcagaaa 960
   cttttccatt taatccattc gatctcacca aggtttggcc tcacaaggac taccctctca 1020
   tcccagttgg taaactggtc ttaaaccgga atccagttaa ttactttgct gaggttgaac 1080
   agatagcctt cgacccaagc aacatgccac ctggcattga ggccagtcct gacaaaatgc 1140
   ttcagggccg cctttttgcc tatcctgaca ctcaccgcca tcgcctggga cccaattatc 1200
   ttcatatacc tgtgaactgt ccctaccgtg ctcgagtggc caactaccag cgtgacggcc 1260
   cgatgtgcat gcaggacaat cagggtggtg ctccaaatta ctaccccaac agctttggtg 1320
   ctccggaaca acagccttct gccctggagc acagcatcca atattctgga gaagtgcgga 1380
   gattcaacac tgccaatgat gataacgtta ctcaggtgcg ggcattctat gtgaacgtgc 1440
   tgaatgagga acagaggaaa cgtctgtgtg agaacattgc cggccacctg aaggatgcac 1500
   aaattttcat ccagaagaaa gcggtcaaga acttcactga ggtccaccct gactacggga 1560
   gccacatcca ggctcttctg gacaagtaca atgctgagaa gcctaagaat gcgattcaca 1620
   cctttgtgca gtccggatct cacttggcgg caagggagaa ggcaaatctg tgaggccggg 1680
   gccctgcacc tgtgcagcga agcttagcgt tcatccgtgt aacccgctca tcactggatg 1740
   aagattctcc tgtgctagat gtgcaaatgc aagctagtgg cttcaaaata gagaatccca 1800
   ctttctatag cagattgtgt aacaatttta atgctatttc cccaggggaa aatgaaggtt 1860
   aggatttaac agtcatttaa aaaaaaaatt tgttttgacg gatgattgga ttattcattt 1920
   aaaatgatta gaaggcaagt ttctagctag aaatatgatt ttatttgaca aaatttgttg 1980
   aaattatgta tgtttacata tcacctcatg gcctattata ttaaaatatg gctataaata 2040
   tataaaaaga aaagataaag atgatctact cagaaatttt tatttttcta aggttctcat 2100
   aggaaaagta catttaatac agcagtgtca tcagaagata acttgagcac cgtcatggct 2160
   taatgtttat tcctgataat aattgatcaa attcattttt ttcactggag ttacattaat 2220
   gttaattcag cactgatttc acaacagatc aatttgtaat tgcttacatt tttacaataa 2280
   ataatctgta cgtaagaaca 2300
<210> 5
   <211> 7185
   <212> DNA
   <213> Homo sapiens
<400> 5
   ctctcgtagc gacgccccca ggggggcaga acgcgtgcgc acgcgcgtgc gaacgcgcgt      60
   gcgcgggcgg tgtttgaggc cggccagtcg tgactgggcg gcaatgaggt cagtgactgc     120
   cgggagtcct gcaggggcgg ggcggcgcca agcgcaggga gcccggctga gtggcagccc     180
   agattgaaga tggatacgtg acaatcccag ggaccgctgc actgacttca tttccttaga     240
   caagacacag tgtagggccc ggcccgtgtt ggccccagga ctcctttgga atatagctgt     300
   ggacaatgaa tcctgcgagc gatgggggca catcagagag catttttgac ctggactatg     360
   catcctgggg gatccgctcc acgctgatgg tcgctggctt tgtcttctac ttgggcgtct     420
   ttgtggtctg ccaccagctg tcctcttccc tgaatgccac ttaccgttct ttggtggcca     480
   gagagaaggt cttctgggac ctggcggcca cgcgtgcagt ctttggtgtt cagagcacag     540
   ccgcaggcct gtgggctctg ctgggggacc ctgtgctgca tgccgacaag gcgcgtggcc     600
   agcagaactg gtgctggttt cacatcacga cagcaacggg attcttttgc tttgaaaatg     660
   ttgcagtcca cctgtccaac ttgatcttcc ggacatttga cttgtttctg gttatccacc     720
   atctctttgc ctttcttggg tttcttggct gcttggtcaa tctccaagct ggccactatc     780
   tagctatgac cacgttgctc ctggagatga gcacgccctt tacctgcgtt tcctggatgc     840
   tcttaaaggc gggctggtcc gagtctctgt tttggaagct caaccagtgg ctgatgattc     900
   acatgtttca ctgccgcatg gttctaacct accacatgtg gtgggtgtgt ttctggcact     960
   gggacggcct ggtcagcagc ctgtatctgc ctcatttgac actgttcctt gtcggactgg    1020
   ctctgcttac gctaatcatt aatccatatt ggacccataa gaagactcag cagcttctca    1080
   atccggtgga ctggaacttc gcacagccag aagccaagag caggccagaa ggcaacgggc    1140
   agctgctgcg gaagaagagg ccatagctgc tccagccggg gctccggggc ggcagcagag    1200
   ctggcacacc gattctggga agccccgcga atgatggctt ttgaattaat gaggcagtga    1260
   atgttttgtg tttacttcta agggaaatac taactttctt tcgcattagt attaattttg 1320
   aagtagctac aaagtatttt taagaaatta taattttatg actgtctggc aggctctgtc 1380
   agtttagccg cgccggaccg tgtcaagcat ctaggagagg agtccatggt gtccaggcat 1440
   cggggcgtca cacctgttga ggagtggggt ggctttgaat gctggaaatg gcttcatagt 1500
   gaagtgcctc ccacagggcg ggtgggtcag cgttgactct ttccagctgc acactcatat 1560
   gccgtgtgtc ttattcagaa gtcacattct tttcagttgg agagaattgg gctaagatag 1620
   aaaataacat gatttgttcc ttattaaagc ttcccagcgt atgaaattct aagctgggtg 1680
   gggtggctca cacccgacgt aatcccagca cgttgggagg ccgaggcagg tggatcactt 1740
   gaggccagga gttcgagacc agcctggtca agatggtgaa accccatctc tactaaaatt 1800
   acaaaaatta gccgggtgtc gtggcacaca cctgtaatcc cagctatttg ggaggccaag 1860
   gcaggagaat tgcctgaacc cgggaggcgg aggttgcagt gagctgagat cgcaccactg 1920
   cactccagca ctccagcctg ggtgacagag caagactctc tctcaaaaaa aaaaaaaaaa 1980
   aaaaattcta gaccgaagtg tacggcagtg ccatctggtg gcaagtggta caaagacaac 2040
   gctgaggggt agtgactcct gtagcagcag agacgccttg gagtttaacc cccaccaacc 2100
   agagcgtggg ctggtaaaga tgaaatcttg caagtttttt ttttttttaa atcatggtac 2160
   ctgttttaaa atgagaagtt attattcata ctgtgttgct catttgacaa aataaggtaa 2220
   ggatttcata atcaggttgt ctgggtttca gagctgtttt taattgactg agttacctac 2280
   aggcacccaa tctagaccct aattctgtgg ttggtgttcc tctgtagatt tcaagacaag 2340
   tagtgatgtc attttctccc tgagctgtag ggtggacctg gacccctcag agatgctcga 2400
   ctgcagggtc ctggggtccc tagagtggtc ctgcagcagc ccctggcaat gagctgcctg 2460
   atgatgagtc ccaaggtaca ttgttctact gttttatacc caggtagaag ttacacagca 2520
   atttagcaat gttgttaaca gacatacaga attgtgtgtg tgtgtgtgtg tgtgtgtgtg 2580
   agtgttttga tataaacgtg caattaaaat tcaaagttta ttttttatca caagcacctt 2640
   tgaaatgtac gtggaaaacg tcggctgctc gtgagaatca tgtgtgactt acgttcctta 2700
   tgttttatgg aactctcgga acagtgtttt gtttttgttt agtttttgtt ttcgtttttt 2760
   tgagacggag cctggctttg gctcccaggc tggagtccag tggtgtgatc tcggctcact 2820
   gcaacctcca cctcccgggt tccagggatt ctcctgcctc accctttcaa gtagctggga 2880
   ttacaggtac ctctaccatg cctggctgat tttttgtatt tttagtagag acagggtttc 2940
   accatgttgg ccaggctggt ctctgactcc tgactaaaag tgatctgccc acctcggcct 3000
   cccaaagttc tgggattacg ggcgtgagcc acttcacccg gcccggagcg gtgtattttg 3060
   attgctggtg ctttaactat attgaccaag gtgtatagta acctatttat cacattttac 3120
   gaagcaattt taatcttaaa cacatccagc aaatggtgtc cctgctgaaa cttttaactg 3180
   gaaacctcac acctggcagg cagcactctc tgcagccgtg cccttgtgga gtcagcagga 3240
   gctggtgttt ttcagccaga cagaggcata ataaagtgac tgatgtttac agaacccaaa 3300
   tgatgtcttc ctctctctca aaataaacta acctattcat attgctgtga atgctttatc 3360
   ttggagatag aaaatataat gataaattca tcatgtgttc agaattcgat ttggagttgg 3420
   aagtggacag cctaattttt atctcacctc cactaggact ttgaccttcc caggggtcca 3480
   cactttacct cctgtatctt catacgcgtg tgtggatgca tagagatgga ggtgctgtag 3540
   agaagcacag gctcacccgc tccgtccagt gtcacccaca cattggtccg gggacccctc 3600
   agtgcagagc ctgaagttgg gggtttaggg gtggaaaagg cttcgtgaaa tgttaatgat 3660
   gcgtacacct tccagatggc acagcacttt cccatggtcc tcactggcct cagtctccct 3720
   gaccggcagt gtgaggggat gtagctatag tggagcatgg tacaagcgac attcatgagc 3780
   tctgacttct tggtgccagg ttggtgtgag cctgggcccg gcccactcag cggcccacat 3840
   tggccagcat tcagctgcac gtgtgtgctg cgtgcttcac atcctctatt gagagttaca 3900
   gcaagtgtta aacgaggtga gttcacataa caggaattct ggaactgctt gaaaactagg 3960
   acgattgggc aatatcggcc ttaactccac ctgatggcag gtgacccgga tagaaaatgg 4020
   ccctgcgttt agccaggatg tggctctcca gcttggcttc agtgtgatca cttgtcagtg 4080
   cgctttctct ttcgatagtg aaatccttct ctatacctat gttttgtttt gtttcttaag 4140
   ttgggaaaca gaatgggcca gggaggttga gtgactgaag accaagggtt ggtgcagcct 4200
   cctcgccgcg ctgcgggggc tgggccgcac aggcttctgc ccttctcggt gtccaggctc 4260
   cttgggtgat gctggagttg tcatggccgc agttcagtgt gagatttttt accaggtatt 4320
   gcgcttaagg gacatgattt tccattttct tcgcccggac aacttgaatg aaatgggcac 4380
   tgttgattcc acttctgtcg aggagcttcg gggctcagag aggtgatgac gtgcccaagg 4440
   tgacgcaact cgtgaacagc cgtgcctgcc ttgggcgcag cctccggcgc cagagctggg 4500
   ctcttcaaca cggcatttag cgcagaaagt cgtggttcag gcagtatggg ccgctgtgac 4560
   aaaacaccta agactgggta gtttataaag aacagacatt caggccaggc acggtgactc 4620
   acgcctgtaa tcccagcact ttgggaggcc gaggcgggtg gatcatttga ggtcaggagt 4680
   ttgaaaccag cctggccaac atggtgaaac cccatctcta ctaaaaaaac aaaactagct 4740
   gggggtggtg gtgcatgcct gtggtcccag ctacttggga ggctaaggta gaagaattgc 4800
   ttgaacctgg gaggcagaga ttgcagtgag ccgagatcac gccattgcac tccagcctgg 4860
   gtgacacagt gagactccat ctcaaaaaaa aaaaaagaac ggacatcatt tctcacagtt 4920
   ctggaggctg gaagtccaag ataaaggtgt gggctgatta gtttctgctg ggaacactgc 4980
   atcctcacgt ggtggagggg atggaaggtc aacaggcctc agctagttct ccgctgcctt 5040
   tttatgaggc attaagccat ttgcaagagt agggggcccc acctcttaat accaccgcaa 5100
   tgacaattac aatgtcatgt gaattctgga ggggacacgg tcacactgca gcatcagcac 5160
   ccggtgaatg tggttctctt cccatgtccg aattttgtga gctccacacg ggcagggttg 5220
   catctgtttt attctctgat tgattctaag ccccatgtcc ctggcacagg atggctgctt 5280
   ggcagtaggg agtggatgaa ccagtagtac ccagggctgc caggcagggc cctgcagggg 5340
   acagacccaa gggctgatat ggggacacct gctgaggggg gaatttacca cggtaagggc 5400
   agggctggga ggctgggcag ccagtttcac cctcgcacag gctgcctggc aagaccagcg 5460
   gctggaggtg atgtcctcag tagagaacgg tggccactgg aaacgtgtgg ccagaagggg 5520
   aaacgctcgg gcctctggtg ggggccagcc tggggtgggg tcggccaggg tcagctgcct 5580
   ttccttcatg ctggggagga gatgttgtgt gcatgtgaga aagcatttga tagcatgcca 5640
   aaagtttcct ccattatgtg cgtgtcaagt gtttgaccag gtcagaactc ttgttctatg 5700
   cgaatgtgct cacgcagaca gaagcggagg ggctccgtcg ctctgccctc acgccgctga 5760
   accgcgaggg tctcccgctc ctcaggcttg cggctcggcc gcgaagtgtg tcaggtgtgc 5820
   atcctgggtg ctcctttgtg ctctgtccta cggtgacaca ggcggcgtgg ggttagacag 5880
   gtaccggtca gactacggtg acacaggcgg cgtgcggtta gacaggtacc ggtcagatta 5940
   cggtggcaca ggcggcgtgg ggttagacag gtaccggtca gattacggtg gcacaggcgg 6000
   cgtggggtta gacaggtacc ggtcagatta cggtggcaca ggcggcgtgg ggttagacag 6060
   gtaccggtca gattacggtg gcacaggcgg cgtggggtta gacaggtacc ggtcagatta 6120
   cggtggcaca ggcagcgtgg ggttagacag gtaccggtca gattacggtg gcacaggcgg 6180
   cgtggggtta gacaggtacc ggtcagatta cggtggcaca ggcggcgtgg ggttagacag 6240
   gtaccggtca gattacggtg acacaggcgg cgtggggtta gacaggtacc ggtcagatta 6300
   cggtggcaca ggctgcgtgg ggttagacag gtaccggtca gattacggtg gcacaggcgg 6360
   cgtggggtta gacaggtact ggtcagatgc acgggctccc taaacccctg ctgtggcttc 6420
   ggcagtaaag acaggacgca cccatgtcac aagaggagca caggcagggg tgttggtgtt 6480
   ggggcagccc tcagggtctc cagaccccag ccccactcac acagcagcct aggaaggaag 6540
   ggcagagtcc caggtgtcag ctggtgggtc tcccaggagc tgcccctccc tggaagtcac 6600
   aggacaggaa tgacagatca gggaactgca ggaagctgcc acctctgggg tcagaatatg 6660
   cccagcctgc gggggctctc tatcggggtc ttcgagagcc agacagcctg ccttgtgctg 6720
   catacctggc tttgctctgt gcagaaccca gcacacgtga ttttgtgtga catgccagca 6780
   gcctggctcc caggacagga ggcctgccct gggggagggg ctgcaggagg agggggggca 6840
   ggcacccatg agtctgtcca gccttgtcac agatgcatcg cccaccctgc ggtcctgatt 6900
   tcagctcacc tcagagtaaa tcagaataaa ctgcacccag actttcacga atgcatgttg 6960
   acgctttcag ttcacccctt tctttgctaa ctttcttcct attttcttct aatgcgagag 7020
   cttattaatt ccatatttat cattttgaat aacttttctc ctttttagta acaaaatgta 7080
   cttcactctt agtaaaatgt atttactatt ttagtaacaa aaatatactt gcctaatcat     7140
   gtttaaaata tagtgatgtg aaaaattcaa aaaaaaaaaa aaaaa                     7185
<210> 6
   <211> 2859
   <212> DNA
   <213> Homo sapiens
<400> 6
   ctttccgcgg cattctttgg gcgtgagtca tgcaggtttg cagccagccc caaagggggt      60
   gtgtgcgcga gcagagcgct ataaatacgg cgcctcccag tgcccacaac gcggcgtcgc     120
   caggaggagc gcgcgggcac agggtgccgc tgaccgaggc gtgcaaagac tccagaattg     180
   gaggcatgat gaagactctg ctgctgtttg tggggctgct gctgacctgg gagagtgggc     240
   aggtcctggg ggaccagacg gtctcagaca atgagctcca ggaaatgtcc aatcagggaa     300
   gtaagtacgt caataaggaa attcaaaatg ctgtcaacgg ggtgaaacag ataaagactc     360
   tcatagaaaa aacaaacgaa gagcgcaaga cactgctcag caacctagaa gaagccaaga     420
   agaagaaaga ggatgcccta aatgagacca gggaatcaga gacaaagctg aaggagctcc     480
   caggagtgtg caatgagacc atgatggccc tctgggaaga gtgtaagccc tgcctgaaac     540
   agacctgcat gaagttctac gcacgcgtct gcagaagtgg ctcaggcctg gttggccgcc     600
   agcttgagga gttcctgaac cagagctcgc ccttctactt ctggatgaat ggtgaccgca     660
   tcgactccct gctggagaac gaccggcagc agacgcacat gctggatgtc atgcaggacc     720
   acttcagccg cgcgtccagc atcatagacg agctcttcca ggacaggttc ttcacccggg     780
   agccccagga tacctaccac tacctgccct tcagcctgcc ccaccggagg cctcacttct     840
   tctttcccaa gtcccgcatc gtccgcagct tgatgccctt ctctccgtac gagcccctga     900
   acttccacgc catgttccag cccttccttg agatgataca cgaggctcag caggccatgg     960
   acatccactt ccatagcccg gccttccagc acccgccaac agaattcata cgagaaggcg    1020
   acgatgaccg gactgtgtgc cgggagatcc gccacaactc cacgggctgc ctgcggatga    1080
   aggaccagtg tgacaagtgc cgggagatct tgtctgtgga ctgttccacc aacaacccct    1140
   cccaggctaa gctgcggcgg gagctcgacg aatccctcca ggtcgctgag aggttgacca    1200
   ggaaatacaa cgagctgcta aagtcctacc agtggaagat gctcaacacc tcctccttgc    1260
   tggagcagct gaacgagcag tttaactggg tgtcccggct ggcaaacctc acgcaaggcg    1320
   aagaccagta ctatctgcgg gtcaccacgg tggcttccca cacttctgac tcggacgttc    1380
   cttccggtgt cactgaggtg gtcgtgaagc tctttgactc tgatcccatc actgtgacgg    1440
   tccctgtaga agtctccagg aagaacccta aatttatgga gaccgtggcg gagaaagcgc    1500
   tgcaggaata ccgcaaaaag caccgggagg agtgagatgt ggatgttgct tttgcaccta    1560
   cgggggcatc tgagtccagc tccccccaag atgagctgca gccccccaga gagagctctg    1620
   cacgtcacca agtaaccagg ccccagcctc caggccccca actccgccca gcctctcccc      1680
   gctctggatc ctgcactcta acactcgact ctgctgctca tgggaagaac agaattgctc      1740
   ctgcatgcaa ctaattcaat aaaactgtct tgtgagctga tcgcttggag ggtcctcttt      1800
   ttatgttgag ttgctgcttc ccggcatgcc ttcattttgc tatggggggc aggcaggggg       1860
   gatggaaaat aagtagaaac aaaaaagcag tggctaagat ggtataggga ctgtcatacc      1920
   agtgaagaat aaaagggtga agaataaaag ggatatgatg acaaggttga tccacttcaa      1980
   gaattgcttg ctttcaggaa gagagatgtg tttcaacaag ccaactaaaa tatattgctg      2040
   caaatggaag cttttctgtt ctattataaa actgtcgatg tattctgacc aaggtgcgac      2100
   aatctcctaa aggaatacac tgaaagttaa ggagaagaat cagtaagtgt aaggtgtact      2160
   tggtattata atgcataatt gatgttttcg ttatgaaaac atttggtgcc cagaagtcca      2220
   aattatcagt tttatttgta agagctattg cttttgcagc ggttttattt gtaaaagctg      2280
   ttgatttcga gttgtaagag ctcagcatcc caggggcatc ttcttgactg tggcatttcc      2340
   tgtccaccgc cggtttatat gatcttcata cctttccctg gaccacaggc gtttctcggc      2400
   ttttagtctg aaccatagct gggctgcagt accctacgct gccagcaggt ggccatgact      2460
   acccgtggta ccaatctcag tcttaaagct caggcttttc gttcattaac attctctgat      2520
   agaattctgg tcatcagatg tactgcaatg gaacaaaact catctggctg catcccaggt      2580
   gtgtagcaaa gtccacatgt aaatttatag cttagaatat tcttaagtca ctgtcccttg      2640
   tctctctttg aagttataaa caacaaactt aaagcttagc ttatgtccaa ggtaagtatt      2700
   ttagcatggc tgtcaaggaa attcagagta aagtcagtgt gattcactta atgatataca      2760
   ttaattagaa ttatggggtc agaggtattt gcttaagtga tcataattgt aaagtatatg      2820
   tcacattgtc acattaatgt caaaaaaaaa aaaaaaaaa                             2859
<210> 7
   <211> 2979
   <212> DNA
   <213> Homo Sapiens
<400> 7
   gggcagcctg ctgtcggctt agaggggatg ggcagtgtgg agggcctggc agagcaagag       60
   gactcatcct tccaaaggga ctttctctgg gaagcctgct cctcgggcca ctgcgaaccc      120
   tctctactct ccgaagggaa ttgtccttcc tggcttccac tacttccacc cctgaatgca      180
   caggcagccc ggcccaagtc tcccactagg gatgcagatg gattcggtgt gaagggctgg      240
   ctgctgttgc ctccggctct tgaaagtcaa gttcagaggc gtgcaaagac tccagaattg      300
   gaggcatgat gaagactctg ctgctgtttg tggggctgct gctgacctgg gagagtgggc      360
   aggtcctggg ggaccagacg gtctcagaca atgagctcca ggaaatgtcc aatcagggaa      420
   gtaagtacgt caataaggaa attcaaaatg ctgtcaacgg ggtgaaacag ataaagactc      480
   tcatagaaaa aacaaacgaa gagcgcaaga cactgctcag caacctagaa gaagccaaga 540
   agaagaaaga ggatgcccta aatgagacca gggaatcaga gacaaagctg aaggagctcc 600
   caggagtgtg caatgagacc atgatggccc tctgggaaga gtgtaagccc tgcctgaaac 660
   agacctgcat gaagttctac gcacgcgtct gcagaagtgg ctcaggcctg gttggccgcc 720
   agcttgagga gttcctgaac cagagctcgc ccttctactt ctggatgaat ggtgaccgca 780
   tcgactccct gctggagaac gaccggcagc agacgcacat gctggatgtc atgcaggacc 840
   acttcagccg cgcgtccagc atcatagacg agctcttcca ggacaggttc ttcacccggg 900
   agccccagga tacctaccac tacctgccct tcagcctgcc ccaccggagg cctcacttct 960
   tctttcccaa gtcccgcatc gtccgcagct tgatgccctt ctctccgtac gagcccctga 1020
   acttccacgc catgttccag cccttccttg agatgataca cgaggctcag caggccatgg 1080
   acatccactt ccatagcccg gccttccagc acccgccaac agaattcata cgagaaggcg 1140
   acgatgaccg gactgtgtgc cgggagatcc gccacaactc cacgggctgc ctgcggatga 1200
   aggaccagtg tgacaagtgc cgggagatct tgtctgtgga ctgttccacc aacaacccct 1260
   cccaggctaa gctgcggcgg gagctcgacg aatccctcca ggtcgctgag aggttgacca 1320
   ggaaatacaa cgagctgcta aagtcctacc agtggaagat gctcaacacc tcctccttgc 1380
   tggagcagct gaacgagcag tttaactggg tgtcccggct ggcaaacctc acgcaaggcg 1440
   aagaccagta ctatctgcgg gtcaccacgg tggcttccca cacttctgac tcggacgttc 1500
   cttccggtgt cactgaggtg gtcgtgaagc tctttgactc tgatcccatc actgtgacgg 1560
   tccctgtaga agtctccagg aagaacccta aatttatgga gaccgtggcg gagaaagcgc 1620
   tgcaggaata ccgcaaaaag caccgggagg agtgagatgt ggatgttgct tttgcaccta 1680
   cgggggcatc tgagtccagc tccccccaag atgagctgca gccccccaga gagagctctg 1740
   cacgtcacca agtaaccagg ccccagcctc caggccccca actccgccca gcctctcccc 1800
   gctctggatc ctgcactcta acactcgact ctgctgctca tgggaagaac agaattgctc 1860
   ctgcatgcaa ctaattcaat aaaactgtct tgtgagctga tcgcttggag ggtcctcttt 1920
   ttatgttgag ttgctgcttc ccggcatgcc ttcattttgc tatggggggc aggcaggggg 1980
   gatggaaaat aagtagaaac aaaaaagcag tggctaagat ggtataggga ctgtcatacc 2040
   agtgaagaat aaaagggtga agaataaaag ggatatgatg acaaggttga tccacttcaa 2100
   gaattgcttg ctttcaggaa gagagatgtg tttcaacaag ccaactaaaa tatattgctg 2160
   caaatggaag cttttctgtt ctattataaa actgtcgatg tattctgacc aaggtgcgac 2220
   aatctcctaa aggaatacac tgaaagttaa ggagaagaat cagtaagtgt aaggtgtact 2280
   tggtattata atgcataatt gatgttttcg ttatgaaaac atttggtgcc cagaagtcca 2340
   aattatcagt tttatttgta agagctattg cttttgcagc ggttttattt gtaaaagctg 2400
   ttgatttcga gttgtaagag ctcagcatcc caggggcatc ttcttgactg tggcatttcc 2460
   tgtccaccgc cggtttatat gatcttcata cctttccctg gaccacaggc gtttctcggc     2520
   ttttagtctg aaccatagct gggctgcagt accctacgct gccagcaggt ggccatgact     2580
   acccgtggta ccaatctcag tcttaaagct caggcttttc gttcattaac attctctgat     2640
   agaattctgg tcatcagatg tactgcaatg gaacaaaact catctggctg catcccaggt     2700
   gtgtagcaaa gtccacatgt aaatttatag cttagaatat tcttaagtca ctgtcccttg     2760
   tctctctttg aagttataaa caacaaactt aaagcttagc ttatgtccaa ggtaagtatt     2820
   ttagcatggc tgtcaaggaa attcagagta aagtcagtgt gattcactta atgatataca     2880
   ttaattagaa ttatggggtc agaggtattt gcttaagtga tcataattgt aaagtatatg     2940
   tcacattgtc acattaatgt caaaaaaaaa aaaaaaaaa                            2979
<210> 8
   <211> 4674
   <212> DNA
   <213> Homo sapiens
<400> 8
   acaccctaat gcctccaaca ataactgttg actttttatt ttcagtcaga gaagcctggc      60
   aaccaagaac tgtttttttg gtggtttacg agaacttaac tgaattggaa aatatttgct      120
   ttaatgaaac aatttactct tgtgcaacac taaattgtgt caatcaagca aataaggaag     180
   aaagtcttat ttataaaatt gcctgctcct gattttactt catttcttct caggctccaa     240
   gaaggggaaa aaaatgaaga ttttgatact tggtattttt ctgtttttat gtagtacccc     300
   agcctgggcg aaagaaaagc attattacat tggaattatt gaaacgactt gggattatgc     360
   ctctgaccat ggggaaaaga aacttatttc tgttgacacg gaacattcca atatctatct     420
   tcaaaatggc ccagatagaa ttgggagact atataagaag gccctttatc ttcagtacac     480
   agatgaaacc tttaggacaa ctatagaaaa accggtctgg cttgggtttt taggccctat     540
   tatcaaagct gaaactggag ataaagttta tgtacactta aaaaaccttg cctctaggcc     600
   ctacaccttt cattcacatg gaataactta ctataaggaa catgaggggg ccatctaccc     660
   tgataacacc acagattttc aaagagcaga tgacaaagta tatccaggag agcagtatac     720
   atacatgttg cttgccactg aagaacaaag tcctggggaa ggagatggca attgtgtgac     780
   taggatttac cattcccaca ttgatgctcc aaaagatatt gcctcaggac tcatcggacc     840
   tttaataatc tgtaaaaaag attctctaga taaagaaaaa gaaaaacata ttgaccgaga     900
   atttgtggtg atgttttctg tggtggatga aaatttcagc tggtacctag aagacaacat     960
   taaaacctac tgctcagaac cagagaaagt tgacaaagac aacgaagact tccaggagag    1020
   taacagaatg tattctgtga atggatacac ttttggaagt ctcccaggac tctccatgtg    1080
   tgctgaagac agagtaaaat ggtacctttt tggtatgggt aatgaagttg atgtgcacgc    1140
   agctttcttt cacgggcaag cactgactaa caagaactac cgtattgaca caatcaacct    1200
   ctttcctgct accctgtttg atgcttatat ggtggcccag aaccctggag aatggatgct 1260
   cagctgtcag aatctaaacc atctgaaagc cggtttgcaa gcctttttcc aggtccagga 1320
   gtgtaacaag tcttcatcaa aggataatat ccgtgggaag catgttagac actactacat 1380
   tgccgctgag gaaatcatct ggaactatgc tccctctggt atagacatct tcactaaaga 1440
   aaacttaaca gcacctggaa gtgactcagc ggtgtttttt gaacaaggta ccacaagaat 1500
   tggaggctct tataaaaagc tggtttatcg tgagtacaca gatgcctcct tcacaaatcg 1560
   aaaggagaga ggccctgaag aagagcatct tggcatcctg ggtcctgtca tttgggcaga 1620
   ggtgggagac accatcagag taaccttcca taacaaagga gcatatcccc tcagtattga 1680
   gccgattggg gtgagattca ataagaacaa cgagggcaca tactattccc caaattacaa 1740
   cccccagagc agaagtgtgc ctccttcagc ctcccatgtg gcacccacag aaacattcac 1800
   ctatgaatgg actgtcccca aagaagtagg acccactaat gcagatcctg tgtgtctagc 1860
   taagatgtat tattctgctg tggatcccac taaagatata ttcactgggc ttattgggcc 1920
   aatgaaaata tgcaagaaag gaagtttaca tgcaaatggg agacagaaag atgtagacaa 1980
   ggaattctat ttgtttccta cagtatttga tgagaatgag agtttactcc tggaagataa 2040
   tattagaatg tttacaactg cacctgatca ggtggataag gaagatgaag actttcagga 2100
   atctaataaa atgcactcca tgaatggatt catgtatggg aatcagccgg gtctcactat 2160
   gtgcaaagga gattcggtcg tgtggtactt attcagcgcc ggaaatgagg ccgatgtaca 2220
   tggaatatac ttttcaggaa acacatatct gtggagagga gaacggagag acacagcaaa 2280
   cctcttccct caaacaagtc ttacgctcca catgtggcct gacacagagg ggacttttaa 2340
   tgttgaatgc cttacaactg atcattacac aggcggcatg aagcaaaaat atactgtgaa 2400
   ccaatgcagg cggcagtctg aggattccac cttctacctg ggagagagga catactatat 2460
   cgcagcagtg gaggtggaat gggattattc cccacaaagg gagtgggaaa aggagctgca 2520
   tcatttacaa gagcagaatg tttcaaatgc atttttagat aagggagagt tttacatagg 2580
   ctcaaagtac aagaaagttg tgtatcggca gtatactgat agcacattcc gtgttccagt 2640
   ggagagaaaa gctgaagaag aacatctggg aattctaggt ccacaacttc atgcagatgt 2700
   tggagacaaa gtcaaaatta tctttaaaaa catggccaca aggccctact caatacatgc 2760
   ccatggggta caaacagaga gttctacagt tactccaaca ttaccaggtg aaactctcac 2820
   ttacgtatgg aaaatcccag aaagatctgg agctggaaca gaggattctg cttgtattcc 2880
   atgggcttat tattcaactg tggatcaagt taaggacctc tacagtggat taattggccc 2940
   cctgattgtt tgtcgaagac cttacttgaa agtattcaat cccagaagga aactggaatt 3000
   tgcccttctg tttctagttt ttgatgagaa tgaatcttgg tacttagatg acaacatcaa 3060
   aacatactct gatcaccccg agaaagtaaa caaagatgat gaggaattca tagaaagcaa 3120
   taaaatgcat gctattaatg gaagaatgtt tggaaaccta caaggcctca caatgcacgt 3180
   gggagatgaa gtcaactggt atctgatggg aatgggcaat gaaatagact tacacactgt 3240
   acattttcac ggccatagct tccaatacaa gcacagggga gtttatagtt ctgatgtctt 3300
   tgacattttc cctggaacat accaaaccct agaaatgttt ccaagaacac ctggaatttg 3360
   gttactccac tgccatgtga ccgaccacat tcatgctgga atggaaacca cttacaccgt 3420
   tctacaaaat gaagacacca aatctggctg aatgaaataa attggtgata agtggaaaaa 3480
   agagaaaaac caatgattca taacaatgta tgtgaaagtg taaaatagaa tgttactttg 3540
   gaatgactat aaacattaaa agaagactgg aagcatacaa ctttgtacat ttgtggggga 3600
   aaactattaa ttttttgcaa atggaaagat caacagacta tataatgata catgactgac 3660
   acttgtacac taggtaataa aactgattca tacagtctaa tgatatcacc gctgttaggg 3720
   ttttataaaa ctgcatttaa aaaaagatct atgaccagat attctcctgg gtgctcctca 3780
   aaggaacact attaaggttc attgaaatgt tttcaatcat tgccttccca ttgatccttc 3840
   taacatgctg ttgacatcac acctaatatt cagagggaat gggcaaggta tgagggaagg 3900
   aaataaaaaa taaaataaat aaaatagaat gacacaaatt tgagttttgt gaacccctga 3960
   acagatggtc ttaaggacgt tatctggaac tggagaaaag cagagttgag agacaattct 4020
   atagattaaa tcctggtaag gacaaacatt gccattagaa gaaaagcttc aaaatagacc 4080
   tgtggcagat gtcacatgag tagaatttct gcccagcctt aactgcattc agaggataat 4140
   atcaatgaac taaacttgaa ctaaaaattt tttaaacaaa aagttataaa tgaagacaca 4200
   tggttgtgaa tacaatgatg tatttcttta ttttcacata cactctagct aaaagagcaa 4260
   gagtacacat caacaaaaat ggaaacaagg ctttggctga aaaaaacatg catttgacaa 4320
   atcatgttaa tagctagaca agaagaaagt tagctttgta aacttctact tcatttgatt 4380
   cagagaaaca gagcatgagt tttcttaaaa gtaacaagaa aaggaacaaa aaaaatgagg 4440
   tttgaaatct tttaccatgg caaaacatta acatctttct caaaaacata gagaaatctg 4500
   gaaaaatcaa gaagataaaa ttctggacca gttagtgaca ttctttcaag catacttgta 4560
   aaatgtttcc ttaaagtgtt cttgggatga aaatgattgt catgtctcca acaacagtga 4620
   actgatgttg ttccttggaa taaaagtcaa tccccacctt aaaaaaaaaa aaaa 4674
<210> 9
   <211> 5518
   <212> DNA
   <213> Homo sapiens
<400> 9
   agggcgcggg agcgcggagc gagtttggtt gcacttacac cggtacttaa gcgcggaccg        60
   gcgtgtcctt ggacttagag agtggggacg tccggcttcg gagcgggagt gttcgttgtg       120
   ccagcgacta aaaagagaat taaatatggg tgatgttgag aaaggcaaga agatttttat       180
   tatgaagtgt tcccagtgcc acaccgttga aaagggaggc aagcacaaga ctgggccaaa      240
   tctccatggt ctctttgggc ggaagacagg tcaggcccct ggatactctt acacagccgc      300
   caataagaac aaaggcatca tctggggaga ggatacactg atggagtatt tggagaatcc      360
   caagaagtac atccctggaa caaaaatgat ctttgtcggc attaagaaga aggaagaaag      420
   ggcagactta atagcttatc tcaaaaaagc tactaatgag taataattgg ccactgcctt      480
   atttattaca aaacagaaat gtctcatgac ttttttatgt gtaccatcct ttaatagatc      540
   tcatacacca gaattcagat catgaatgac tgacagaata ttttgttggg cagtcctgat      600
   ttaaaactaa gactggcttg tggttaaatg aatatgttca gtttttgaat tttaatagta      660
   actccaattc agtaaatggt atcactgttt acccctttta aagatatgat tagacttcgt      720
   tagtaatgtt caacttttca caaagatggt gagtgccatc ttaaaactta ctggagattg      780
   gttttatatt tagatttata taactggtta tgtgaatata tttaaatact ggggaaattg      840
   cttcactgtc ttagaaccaa gcaagattca cctgtgtttt gtgttcatgt tcatttgcct      900
   cttaaaggca agggttgaag ataaataagg tagcaatgtc tatagttttg gccttaacta      960
   tgccaatcta attataattc cctgtattta aaatggtttc ttttacttat tgaaaggcat     1020
   tttagtgtgg tttatgtgta atattaaaga ttattcaaca cctctcacat cttacagatc     1080
   tataaggtca catgctttta aaatagtagc aagttaaact tcactcttga attctttaca     1140
   atctaagtca aactaagtta taatttagga ttgtctttaa acagccattc agaaacaaaa     1200
   ctgtagaact gtgtatttga ttgggaatgg tgcttttgcc aacttaaaag gattaaagta     1260
   acggagatat acacaaattt taaaattatg tgtgatcaca agactaaaga taattaaaaa     1320
   gaaaaccaca gatcatgact ttttgactgt gcttgatttc atgactgatg cacaaatttt     1380
   aatgattaaa aagtgcagga gccctaaatg tcagtgcagc agccctaaat gtcagtgcag     1440
   cagtgttaac cagtcatggt gctagattgt ttacttggtt ttctaggact gcctcaacta     1500
   gaataacact tcactaattg actcttagtt tctttgctca gattgagaac tgcagcattt     1560
   atgccagaca tggacagagg aatgcctgtg gtcatagttt tgtgatgtgt aacagtgtat     1620
   aattacatac tgaattattt catgcatagt ctgtgccata cacatttaga gtagtccttg     1680
   gagattttat ggagatggtg agcacaaggt aagtcataaa gaataatgag aaaataaatc     1740
   tatgctggtg cagctgagaa ctgtatcttt gtgggacagt gagaagactg agaagatgtg     1800
   aatccatggt ctcaaaggtg atagggacga ttagataggt gttttaaggc ctgaaagcaa     1860
   tttataacat atgagtctta tttttattta tagaaatgtg gaaagcttgc tgtaattcat     1920
   atttgaagtc ctagtctgag ttctggtggg gaatttaaaa atgcatcctg gaaatccttt     1980
   aaagatttca gactttgaaa ggccttgtag cagaggactt ggtgactgta taaagttagt     2040
   ggtattcagg gacagtgtag caagtagctt acaaggggac aattctggac taatgagaaa     2100
   gacctgaagt gaaggctaga gagttgattt tttttttttt tggcatcctg gaaatgatac     2160
   aggaaacata ttaagataga tacagaaatg tgttcaacct tccatcttgg ctagttgtgg 2220
   cgtttagttt gttttttgag acatggtcac gctgtgtcgc caaggctgga gtgcagtggt 2280
   gcgatctcgg ctggctgcaa cctctatttc ccaggctcaa gcgattctct cacttcagct 2340
   tcccaagtag ctgggactac aggtgttcgc caccatgccc agctaatttt tttgtagaga 2400
   tggagttttg ccatgtggtc ccaggctggt ctcaaactcc tgagctcaag caatccgtcc 2460
   acttgccttg gctccccaaa gtgctgggat tacaggcgtg agccaccagg ccctgcctgg 2520
   ttttcaaatt cagaaatctt attatttaac ccagaagtaa tcagcccagt agtaacttag 2580
   gtttaatttt ttttcaggtt taaaattttt ctcatttatt ttttctgaga cggagtttcg 2640
   cccttttcgc ccaggctgag tacagtggtg caatctcact gcaacctccg ccttccagtt 2700
   gcaagtgatt ctcctgcctc agcctcctga gtagctggga ttacaggcac ccgccaccac 2760
   gcctggctaa tttttgtatt tttagtggag atggtgtttc accatgttgg ccagactggt 2820
   cttggactcc tgacctcgtg atccacccac cttggcctcc caaagttcta ggattacagg 2880
   tgtgagccac cacgtccggc caatttttct catttctatg cctcctatat taaggtctgt 2940
   gttggcacag atgagtaact gccatgttct aggtcagtta tacccaagca cttctggtgg 3000
   tttaaaatgt gattctgtaa cttttttatt tttatttttt tgagataatt tcactcttgt 3060
   tgcccaggct ggagtgcaat ggcgtgatcg ctgctcaccg caacctccgc cttccaggtt 3120
   caagcgattc tcctgactca gcctctcaag tagctgggat tacaagcatg cgccaccatg 3180
   cccagcttat tttgtgtttt tagtagagac agggtttctc catgctggac aggctggtct 3240
   tgaactcccg acgtcgggtg atctgcctgc ctcggcctcc caaagtgctg ggattacagg 3300
   cgtgagccac cacgcctggc caattatgta attttttaaa aaggacattt ctatcaggga 3360
   tatatacctt cagaaataag gaaatagggg aaaaaaagag cactataaac cacatgtttt 3420
   catttctagt gcttcgctgt aagtggctag gttggtagaa tcaaaaacaa gggccagatg 3480
   tatttaaggg gtattcagat gccacctaca tgcttatttt gtctagaaca gtgctgtcta 3540
   atagaacttt ctgtgacgat ggatattttg tagacttttg ctgtccagtg tggtagccac 3600
   taaccacatg tggctgttaa gcccttgaaa tatagctagt gtgactagaa agtattttat 3660
   tttaaattta cataggcaca agtggctagt ggctactgta ttgacattct gggtctagga 3720
   ctagaaccag tggtctgtaa caaaagtact ttctctttac tctattaaat ctagaattag 3780
   ccgggcatgg tcgctcatgc ctgtaatccc agcactttgg gaggccaagg caggcagatc 3840
   acttgaggtc aggcgtttga gaccagcctg gtcaacatgg cgaaaccctg tctctacaaa 3900
   aaacataaaa gttagccagg tgtggtggtg ggcacctgta atctcagcta cttgggaggc 3960
   tgaggcacaa gaatcacttg aacttgggag gtggaggttg cagtgagcca agattgtgcc 4020
   actgcactca agcctgggtg acgagtgaaa ctgtctccaa aaaaaaaaaa aaatctagaa 4080
   ttcttggaag tacattatat tgcccttcag aatagattcc agttcctgtt gtgctcacct 4140
   ttataatttt accataagtt ttacctattc gtaagttggc agttttagat agataacatt 4200
   ctggtggtag ctagggattt accttttgtc atccttttcc tgcacttctc ttgaattcct 4260
   ttatagatgt acagttttgc tttaaccact gaagattgct gtaaattata aagggtgtga 4320
   tagaatccac atggctgtca agaaggagat cttaccaagg acagttgact gactagtctc 4380
   agattgtttc atatcattta tacttgggta agagtaaact agataactgg gcgtcgtggt 4440
   gcacacctgt agtcccagcc actctgaggc aggaggactg cttgtgccca gaagttcgag 4500
   gctgcagtgt agctgtgatt gtgctactgc actccagcct gggcaacaga taaaggaaac 4560
   tccatctctt ttaaaaaaaa aaaaagtggt ctgggtgcag taggtcatgc ctgcaatccc 4620
   agcactttgg gaggccaagg caggcagatc acctgaggtc aggagcttga gaccagcctg 4680
   gccaacatgg tgaaacccca tctctaccaa aaatataaaa actagctggg cgtggtggcg 4740
   gcacctgtaa ttgcagctat ttgagaggct gaggcaggag aatcgcttga acctgggaga 4800
   tgggggttgc agtgagccaa gaccgcccca tcgcactcca gcctgggcaa caatagtgaa 4860
   actccgtctc aaaaagaaaa aaagtttcct tagaatggaa aatattcatt catgagctct 4920
   tttggcaatc cgtcatcagt atattctgaa aaccaataag atgttgccaa gttgggggcg 4980
   agagctatgt aatgcaaggc atatgcctga tgaagtatac aaatacacct gaccagaaac 5040
   tttgtctccc acataagtct cttctaggca ctgtcggggt acatactgag ctgctgcttt 5100
   ggctgtattt tctgtgcctc agaataacca ttgtcctggt gttcatatcc ttagagttca 5160
   gtacaaaatg ttggatatcc atttaatagg ttccaggtta tcttagttgg agtttggggt 5220
   atttgaaaac gtcatgcctt caggctatca tttccctcag aaagctaagt aaatttactg 5280
   cattcatttc tcaaagagta aaagtgcagg ttgtatgtgt ctatgaacat ttaaacatgt 5340
   taaaatgtta aatttaacat tttaaattta aacatttaaa tatgtctgta acttgaacag 5400
   tgtagtttca gaaaggacca ctgggctagt gtaatgcaga aaatgctggg tctaggatta 5460
   ggagaaaatt gtgtttagtg tgtatcaata aacagcccgt ggacccaaaa aaaaaaaa 5518
<210> 10
   <211> 4286
   <212> DNA
   <213> Homo sapiens
<400> 10
   aatcgcgagg cggcgggcga tcccgggctc cccgggctgt gggctacagg cgcagagcgg      60
   gccaggcgcg gagctggcgg cagtgacagg aggcgcgaac ccgcagcgct taccgcgcgg     120
   cgccgcacca tggagcccgc cgtgtcgctg gccgtgtgcg cgctgctctt cctgctgtgg     180
   gtgcgcctga aggggctgga gttcgtgctc atccaccagc gctgggtgtt cgtgtgcctc     240
   ttcctcctgc cgctctcgct tatcttcgat atctactact acgtgcgcgc ctgggtggtg     300
   ttcaagctca gcagcgctcc gcgcctgcac gagcagcgcg tgcgggacat ccagaagcag 360
   gtgcgggaat ggaaggagca gggtagcaag accttcatgt gcacggggcg ccctggctgg 420
   ctcactgtct cactacgtgt cgggaagtac aagaagacac acaaaaacat catgatcaac 480
   ctgatggaca ttctggaagt ggacaccaag aaacagattg tccgtgtgga gcccttggtg 540
   accatgggcc aggtgactgc cctgctgacc tccattggct ggactctccc cgtgttgcct 600
   gagcttgatg acctcacagt ggggggcttg atcatgggca caggcatcga gtcatcatcc 660
   cacaagtacg gcctgttcca acacatctgc actgcttacg agctggtcct ggctgatggc 720
   agctttgtgc gatgcactcc gtccgaaaac tcagacctgt tctatgccgt accctggtcc 780
   tgtgggacgc tgggtttcct ggtggccgct gagatccgca tcatccctgc caagaagtac 840
   gtcaagctgc gtttcgagcc agtgcggggc ctggaggcta tctgtgccaa gttcacccac 900
   gagtcccagc ggcaggagaa ccacttcgtg gaagggctgc tctactccct ggatgaggct 960
   gtcattatga caggggtcat gacagatgag gcagagccca gcaagctgaa tagcattggc 1020
   aattactaca agccgtggtt ctttaagcat gtggagaact atctgaagac aaaccgagag 1080
   ggcctggagt acattccctt gagacactac taccaccgcc acacgcgcag catcttctgg 1140
   gagctccagg acattatccc ctttggcaac aaccccatct tccgctacct ctttggctgg 1200
   atggtgcctc ccaagatctc cctcctgaag ctgacccagg gtgagaccct gcgcaagctg 1260
   tacgagcagc accacgtggt gcaggacatg ctggtgccca tgaagtgcct gcagcaggcc 1320
   ctgcacacct tccaaaacga catccacgtc taccccatct ggctgtgtcc gttcatcctg 1380
   cccagccagc caggcctagt gcaccccaaa ggaaatgagg cagagctcta catcgacatt 1440
   ggagcatatg gggagccgcg tgtgaaacac tttgaagcca ggtcctgcat gaggcagctg 1500
   gagaagtttg tccgcagcgt gcatggcttc cagatgctgt atgccgactg ctacatgaac 1560
   cgggaggagt tctgggagat gtttgatggc tccttgtacc acaagctgcg agagaagctg 1620
   ggttgccagg acgccttccc cgaggtgtac gacaagatct gcaaggccgc caggcactga 1680
   gctggagccc gcctggagag acagacacgt gtgagtggtc aggcatcttc ccttcactca 1740
   agcttggctg ctttcctaga tccacacttt caaagagaaa cccctccaga actcccaccc 1800
   tgacagccca acaccacctt cctcctggct tccagggggc agcccagtgg aatggaaaga 1860
   atgtgggatt tggagtcaga caagcctgag tccagttccc cgtttagaac tcattagctg 1920
   tgtgactctg ggtgagtccc ttaacccctc tgagcccggg tctcttcatt agttgaaagg 1980
   gatagtaata cctacttgca ggttgttgtc atctgagttg agcactggtc acattgaagg 2040
   tgctgggtaa gtggtagctc ttgttgcttc ccgttcagcg tcacatctgc agtggagcct 2100
   gaaaaggctc cacattaggt cacctgtgca cagccatggc tggaatgatg aaggggatac 2160
   gctggagttg ccctgccatc gcctccatca gccagacgag gtcctcacag gagaaggaca 2220
   gctcttcccc accctgggat ctcaggaggg cagccacgga gtggggaggc cccagatgcg 2280
   ctgtgccaaa gccaggtccg aggccaaagt tctccctgcc atccttggtg ccgtcctgcc 2340
   ccttcctcct tcatgcctgg gcctgcaggc ccaccccagc caccactgag tccactcgga 2400
   gtgccctgtg ttcctggaga aggcattcca gggttgaatc ttgtcccagc ctcagcctgg 2460
   gacacctagg tggagagagt ggtctccgct ctgaattgga tccaggggac ctgggctcat 2520
   tcttcttggc tcaccaaccc tgcaggcctc atctttccca aaacccactt tgtcttggtg 2580
   ggagtgggtc cgcgctgctc tgcagcaggg gctggggagt ggacagcatc aggtgggaaa 2640
   gtggagtcca ccctcatgtt tctgtaggat tctcaccgtg gggctggaag aaaagagcat 2700
   cgacttgatt tctccaacca ctcatccctc tttttctttc ttccaccact ccccacccca 2760
   gctgtagtta atttcagtgc cttacaaatc ctaagctcag agaaagttcc atttccgttc 2820
   cagagggaag ggaacctccc taggtccttc cctggcttgt tataacgcaa agcttggttg 2880
   tttatgcaac tctatcttaa gaactgccca gcctcagctg aaaacccgaa tctgagaagg 2940
   aattgcgtca tgtaagggaa gctggaatta agggagctga gccagtcatg gttgtggcgt 3000
   gtgagtcagg agacctaggt ttcagcccct ctctactgtc agcgagctgt gcaacgtggg 3060
   caagtcattg tcctctgagc tgcagtttcc tcatctgtca catcgctaca gacaagacct 3120
   ccctggaacc cttctgattg tcttagacac tgtggttgca aaacccacgg aaagcctcat 3180
   ttgtgtggaa agtcagagga aaaatgatcc agtggacact tggggattat ctgtcattca 3240
   agatccttcc ttcaacccca aggtcagctc ccatctcatt tccagaaagg ctcatacctg 3300
   gcttgcaggg aagcatctgt cttgtcattc caggtgccag aatcctctca gagtcattga 3360
   agggtgttca cccatcccac ccaaggcttg gcacactgcc agtgtcttag cagggtcttg 3420
   tgagggctgg gggcatccag gcactcagaa ggcaaaggaa ccaccctacc catttggcct 3480
   ctggaggggg cagaagaaag aaataaacct catcctatat tttacaaagc atgtgaattc 3540
   tggcattagc tctcatagga gacccatgtg cttccttgct cagtgcaaaa ctgatgattc 3600
   tacttgctgt agatgaatgg ttaacacgag ctagttaaac agtgccattg ttttgccagt 3660
   gaagcctcca accctaagcc actgggacgg tggccagaga tgccagcagc ctctgtcgcc 3720
   cttagtcata taaccaaaat ccagacctta tccacaaccc ggggcttgga aaggaaggta 3780
   ttttggaatc acaccctccg gttatgttgc tccagtaaaa tcttgcctgg aaagaggcag 3840
   tcttcttagc atggtgagct gagttcatgg cttttttttg tagccagtcc tgtccctggc 3900
   catccatgtg atggttttgg atggagttaa acttgatgcc agtgggcagt gcatgtggaa 3960
   agtatcagag taaggctctc ccctccagag ccctgagttt cttggctgca tgaaggtttt 4020
   ctttagaatc agaattgtag ccagtttctt tggccagaag gatgaatact tggatattac 4080
   tgaaagggag gggtggagat gggtgtggca gtgtatggtg tgtgattttt attttcttct 4140
   ttggtcatgg gggccaagga gaaaggcatg aatcttccct gtcaggctct tacagccaca 4200
   ggcactgtgt ctactgtctg gaagacatgt ccccatggct gtggggccgc tgcttctgtt 4260
   taaataaaag tggcctggaa gctggc 4286
<210> 11
   <211> 1024
   <212> **DNA**
   <213> Homo sapiens
<400> 11
   cttcctggct cctccttcct ccccacccct ctaataggct cataagtggg ctcaggcctc 60
   tctgcggggc tcactctgcg cttcaccatg gctttcattg ccaagtcctt ctatgacctc 120
   agtgccatca gcctggatgg ggagaaggta gatttcaata cgttccgggg cagggccgtg 180
   ctgattgaga atgtggcttc gctctgaggc acaaccaccc gggacttcac ccagctcaac 240
   gagctgcaat gccgctttcc caggcgcctg gtggtccttg gcttcccttg caaccaattt 300
   ggacatcagg agaactgtca gaatgaggag atcctgaaca gtctcaagta tgtccgtcct 360
   gggggtggat accagcccac cttcaccctt gtccaaaaat gtgaggtgaa tgggcagaac 420
   gagcatcctg tcttcgccta cctgaaggac aagctcccct acccttatga tgacccattt 480
   tccctcatga ccgatcccaa gctcatcatt tggagccctg tgcgccgctc agatgtggcc 540
   tggaactttg agaagttcct catagggccg gagggagagc ccttccgacg ctacagccgc 600
   accttcccaa ccatcaacat tgagcctgac atcaagcgcc tccttaaagt tgccatatag 660
   atgtgaactg ctcaacacac agatctccta ctccatccag tcctgaggag ccttaggatg 720
   cagcatgcct tcaggagaca ctgctggacc tcagcattcc cttgatatca gtccccttca 780
   ctgcagagcc ttgcctttcc cctctgcctg tttccttttc ctctcccaac cctctggttg 840
   gtgattcaac ttgggctcca agacttgggt aagctctggg ccttcacaga atgatggcac 900
   cttcctaaac cctcatgggt ggtgtctgag aggcgtgaag ggcctggagc cactctgcta 960
   gaagagacca ataaagggca ggtgtggaaa cggcaaaaaa aaaaaaaaaa aaaaaaaaaa 1020
   aaaa 1024
<210> 12
   <211> 1550
   <212> DNA
   <213> Homo sapiens
<400> 12
   tcaacgcctg cctcccctcg agcgtcctca gcgcagccgc cgcccgcgga gccagcacga        60
   acgagcccag caccggccgg atggagcgtc cgcaacccga cagcatgccc caggatttgt       120
   cagaggccct gaaggaggcc accaaggagg tgcacaccca ggcagagaat gctgagttca       180
   tgaggaactt tcagaagggc caggtgaccc gagacggctt caagctggtg atggcctccc       240
   tgtaccacat ctatgtggcc ctggaggagg agattgagcg caacaaggag agcccagtct       300
   tcgcccctgt ctacttccca gaagagctgc accgcaaggc tgccctggag caggacctgg       360
   ccttctggta cgggccccgc tggcaggagg tcatccccta cacaccagcc atgcagcgct       420
   atgtgaagcg gctccacgag gtggggcgca cagagcccga gctgctggtg gcccacgcct       480
   acacccgcta cctgggtgac ctgtctgggg gccaggtgct caaaaagatt gcccagaaag       540
   ccctggacct gcccagctct ggcgagggcc tggccttctt caccttcccc aacattgcca       600
   gtgccaccaa gttcaagcag ctctaccgct cccgcatgaa ctccctggag atgactcccg       660
   cagtcaggca gagggtgata gaagaggcca agactgcgtt cctgctcaac atccagctct       720
   ttgaggagtt gcaggagctg ctgacccatg acaccaagga ccagagcccc tcacgggcac       780
   cagggcttcg ccagcgggcc agcaacaaag tgcaagattc tgcccccgtg gagactccca        840
   gagggaagcc cccactcaac acccgctccc aggctccgct tctccgatgg gtccttacac       900
   tcagctttct ggtggcgaca gttgctgtag ggctttatgc catgtgaatg caggcatgct       960
   ggctcccagg gccatgaact ttgtccggtg gaaggccttc tttctagaga gggaattctc      1020
   ttggctggct tccttaccgt gggcactgaa ggctttcagg gcctccagcc ctctcactgt      1080
   gtccctctct ctggaaagga ggaaggagcc tatggcatct tccccaacga aaagcacatc      1140
   caggcaatgg cctaaacttc agagggggcg aaggggtcag ccctgccctt cagcatcctc      1200
   agttcctgca gcagagcctg gaagacaccc taatgtggca gctgtctcaa acctccaaaa      1260
   gccctgagtt tcaagtatcc ttgttgacac ggccatgacc actttccccg tgggccatgg      1320
   caatttttac acaaacctga aaagatgttg tgtcttgtgt ttttgtctta tttttgttgg       1380
   agccactctg ttcctggctc agcctcaaat gcagtatttt tgttgtgttc tgttgttttt      1440
   atagcagggt tggggtggtt tttgagccat gcgtgggtgg ggagggaggt gtttaacggc      1500
   actgtggcct tggtctaact tttgtgtgaa ataataaaca acattgtctg                 1550
<210> 13
   <211> 2339
   <212> DNA
   <213> Homo sapiens
<400> 13
   acgcggtgcc gcggggcggg agtagaggcg gagggagggg acacgggctc attgcggtgt 60
   gcgccctgca ctctgtccct cactcgccgc cgacgacctg tctcgccgag cgcacgcctt 120
   gccgccgccc cgcagaaatg cttcggttac ccacagtctt tcgccagatg agaccggtgt 180
   ccagggtact ggctcctcat ctcactcggg cttatgccaa agatgtaaaa tttggtgcag 240
   atgcccgagc cttaatgctt caaggtgtag accttttagc cgatgctgtg gccgttacaa 300
   tggggccaaa gggaagaaca gtgattattg agcagagttg gggaagtccc aaagtaacaa 360
   aagatggtgt gactgttgca aagtcaattg acttaaaaga taaatacaaa aacattggag 420
   ctaaacttgt tcaagatgtt gccaataaca caaatgaaga agctggggat ggcactacca 480
   ctgctactgt actggcacgc tctatagcca aggaaggctt cgagaagatt agcaaaggtg 540
   ctaatccagt ggaaatcagg agaggtgtga tgttagctgt tgatgctgta attgctgaac      600
   ttaaaaagca gtctaaacct gtgaccaccc ctgaagaaat tgcacaggtt gctacgattt      660
   ctgcaaacgg agacaaagaa attggcaata tcatctctga tgcaatgaaa aaagttggaa      720
   gaaagggtgt catcacagta aaggatggaa aaacactgaa tgatgaatta gaaattattg      780
   aaggcatgaa gtttgatcga ggctatattt ctccatactt tattaataca tcaaaaggtc      840
   agaaatgtga attccaggat gcctatgttc tgttgagtga aaagaaaatt tctagtatcc      900
   agtccattgt acctgctctt gaaattgcca atgctcaccg taagcctttg gtcataatcg      960
   ctgaagatgt tgatggagaa gctctaagta cactcgtctt gaataggcta aaggttggtc     1020
   ttcaggttgt ggcagtcaag gctccagggt ttggtgacaa tagaaagaac cagcttaaag     1080
   atatggctat tgctactggt ggtgcagtgt ttggagaaga gggattgacc ctgaatcttg     1140
   aagacgttca gcctcatgac ttaggaaaag ttggagaggt cattgtgacc aaagacgatg     1200
   ccatgctctt aaaaggaaaa ggtgacaagg ctcaaattga aaaacgtatt caagaaatca     1260
   ttgagcagtt agatgtcaca actagtgaat atgaaaagga aaaactgaat gaacggcttg     1320
   caaaactttc agatggagtg gctgtgctga aggttggtgg gacaagtgat gttgaagtga     1380
   atgaaaagaa agacagagtt acagatgccc ttaatgctac aagagctgct gttgaagaag     1440
   gcattgtttt gggagggggt tgtgccctcc ttcgatgcat tccagccttg gactcattga     1500
   ctccagctaa tgaagatcaa aaaattggta tagaaattat taaaagaaca ctcaaaattc     1560
   cagcaatgac cattgctaag aatgcaggtg ttgaaggatc tttgatagtt gagaaaatta     1620
   tgcaaagttc ctcagaagtt ggttatgatg ctatggctgg agattttgtg aatatggtgg     1680
   aaaaaggaat cattgaccca acaaaggttg tgagaactgc tttattggat gctgctggtg     1740
   tggcctctct gttaactaca gcagaagttg tagtcacaga aattcctaaa gaagagaagg     1800
   accctggaat gggtgcaatg ggtggaatgg gaggtggtat gggaggtggc atgttctaac     1860
   tcctagacta gtgctttacc tttattaatg aactgtgaca ggaagcccaa ggcagtgttc     1920
   ctcaccaata acttcagaga agtcagttgg agaaaatgaa gaaaaaggct ggctgaaaat     1980
   cactataacc atcagttact ggtttcagtt gacaaaatat ataatggttt actgctgtca     2040
   ttgtccatgc ctacagataa tttattttgt atttttgaat aaaaaacatt tgtacattcc     2100
   tgatactggg tacaagagcc atgtaccagt gtactgcttt caacttaaat cactgaggca     2160
   tttttactac tattctgtta aaatcaggat tttagtgctt gccaccacca gatgagaagt     2220
   taagcagcct ttctgtggag agtgagaata attgtgtaca aagtagagaa gtatccaatt     2280
   atgtgacaac ctttgtgtaa taaaaatttg tttaaagtta aaaaaaaaaa aaaaaaaaa      2339
<210> 14
   <211> 2319
   <212> DNA
   <213> Homo sapiens
<400> 14
   gccccgacgc gcaccgcgat tcgccccaag ggccctgcgc aggacgctga cgcgaagact 60
   cggaggcgga agaaaaaagg agctgtttct aggcttttct aggcgcccag ccgagaaatg 120
   cttcggttac ccacagtctt tcgccagatg agaccggtgt ccagggtact ggctcctcat 180
   ctcactcggg cttatgccaa agatgtaaaa tttggtgcag atgcccgagc cttaatgctt 240
   caaggtgtag accttttagc cgatgctgtg gccgttacaa tggggccaaa gggaagaaca 300
   gtgattattg agcagagttg gggaagtccc aaagtaacaa aagatggtgt gactgttgca 360
   aagtcaattg acttaaaaga taaatacaaa aacattggag ctaaacttgt tcaagatgtt 420
   gccaataaca caaatgaaga agctggggat ggcactacca ctgctactgt actggcacgc 480
   tctatagcca aggaaggctt cgagaagatt agcaaaggtg ctaatccagt ggaaatcagg 540
   agaggtgtga tgttagctgt tgatgctgta attgctgaac ttaaaaagca gtctaaacct 600
   gtgaccaccc ctgaagaaat tgcacaggtt gctacgattt ctgcaaacgg agacaaagaa 660
   attggcaata tcatctctga tgcaatgaaa aaagttggaa gaaagggtgt catcacagta 720
   aaggatggaa aaacactgaa tgatgaatta gaaattattg aaggcatgaa gtttgatcga 780
   ggctatattt ctccatactt tattaataca tcaaaaggtc agaaatgtga attccaggat 840
   gcctatgttc tgttgagtga aaagaaaatt tctagtatcc agtccattgt acctgctctt 900
   gaaattgcca atgctcaccg taagcctttg gtcataatcg ctgaagatgt tgatggagaa 960
   gctctaagta cactcgtctt gaataggcta aaggttggtc ttcaggttgt ggcagtcaag 1020
   gctccagggt ttggtgacaa tagaaagaac cagcttaaag atatggctat tgctactggt 1080
   ggtgcagtgt ttggagaaga gggattgacc ctgaatcttg aagacgttca gcctcatgac 1140
   ttaggaaaag ttggagaggt cattgtgacc aaagacgatg ccatgctctt aaaaggaaaa 1200
   ggtgacaagg ctcaaattga aaaacgtatt caagaaatca ttgagcagtt agatgtcaca 1260
   actagtgaat atgaaaagga aaaactgaat gaacggcttg caaaactttc agatggagtg 1320
   gctgtgctga aggttggtgg gacaagtgat gttgaagtga atgaaaagaa agacagagtt 1380
   acagatgccc ttaatgctac aagagctgct gttgaagaag gcattgtttt gggagggggt 1440
   tgtgccctcc ttcgatgcat tccagccttg gactcattga ctccagctaa tgaagatcaa 1500
   aaaattggta tagaaattat taaaagaaca ctcaaaattc cagcaatgac cattgctaag 1560
   aatgcaggtg ttgaaggatc tttgatagtt gagaaaatta tgcaaagttc ctcagaagtt 1620
   ggttatgatg ctatggctgg agattttgtg aatatggtgg aaaaaggaat cattgaccca 1680
   acaaaggttg tgagaactgc tttattggat gctgctggtg tggcctctct gttaactaca 1740
   gcagaagttg tagtcacaga aattcctaaa gaagagaagg accctggaat gggtgcaatg 1800
   ggtggaatgg gaggtggtat gggaggtggc atgttctaac tcctagacta gtgctttacc 1860
   tttattaatg aactgtgaca ggaagcccaa ggcagtgttc ctcaccaata acttcagaga    1920
   agtcagttgg agaaaatgaa gaaaaaggct ggctgaaaat cactataacc atcagttact    1980
   ggtttcagtt gacaaaatat ataatggttt actgctgtca ttgtccatgc ctacagataa    2040
   tttattttgt atttttgaat aaaaaacatt tgtacattcc tgatactggg tacaagagcc    2100
   atgtaccagt gtactgcttt caacttaaat cactgaggca tttttactac tattctgtta    2160
   aaatcaggat tttagtgctt gccaccacca gatgagaagt taagcagcct ttctgtggag    2220
   agtgagaata attgtgtaca aagtagagaa gtatccaatt atgtgacaac ctttgtgtaa    2280
   taaaaatttg tttaaagtta aaaaaaaaaa aaaaaaaaa                           2319
<210> 15
   <211> 2319
   <212> DNA
   <213> Homo sapiens
<400> 15
   gccccgacgc gcaccgcgat tcgccccaag ggccctgcgc aggacgctga cgcgaagact      60
   cggaggcgga agaaaaaagg agctgtttct aggcttttct aggcgcccag ccgagaaatg     120
   cttcggttac ccacagtctt tcgccagatg agaccggtgt ccagggtact ggctcctcat     180
   ctcactcggg cttatgccaa agatgtaaaa tttggtgcag atgcccgagc cttaatgctt     240
   caaggtgtag accttttagc cgatgctgtg gccgttacaa tggggccaaa gggaagaaca     300
   gtgattattg agcagagttg gggaagtccc aaagtaacaa aagatggtgt gactgttgca     360
   aagtcaattg acttaaaaga taaatacaaa aacattggag ctaaacttgt tcaagatgtt     420
   gccaataaca caaatgaaga agctggggat ggcactacca ctgctactgt actggcacgc     480
   tctatagcca aggaaggctt cgagaagatt agcaaaggtg ctaatccagt ggaaatcagg     540
   agaggtgtga tgttagctgt tgatgctgta attgctgaac ttaaaaagca gtctaaacct     600
   gtgaccaccc ctgaagaaat tgcacaggtt gctacgattt ctgcaaacgg agacaaagaa     660
   attggcaata tcatctctga tgcaatgaaa aaagttggaa gaaagggtgt catcacagta     720
   aaggatggaa aaacactgaa tgatgaatta gaaattattg aaggcatgaa gtttgatcga     780
   ggctatattt ctccatactt tattaataca tcaaaaggtc agaaatgtga attccaggat     840
   gcctatgttc tgttgagtga aaagaaaatt tctagtatcc agtccattgt acctgctctt     900
   gaaattgcca atgctcaccg taagcctttg gtcataatcg ctgaagatgt tgatggagaa     960
   gctctaagta cactcgtctt gaataggcta aaggttggtc ttcaggttgt ggcagtcaag    1020
   gctccagggt ttggtgacaa tagaaagaac cagcttaaag atatggctat tgctactggt    1080
   ggtgcagtgt ttggagaaga gggattgacc ctgaatcttg aagacgttca gcctcatgac    1140
   ttaggaaaag ttggagaggt cattgtgacc aaagacgatg ccatgctctt aaaaggaaaa    1200
   ggtgacaagg ctcaaattga aaaacgtatt caagaaatca ttgagcagtt agatgtcaca    1260
   actagtgaat atgaaaagga aaaactgaat gaacggcttg caaaactttc agatggagtg 1320
   gctgtgctga aggttggtgg gacaagtgat gttgaagtga atgaaaagaa agacagagtt 1380
   acagatgccc ttaatgctac aagagctgct gttgaagaag gcattgtttt gggagggggt 1440
   tgtgccctcc ttcgatgcat tccagccttg gactcattga ctccagctaa tgaagatcaa 1500
   aaaattggta tagaaattat taaaagaaca ctcaaaattc cagcaatgac cattgctaag 1560
   aatgcaggtg ttgaaggatc tttgatagtt gagaaaatta tgcaaagttc ctcagaagtt 1620
   ggttatgatg ctatggctgg agattttgtg aatatggtgg aaaaaggaat cattgaccca 1680
   acaaaggttg tgagaactgc tttattggat gctgctggtg tggcctctct gttaactaca 1740
   gcagaagttg tagtcacaga aattcctaaa gaagagaagg accctggaat gggtgcaatg 1800
   ggtggaatgg gaggtggtat gggaggtggc atgttctaac tcctagacta gtgctttacc 1860
   tttattaatg aactgtgaca ggaagcccaa ggcagtgttc ctcaccaata acttcagaga 1920
   agtcagttgg agaaaatgaa gaaaaaggct ggctgaaaat cactataacc atcagttact 1980
   ggtttcagtt gacaaaatat ataatggttt actgctgtca ttgtccatgc ctacagataa 2040
   tttattttgt atttttgaat aaaaaacatt tgtacattcc tgatactggg tacaagagcc 2100
   atgtaccagt gtactgcttt caacttaaat cactgaggca tttttactac tattctgtta 2160
   aaatcaggat tttagtgctt gccaccacca gatgagaagt taagcagcct ttctgtggag 2220
   agtgagaata attgtgtaca aagtagagaa gtatccaatt atgtgacaac ctttgtgtaa 2280
   taaaaatttg tttaaagtta aaaaaaaaaa aaaaaaaaa 2319
<210> 16
   <211> 909
   <212> DNA
   <213> Homo sapiens
<400> 16
   tcgtgacaaa gcaaattgtc tggtatcatg aatttggaac accattccag accaaaattg 60
   aaaaaatggt tgacctcacc caggtaatgg atgatgaagt attcatggct tttgcatcct 120
   atgcaacaat tattctttca aaaatgatgc ttatgagtac tgcaactgca ttctatagat 180
   tgacaagaaa ggtttttgcc aatccagaag actgtgtagc atttggcaaa ggagaaaatg 240
   ccaagaagta tcttcgaaca gatgacagag tagaacgtgt acgcagagcc cacctgaatg 300
   accttgaaaa tattattcca tttcttggaa ttggcctcct gtattccttg agtggtcccg 360
   acccctctac agccatcctg cacttcagac tatttgtcgg agcacggatc taccacacca 420
   ttgcatattt gacacccctt ccccagccaa atagagcttt gagttttttt gttggatatg 480
   gagttactct ttccatggct tacaggttgc tgaaaagtaa attgtacctg taaagaaaat 540
   catacaactc agcatccagt tggcttttta agaattctgt acttccaatt tataatgaat 600
   actttcttag attttaggta ggaggggagc agaggaatta tgaactgggg taaacccatt 660
   ttgaatatta gcattgccaa tatcctgtat tcttgtttta catttggatt agaaatttaa 720
   catagtaatt cttaagtctt ttgtctgatt tttaaagtac tttcttataa atttggatca 780
   tgttatgatt tgtaacattc acacaacacc tcacttttga atctataaaa gaattgcacg 840
   tatgagaaac ctatatttca atactgctga aacagacatg aaataaagaa tttaaagaat 900
   gaaaaaaaa 909
<210> 17
   <211> 953
   <212> DNA
   <213> Homo sapiens
<400> 17
   agtccctgca ttgcgcgcga cccggcggcg ggacaggctt gctgcttcct cctcctcggc 60
   ctcaccattc cagaccaaaa ttgaaaaaat ggttgacctc acccaggtaa tggatgatga 120
   agtattcatg gcttttgcat cctatgcaac aattattctt tcaaaaatga tgcttatgag 180
   tactgcaact gcattctata gattgacaag aaaggttttt gccaatccag aagactgtgt 240
   agcatttggc aaaggagaaa atgccaagaa gtatcttcga acagatgaca gagtagaacg 300
   tgtacgcaga gcccacctga atgaccttga aaatattatt ccatttcttg gaattggcct 360
   cctgtattcc ttgagtggtc ccgacccctc tacagccatc ctgcacttca gactatttgt 420
   cggagcacgg atctaccaca ccattgcata tttgacaccc cttccccagc caaatagagc 480
   tttgagtttt tttgttggat atggagttac tctttccatg gcttacaggt tgctgaaaag 540
   taaattgtac ctgtaaagaa aatcatacaa ctcagcatcc agttggcttt ttaagaattc 600
   tgtacttcca atttataatg aatactttct tagattttag gtaggagggg agcagaggaa 660
   ttatgaactg gggtaaaccc attttgaata ttagcattgc caatatcctg tattcttgtt 720
   ttacatttgg attagaaatt taacatagta attcttaagt cttttgtctg atttttaaag 780
   tactttctta taaatttgga tcatgttatg atttgtaaca ttcacacaac acctcacttt 840
   tgaatctata aaagaattgc acgtatgaga aacctatatt tcaatactgc tgaaacagac 900
   atgaaataaa gaatttaaag aatcaaaaaa aaaaaaaaaa aaaaaaaaaa aaa 953
<210> 18
   <211> 987
   <212> DNA
   <213> Homo sapiens
<400> 18
   ggcagatgga agacttgggg gggtctctgc cagctggaag tgcttggctc cacttagcag 60
   ctaaacttag cttttcaatc gatcgctttt gaaagggaat tgtatttctg tccccgtgcg 120
   gattccagac caaaattgaa aaaatggttg acctcaccca ggtaatggat gatgaagtat 180
   tcatggcttt tgcatcctat gcaacaatta ttctttcaaa aatgatgctt atgagtactg 240
   caactgcatt ctatagattg acaagaaagg tttttgccaa tccagaagac tgtgtagcat 300
   ttggcaaagg agaaaatgcc aagaagtatc ttcgaacaga tgacagagta gaacgtgtac     360
   gcagagccca cctgaatgac cttgaaaata ttattccatt tcttggaatt ggcctcctgt     420
   attccttgag tggtcccgac ccctctacag ccatcctgca cttcagacta tttgtcggag     480
   cacggatcta ccacaccatt gcatatttga caccccttcc ccagccaaat agagctttga     540
   gtttttttgt tggatatgga gttactcttt ccatggctta caggttgctg aaaagtaaat     600
   tgtacctgta aagaaaatca tacaactcag catccagttg gctttttaag aattctgtac     660
   ttccaattta taatgaatac tttcttagat tttaggtagg aggggagcag aggaattatg     720
   aactggggta aacccatttt gaatattagc attgccaata tcctgtattc ttgttttaca     780
   tttggattag aaatttaaca tagtaattct taagtctttt gtctgatttt taaagtactt     840
   tcttataaat ttggatcatg ttatgatttg taacattcac acaacacctc acttttgaat     900
   ctataaaaga attgcacgta tgagaaacct atatttcaat actgctgaaa cagacatgaa     960
   ataaagaatt taaagaatga aaaaaaa                                        987
<210> 19
   <211> 917
   <212> DNA
   <213> Homo sapiens
<400> 19
   gaattcaagt cctaaagcct acagttttga atactactga aatgacaagt tattccagac       60
   caaaattgaa aaaatggttg acctcaccca ggtaatggat gatgaagtat tcatggcttt      120
   tgcatcctat gcaacaatta ttctttcaaa aatgatgctt atgagtactg caactgcatt      180
   ctatagattg acaagaaagg tttttgccaa tccagaagac tgtgtagcat ttggcaaagg      240
   agaaaatgcc aagaagtatc ttcgaacaga tgacagagta gaacgtgtac gcagagccca      300
   cctgaatgac cttgaaaata ttattccatt tcttggaatt ggcctcctgt attccttgag      360
   tggtcccgac ccctctacag ccatcctgca cttcagacta tttgtcggag cacggatcta     420
   ccacaccatt gcatatttga caccccttcc ccagccaaat agagctttga gtttttttgt     480
   tggatatgga gttactcttt ccatggctta caggttgctg aaaagtaaat tgtacctgta      540
   aagaaaatca tacaactcag catccagttg gctttttaag aattctgtac ttccaattta      600
   taatgaatac tttcttagat tttaggtagg aggggagcag aggaattatg aactggggta      660
   aacccatttt gaatattagc attgccaata tcctgtattc ttgttttaca tttggattag      720
   aaatttaaca tagtaattct taagtctttt gtctgatttt taaagtactt tcttataaat      780
   ttggatcatg ttatgatttg taacattcac acaacacctc acttttgaat ctataaaaga      840
   attgcacgta tgagaaacct atatttcaat actgctgaaa cagacatgaa ataaagaatt      900
   taaagaatga aaaaaaa                                                     917
<210> 20
   <211> 688
   <212> DNA
   <213> Homo sapiens
<400> 20
   agccccgccc caggcgaggg cgccgcaccc acaccgcgct gcgcagtttt gttctgctcc        60
   agctgttcga aggtgatcca gacgcaagat ggctgtcctc tctaaggaat atggttttgt       120
   gcttctaact ggtgctgcca gctttataat ggtggcccac ctagccatca atgtttccaa       180
   ggcccgcaag aagtacaaag tggagtatcc tatcatgtac agcacggacc ctgaaaatgg       240
   gcacatcttc aactgcattc agcgagccca ccagaacacg ttggaagtgt atcctccctt       300
   cttatttttt ctagctgttg gaggtgttta ccacccgcgt atagcttctg gcctgggctt       360
   ggcctggatt gttggacgag ttctttatgc ttatggctat tacacgggag aacccagcaa       420
   gcgtagtcga ggagccctgg ggtccatcgc cctcctgggc ttggtgggca caactgtgtg       480
   ctctgctttc cagcatcttg gttgggttaa aagtggcttg ggcagtggac ccaaatgctg       540
   ccattaaaga attatagggg tttaaaaact ctcattcatt ttaaatgact tacctttatt       600
   tccagttaca ttttttttct aaatataata aaaacttacc tggcatcagc ctcataccta        660
   aaaaaaaaaa aaaaaaaaaa aaaaaaaa                                            688
<210> 21
   <211> 7986
   <212> DNA
   <213> Homo sapiens
<400> 21
   ggaagtgacg ttaggggaag gtgggggcaa tcatggtgcc gctggggagg ggagaagctg        60
   ctgctgccgc cgttgccggg agccgcggag acaagtcatt acgttttcat ttctcacaac       120
   tgggctgagc acaactgaac catgggggaa cacagtccag acaacaacat catctacttt       180
   gaggcagagg aagatgagct gacccccgat gataaaatgc tcaggtttgt ggataaaaac       240
   ggactggtgc cttcctcatc tggaactgtt tatgatagga ccactgttct tattgagcag       300
   gaccctggca ctttggagga tgaagatgac gacggacagt gcggagaaca cttgcctttt       360
   ctagtagggg gtgaagaggg ctttcacctg atagatcatg aagcaatgtc ccagggttat       420
   gtgcagcaca ttatctcacc agatcagatt catttgacaa taaaccctgg ttccacaccc       480
   atgccaagaa atattgaagg tgcaaccctc actctgcagt cggaatgtcc ggaaacaaaa       540
   cgtaaagaag taaagcggta ccaatgtacc tttgagggct gtccccgcac ctacagcaca       600
   gcaggcaacc tgcgaaccca ccagaagact caccgaggag agtacacctt tgtctgtaat       660
   caggagggct gtggcaaagc cttccttacc tcttacagcc tcaggatcca cgtgcgagtg       720
   cacacgaagg agaagccatt tgagtgtgac gtgcagggct gtgagaaggc attcaacaca       780
   ctgtacaggc tgaaagcaca tcagaggctt cacacaggga aaacgtttaa ctgtgaatct       840
   gaaggctgca gcaaatactt caccacactc agtgatctga ggaagcacat tcgaactcat 900
   acaggggaaa agccatttcg gtgcgatcac gatggctgtg gaaaagcatt tgcagcaagc 960
   caccacctta aaactcacgt tcgtacacat actggtgaaa gacccttctt ctgccccagt 1020
   aatggctgtg agaaaacatt cagcactcaa tacagtctca aaagtcacat gaaaggtcat 1080
   gataacaaag gacactcata caatgcactt ccacaacaca atggatcaga ggatacaaat 1140
   cactcacttt gtctaagtga cttgagcctt ctgtccacag attctgaatt gcgagaaaat 1200
   tccagtacga cccagggcca ggacctcagc acaatttcac cagcaatcat ctttgaatca 1260
   atgttccaga attcagatga tacggcaatt caggaagatc ctcaacagac agcttccttg 1320
   actgaaagtt ttaatggtga tgcagagtca gtcagtgatg ttccgccatc cacaggaaat 1380
   tcagcatctt tatctcttcc acttgtactg caacctggcc tctccgagcc accccagcct 1440
   ctactacctg cctcagctcc gtctgctcct ccgcctgctc cctccctagg acctggctcc 1500
   cagcaagctg catttggcaa cccccctgct ctcttacaac ctccagaagt gcctgttccc 1560
   cacagcacac agtttgctgc taatcatcaa gagtttcttc cgcaccccca ggcaccgcag 1620
   cccattgtac caggactttc tgttgttgct ggggcttctg catcagcagc ggcagtggca 1680
   tcagctgtgg cagcaccagc cccaccacaa agtactactg agcccctgcc agccatggtc 1740
   cagactctgc ccctgggtgc caactctgtc ctaactaata atcccacaat aaccatcacc 1800
   ccaactccca acacagctat cctgcagtcc agcctagtca tgggagaaca gaacttacaa 1860
   tggatattaa atggtgccac cagttctcca caaaaccaag aacaaattca gcaagcatct 1920
   aaagttgaga aggtgttttt taccactgca gtaccagtag ccagtagccc agggagctct 1980
   gtccagcaga ttggcctcag tgttcctgtg atcatcatca aacaagaaga ggcatgtcag 2040
   tgtcagtgtg catgccggga ctctgcaaag gagcgggcat ccagcaggag aaagggctgc 2100
   tcctccccac cccctccaga gccgagcccc caggctcctg atgggcccag cctgcagctc 2160
   ccagcgcaga ctttctcttc agcccctgtt cccgggtcat catcctctac cttgccctcc 2220
   tcctgtgagc aaagccgaca agcagagact ccttcagacc ctcagacaga aacattaagt 2280
   gccatggatg tgtcagagtt tctatccctc cagagcctgg acaccccgtc caatctgatt 2340
   cccattgaag cactactgca gggggaggag gagatgggcc tcaccagcag cttctccaag 2400
   tgaagggccc atgtgtgctc acctctggga aaagcgggtg agcaggaggc atgaggtaca 2460
   atgcctgcca tcatgggtca gaaatttgaa ggatgaagaa atctactgtt tgaaatcctc 2520
   acctttcaga cgtattttct ttattcacat cccaggagca tccattttaa ggaactattc 2580
   tttggaaaaa aacaaaaaac aaaaaaaaca acaaaaaaag ctaagttata agtgaactgt 2640
   ttggctgcac tgtatgtcac ttttgcttgt tgtcatgtga acttggaaac taaggttact 2700
   cgtgtgcata aaaattctaa atgaaagggt gtggtttcca tcaatctgat gctgcccatc 2760
   gcttgcactg gggtctttgt ggatcgggca ggagttttca gtgtgttggg tgttgctcct 2820
   tcctatgtgt cttttgaatc tgaggctgac atttgcttgg aaggccagac ccttgctcca 2880
   tcagagaggg cagtggcaaa ggccagtgag gcagctgtga gttggacagg gttcaggtga 2940
   gatggtgttg tcatttgtgc ttagtgttgg tggtgctcag ggtggataac acgggtcgtt 3000
   ctgcagcccg cttcagcaca aataggcagc ttaaggcctg gctcacaggc tgtggggttg 3060
   atctggctct gcagaggccc taggcagctt gttgactgct gtctgttgat gacgtgtgtg 3120
   caaagcaggc tctagcaaca tgatcactgt ccttgccttc ctggttcttt ctctcggttg 3180
   gttgccaggg cttgcagatc gcagtgaatt ttccttgggg aacatcgctg ttttgtccta 3240
   gagtgaactt gtggcttatg gccagtgctg tttggtggtc tgccttcttt ttaatggtat 3300
   tttcttcctc agagcagaag ggctgcattt tgcttatcag aagaaggtgc agatttaagg 3360
   gaattcatat gaggtggcat gtaattggca ggccaggtgt cctggttcca ggttccagcc 3420
   aggctttggg ttgccccctc catctctgcc cccctctgga ttttgcatac agcctcatac 3480
   agtgcaaaca aggatgtgac ttgctcagct tagtcatgtg atttatttaa aaaaaaaaaa 3540
   aaaagaaaca caaaacgatg atcttctact cagggtatag caaaacaaaa aaattccctt 3600
   tccaccaaaa agcctgaaat gttgcaataa gttatctcat ttggaatgtt tcattaagtt 3660
   gtgttatagg aaaaaattgt gtgtgtgtgt tatagaatta tatccatatg tctgcctttg 3720
   gctccaagtc attgcctctt aaaataaaag atacaatcca tactagcatg aaaagtttcc 3780
   ctcaacaggc tatattaaca tagtcatgag tgctgaccaa actcaccgag ctcagaggcc 3840
   aggcatggcc tgaggtgcag aataggcctc tgcctcccaa gagccctttc cttgccctga 3900
   gcaaggagtg gtgttccaca aacaaggctg ctcttctaag ccaacagtgt caggcaggaa 3960
   gcagccataa ttttgccttg cattttcatt ccctaatgta aagggatctg cattggtcac 4020
   tctcctgttc tctgagccat tgctcagggc cagccaagat attattgaga acagataatt 4080
   taccttggag ccagaggccc tccctgcctt tagcaaggat gttcagggac agacaaagag 4140
   ggcagtggtg gtgaatgttg ttactgccat gaggagaaat ggcagtaaga aatcttaact 4200
   acaagcagcc aatttctcat tccaggaccc tagccagaat aattgacttc tttttttttt 4260
   tgagacagag ttttgctttt gttgccctgg ctggagtgca gtggcgcaat cttggctcac 4320
   cgcaacctcc acttcccagg ttcaagcaat tctgcctcag cctcccgagt agctgggatt 4380
   acaggcatgc gccaccacgc ctggctaatt ttgtattttt agtagagacg gggtttctcc 4440
   atgttggtca ggctggtctc gaactcccaa cgtcaggtga tcctcccgcc tcagtctccc 4500
   aaagtgctgg gattacaggt gtgagccacc atgcctggtc tagggaattg acttcttttt 4560
   gaccttctgc actcccttcc ccaaaaggat tgtggcttct gttgaccatt gacctcagca 4620
   gaagttgata aggcaggagg tttccagtcc tcctggaaaa ccaactgctg agcatgagtt 4680
   gtccttggct gtctctgggc ctcgcaccca tgggaagttt tgaggtaggg cccttgctgt 4740
   ttacaacttc tgagagagta gtgatgggac cccaaagtaa gcttgtatca gagggcagta     4800
   atgaccttcc catatcccca tcctgtggtc acctgggatt tgggttccct ggggcggaag     4860
   tgggaaatag gagcccaggg aaggacatct gaagcaccca cagtttaaat agcgactctt     4920
   cttggttagc cagggctgtg ctcatgttgg gccctaccca ggtcagtggc cttttcactc     4980
   tcaaagatcg ggggtgacga agcagcatct taaacagtgt ttaggctgac agatttttcc      5040
   agttgaagaa gctgaagtat ctgccttttg agggtgactc tagattacaa gagagactat     5100
   cagatccaat cagcttttga aaaatcagga atgtgcttaa aatgccagtg agtggttgtg     5160
   aagatcaggt ttatttccca ggataggcag tctttctttc cctccttttc cagttctctt      5220
   ttcctacctg tccttccata agcctggtct ctagtgccga ggaccttgga gaagagaggg     5280
   cccctagcta agctggagcc agaaagaacc ttcatggtga gtgtggtttc ccaaacttgg     5340
   gaaatgaacc tagggtgagg ttagggagga tttgtagaat tctagcaggt aaaaatcaag     5400
   cctttttccc ctcccatagt aaacttaacc attttcaccc tgtacgttat cttctagctc      5460
   ctactaacat ctccaattag acaaccgctt caaagggtgc ttagcaccag ggattggggt     5520
   tcatggactt aggaggtggt aaggaaactg gctggcacca ccaaactgcc ttagtgaact     5580
   tggcccttcc caggcaggta ggttgggcat tgagggaagg tggcccagtc tttgctggca     5640
   gggtctggcc agtctcatgg gggcaccacc agtttggtga cagaagtggt gtcatttatt     5700
   gaattccacc tccgtttaga aggagatcat ggtacaaagc ccaggagggg ccttaagatg     5760
   tgaagatctc tctgagtaaa aggcaagggg cttctcttct tcacctctgg gatagttggt     5820
   agatcggaga gttttatttt cagggtcaag gctgtggact gatggggata ttggagggtg     5880
   ggtgggtttt cctgagagac tttgtataat gctgaatgtg tccagaggga caagtttgca     5940
   gaacctcata ttggtatatt aaagaaataa taaaataaaa aagcacttta ggttatttta     6000
   tctttaaccc gattgctgca atttcttttg tgtgtatata tacatatata tactttccac     6060
   aaagttttat tttttgctca gaataaaaag ttaaattgag gtgtgaaaag aaaagcactt     6120
   accttggtgc aatatgtgta gcttgatggt cgttgtccca tgtggccctg gcctggcagc     6180
   gtttttccgc tcaatcagcc ctgtgctgtg agactgtcca tagggaaaca ctattatgca     6240
   ttctcagcaa ccgctcaatc tatgcaagcc ttccctgtgt gccccagggc gccccctcag     6300
   gctctctgaa gaactgctgt gggtcctgtt ttctgctgac tgttgaggcc ctttttcatc     6360
   acttcttggt ctctcgccat cttttccctc ttcaccatta caaaatgatg cctgaaagga     6420
   aggaacagat tgttcctagg tagaaacctg gcaccttcta gacttttata tttgtaatca     6480
   catccattgt ccttaaagac ttttccagag tgattgaaac cattgatttg tggaactgca     6540
   acaatatttc tcaagagttt acaattgtct tacaccacca atcagaaata tgtttgggga     6600
   ggcatgggtg gcagggggca acatgagcca tttccatact gctccccata ctacctgtgt     6660
   tggttcctta agcaaaaagg cctccagcct tcattaaatc ctataaacaa acattacatc     6720
   ccatgaatca ttatcaactt tcttccttta ctccacctcc cccattttat cttcaattct 6780
   caggctatag aatagacaag ttttagaata tacttcagcc aaagcagaag gattttcata 6840
   gatccaatat gcaaatagtt ctgctgtgac atagatcaga aattgtctgt tccttaatat 6900
   ttgggggtgg aggcaacaaa actgaagcat atttctgatt ggttagccat tttggcctgt 6960
   ttcctcttgc atgtttttta gggagagagg gaaacagtcc tgtatttctt ctgatgccct 7020
   ttggggaagc cgacgaccac tgggcatttc tcactgttac tcctgttcaa gagagggctt 7080
   ctcagtctgc actgaaaaat gcaaattaaa ctggatcttt atgtcaatgt gtacatagta 7140
   caagcttttt tactggaatt gaggtttaaa accacacact gccctttggg tggtgtgcct 7200
   gttgggccaa aaattgggtg ataatgtagt gtcactttct cagctcaatg cagtttctac 7260
   tttttcttat gggaaaattt ttcataaaac ctttttgcac caaaacccag gggtgttttt 7320
   tgcaatatcc ttgttatcct cgtagtgtgc caagtcagag gctttctctt gcccttttcc 7380
   tgctgtgttc tcaggcctcc caagggctat ttgactcaac agtctacatc cttcgttgtg 7440
   ttttggagaa tgtgggggtg ggggtcagag ttcaaggtgt ctgttccctt ttcctgtgaa 7500
   ctctttctag tccctatttg gggagggtgg ctggaaacag atttttgctg aatttctggc 7560
   tcagatcttc tagccaggaa aggcaagagc ccccaagagc cctttttttt gacatacact 7620
   aatcattggc cggggtcttg gtgacaactt ttaaaatccc aaatagtttt atttggatta 7680
   tgtaaaagta agtgtgaaac atgggaacaa cggacttcca ctgagcgatg tgaaaacgtt 7740
   acaggttcag tacttccaaa ggaagaaacc tccaaaccca aaaaagaata aatatgaatt 7800
   tgtatttttg aagaatgtga aataatggtg tttgcttaat tgctcatttt gtataaactt 7860
   aatattgtac tttaaaatat ctgctaaaaa gtgaaaattt aactttttgg aattgaaaaa 7920
   gcaatattaa atactaatga aatcctaatt aaatgcttat ttaaatctgg taaaaaaaaa 7980
   aaaaaa 7986
<210> 22
   <211> 3417
   <212> DNA
   <213> Homo sapiens
<400> 22
   ttctccaggc ccggctgaca gagttagccg aggccgccat attgaataag cgacccggcc      60
   tcctaggggg tcgtcgtggt ccagacagtt tagcagaaca gcctccgcgg ctccggggag     120
   aagcaatatg ttaaggatac ctgtaagaaa ggccttagta ggcctttcta agtctcctaa     180
   aggatgtgtt cgaacaactg ccacagcagc aagcaacttg attgaagtat ttgttgatgg     240
   tcagtctgtc atggtggaac cgggaacgac cgtcctccaa gcttgtgaga aggttggcat     300
   gcagatccct cgattctgtt atcatgaaag gttgtctgtt gctggaaact gcaggatgtg     360
   ccttgttgaa attgagaaag cccctaaggt tgtagctgct tgtgccatgc cagtaatgaa     420
   gggttggaat atcctaacaa actcagaaaa atccaaaaaa gccagggaag gtgtgatgga      480
   gttcttatta gcaaatcacc cattggactg tcctatttgt gaccagggag gtgaatgtga      540
   tctgcaggac cagtccatga tgtttggaaa tgataggagc cgatttttag aggggaagcg      600
   tgctgtggaa gacaagaaca ttgggccatt ggtaaagacc atcatgacaa gatgtataca      660
   gtgtactcgc tgcatcaggt ttgcaagtga gattgcagga gtagatgatt tgggaacaac      720
   aggcagagga aatgatatgc aagttggcac atacattgaa aagatgttca tgtctgaact      780
   gtctgggaat atcattgata tctgccctgt aggtgcccta acctctaagc cctatgcctt      840
   tactgcccgg ccttgggaaa caagaaagac agaatccatt gatgtaatgg atgcggttgg      900
   aagtaatatt gtggttagca caagaactgg agaagtgatg aggattttgc cacgtatgca      960
   tgaggacatc aatgaagagt ggatctctga taaaaccaga tttgcctatg atgggctaaa     1020
   acgtcaaaga cttaccgagc caatggtcag aaatgaaaaa gggcttttaa cctatacttc     1080
   ttgggaggat gcgctctctc gcgtagctgg aatgttgcag agttttcaag gcaaagatgt     1140
   ggcagcaatt gcaggtggct tggtggatgc tgaagccctg gtagctctca aagatttgct     1200
   taatagagtg gactctgaca ccttatgcac tgaagaggtc ttccccactg caggagctgg     1260
   cacagatttg cgttccaatt atcttcttaa tactacaatt gctggtgtgg aagaggcaga     1320
   tgttgttctt ctggttggta caaacccacg ttttgaggca ccactgttta atgctagaat     1380
   tcgaaagagc tggctgcata atgacttaaa agtggccctt ataggcagtc cagtggacct     1440
   cacttacaca tatgaccacc tgggagactc cccccaaatt cttcaagaca ttgcttcggg     1500
   aagccatcca tttagccagg tcctaaagga agctaaaaaa ccaatggtgg ttttaggcag     1560
   ttctgcactc caaagaaatg atggagcagc aattcttgca gctgtttcta gcattgcaca     1620
   aaagattcgg atgactagtg gtgttactgg tgattggaaa gttatgaata tccttcatag     1680
   gattgcaagt caagtagctg ctttggacct tggctataag cctggggtgg aagcaattcg     1740
   gaagaaccct cccaaggtgc tgtttctcct gggagcagat ggaggttgta tcacacgaca     1800
   ggatttgcca aaggattgtt tcattattta tcaaggacat catggtgatg ttggggctcc     1860
   catagctgat gttattctcc caggagctgc ttacacagag aagtctgcta catatgtcaa     1920
   cactgagggt agagctcagc agactaaggt agcagtgaca cctcctggct tggcaagaga     1980
   agactggaaa attataagag cactctctga gattgctgga atgactcttc catatgatac     2040
   tctggatcaa gtaaggaaca gattggaaga agtctctcct aatcttgttc gatatgatga     2100
   tattgaaggg gctaattact tccagcaagc aaatgagctc tcaaagctag tgaaccagca     2160
   gcttcttgct gacccacttg ttccacctca gctaactata aaagacttct acatgacaga     2220
   ttcaattagc agagcctcac agacaatggc caaatgtgtc aaagctgtca cagagggtgc     2280
   ccaggcagta gaggaaccat ccatatgctg aagcttctac taggatccca gttttgccgc     2340
   agataattaa tggacaactg tagtgcagtg atcctttaca ggtttatttc tttgtaaaaa      2400
   aaaataataa taatttgaat catgtaatat ttaaggttat actatgccta tttgaaaatg      2460
   atattagtta tcaactttgc agtttgaaaa acatgtattg tgtgtaaagg ttaaataaca      2520
   aaactatgca gatgctctta aaagcattga taacctttgt gacgaacata aagagatcct      2580
   taaattatga gttgttggct tatcttcata aataatttgt ctgtaaaatg gatgaaatga      2640
   aaagaggttc aattaaaacc tacttttttc tagtgctaaa gaaaagattt aagcaccttg      2700
   tcaagctggg taaataggaa aaatacataa tcatgctcag atatgtatct aggataatta      2760
   taattaataa taatcatagt aacaatggct aatgataatt tagctttatt atatgtgcta      2820
   agcactctgg ttttacatgc attatcttct tttgttcttg ctacaaccct gtgagatagt      2880
   agtattatct cattttactg atgaagactg aagcctaggt atattaaata gcttgcccaa      2940
   ggccacacag caaaagtcac caactcaaac ctacttctta tttactccaa agcctgttgt      3000
   tcttaactgc aacatattat ttcgtctcat taatgttgat tctataggtt gttacttcta      3060
   aaaattagta ttgagtttaa tggtgaacac atttttctat tttctcttga atctgcttct      3120
   ataatgtcat ggtgatttat gtggcttttt tttttttata agttatacat gtatgcatgt      3180
   atacttatga gacctccctt ggaatgaggg aggtctcaag agatataatt tagattctca      3240
   ttgatgttct gtattcatta tcctaacacc atctgtagtg ttaaatcaac taaattattt      3300
   cagcaatagg agacaaaaca accagctttc ataattttta attgtcaaaa ccaaaaggaa      3360
   tcagaataag gatcactgag aatttaaaaa aataaaaaag gaagtaaaaa ttttaca         3417
<210> 23
   <211> 2884
   <212> DNA
   <213> Homo sapiens
<400> 23
   aaatcaggga ggcgcagctc ctacaccaac gcctttccgg ggctccgggt gtgtttgttc 60
   caactgttta aactgtttca aagcgtccga actccagcga ccttcgcaaa caactcttta 120
   tctcgcgggc gagagcgctg cccttatttg cgggggaggg caaactgaac gccggcaccg 180
   gggagctaac ggagacctcc tctaggtccc ccgcctgctg ggaccccagc tggcagtccc 240
   ttcccgcccc cggaccgcga gcttcttgcg tcagccccgg cgcgggtggg ggattttcgg 300
   aagctcagcc cgcgcggccg gcgggggaag gaagggcccg gactcttgcc ccgcccttgt 360
   ggggcgggag gcggagcggg gcaggggccc gccggcgtgt agccgattac cgagtgccgg 420
   ggagcccgga ggagccgccg acgcagccgc caccgccgcc gccgccgcca ccagagccgc 480
   cctgtccgcg ccgcgcctcg gcagccggaa cagggccgcc gtcggggagc cccaacacac 540
   ggtccacagc tcatcatgat ggacttggag ctgccgccgc cgggactccc gtcccagcag 600
   gacatggatt tgattgacat actttggagg caagatatag atcttggagt aagtcgagaa 660
   gtatttgact tcagtcagcg acggaaagag tatgagctgg aaaaacagaa aaaacttgaa 720
   aaggaaagac aagaacaact ccaaaaggag caagagaaag cctttttcgc tcagttacaa 780
   ctagatgaag agacaggtga atttctccca attcagccag cccagcacat ccagtcagaa 840
   accagtggat ctgccaacta ctcccaggtt gcccacattc ccaaatcaga tgctttgtac 900
   tttgatgact gcatgcagct tttggcgcag acattcccgt ttgtagatga caatgaggtt 960
   tcttcggcta cgtttcagtc acttgttcct gatattcccg gtcacatcga gagcccagtc 1020
   ttcattgcta ctaatcaggc tcagtcacct gaaacttctg ttgctcaggt agcccctgtt 1080
   gatttagacg gtatgcaaca ggacattgag caagtttggg aggagctatt atccattcct 1140
   gagttacagt gtcttaatat tgaaaatgac aagctggttg agactaccat ggttccaagt 1200
   ccagaagcca aactgacaga agttgacaat tatcattttt actcatctat accctcaatg 1260
   gaaaaagaag taggtaactg tagtccacat tttcttaatg cttttgagga ttccttcagc 1320
   agcatcctct ccacagaaga ccccaaccag ttgacagtga actcattaaa ttcagatgcc 1380
   acagtcaaca cagattttgg tgatgaattt tattctgctt tcatagctga gcccagtatc 1440
   agcaacagca tgccctcacc tgctacttta agccattcac tctctgaact tctaaatggg 1500
   cccattgatg tttctgatct atcactttgc aaagctttca accaaaacca ccctgaaagc 1560
   acagcagaat tcaatgattc tgactccggc atttcactaa acacaagtcc cagtgtggca 1620
   tcaccagaac actcagtgga atcttccagc tatggagaca cactacttgg cctcagtgat 1680
   tctgaagtgg aagagctaga tagtgcccct ggaagtgtca aacagaatgg tcctaaaaca 1740
   ccagtacatt cttctgggga tatggtacaa cccttgtcac catctcaggg gcagagcact 1800
   cacgtgcatg atgcccaatg tgagaacaca ccagagaaag aattgcctgt aagtcctggt 1860
   catcggaaaa ccccattcac aaaagacaaa cattcaagcc gcttggaggc tcatctcaca 1920
   agagatgaac ttagggcaaa agctctccat atcccattcc ctgtagaaaa aatcattaac 1980
   ctccctgttg ttgacttcaa cgaaatgatg tccaaagagc agttcaatga agctcaactt 2040
   gcattaattc gggatatacg taggaggggt aagaataaag tggctgctca gaattgcaga 2100
   aaaagaaaac tggaaaatat agtagaacta gagcaagatt tagatcattt gaaagatgaa 2160
   aaagaaaaat tgctcaaaga aaaaggagaa aatgacaaaa gccttcacct actgaaaaaa 2220
   caactcagca ccttatatct cgaagttttc agcatgctac gtgatgaaga tggaaaacct 2280
   tattctccta gtgaatactc cctgcagcaa acaagagatg gcaatgtttt ccttgttccc 2340
   aaaagtaaga agccagatgt taagaaaaac tagatttagg aggatttgac cttttctgag 2400
   ctagtttttt tgtactatta tactaaaagc tcctactgtg atgtgaaatg ctcatacttt 2460
   ataagtaatt ctatgcaaaa tcatagccaa aactagtata gaaaataata cgaaacttta 2520
   aaaagcattg gagtgtcagt atgttgaatc agtagtttca ctttaactgt aaacaatttc 2580
   ttaggacacc atttgggcta gtttctgtgt aagtgtaaat actacaaaaa cttatttata 2640
   ctgttcttat gtcatttgtt atattcatag atttatatga tgatatgaca tctggctaaa 2700
   aagaaattat tgcaaaacta accactatgt acttttttat aaatactgta tggacaaaaa 2760
   atggcatttt ttatattaaa ttgtttagct ctggcaaaaa aaaaaaattt taagagctgg 2820
   tactaataaa ggattattat gactgttaaa ttaaaaaaaa aaaaaaaaaa aaaaaaaaaa 2880
   aaaa 2884
<210> 24
   <211> 2771
   <212> DNA
   <213> Homo sapiens
<400> 24
   aagcccagcc ccgggacgag ggaggagcgc cttaagtgcc cagcgggctc agaagccccg 60
   acgtgtggcg gctgagccgg gccccgcgca ctttctcggc cggggagggg ttcgggctcg 120
   ggcacccgga gttggcccct cgtaacgccg cgggaaagtg cgggcgaggg cagtggactc 180
   tgaggccgga gtcggcggca cccggggctt ctagttcgga cgcggtgccc cctggtggcg 240
   ctcaccgcgc gcgtggcctt ggcttccgtg acagcgctcg gttggccgtc acagcagccc 300
   tcggttggcc ctttcctgct ttatagcgtg caaacctcgc cgcgccaggg ccaagggaca 360
   ggttggagct gttgatctgt tgcgcaattg ctattttccc cagagcggct ttgtctttgg 420
   atttagcgtt tcagaattgc aattccaaaa tgtgtaagac gggatattct cttctgtgct 480
   gtcaagggac atggatttga ttgacatact ttggaggcaa gatatagatc ttggagtaag 540
   tcgagaagta tttgacttca gtcagcgacg gaaagagtat gagctggaaa aacagaaaaa 600
   acttgaaaag gaaagacaag aacaactcca aaaggagcaa gagaaagcct ttttcgctca 660
   gttacaacta gatgaagaga caggtgaatt tctcccaatt cagccagccc agcacatcca 720
   gtcagaaacc agtggatctg ccaactactc ccaggttgcc cacattccca aatcagatgc 780
   tttgtacttt gatgactgca tgcagctttt ggcgcagaca ttcccgtttg tagatgacaa 840
   tgaggtttct tcggctacgt ttcagtcact tgttcctgat attcccggtc acatcgagag 900
   cccagtcttc attgctacta atcaggctca gtcacctgaa acttctgttg ctcaggtagc 960
   ccctgttgat ttagacggta tgcaacagga cattgagcaa gtttgggagg agctattatc 1020
   cattcctgag ttacagtgtc ttaatattga aaatgacaag ctggttgaga ctaccatggt 1080
   tccaagtcca gaagccaaac tgacagaagt tgacaattat catttttact catctatacc 1140
   ctcaatggaa aaagaagtag gtaactgtag tccacatttt cttaatgctt ttgaggattc 1200
   cttcagcagc atcctctcca cagaagaccc caaccagttg acagtgaact cattaaattc 1260
   agatgccaca gtcaacacag attttggtga tgaattttat tctgctttca tagctgagcc 1320
   cagtatcagc aacagcatgc cctcacctgc tactttaagc cattcactct ctgaacttct 1380
   aaatgggccc attgatgttt ctgatctatc actttgcaaa gctttcaacc aaaaccaccc 1440
   tgaaagcaca gcagaattca atgattctga ctccggcatt tcactaaaca caagtcccag     1500
   tgtggcatca ccagaacact cagtggaatc ttccagctat ggagacacac tacttggcct     1560
   cagtgattct gaagtggaag agctagatag tgcccctgga agtgtcaaac agaatggtcc     1620
   taaaacacca gtacattctt ctggggatat ggtacaaccc ttgtcaccat ctcaggggca     1680
   gagcactcac gtgcatgatg cccaatgtga gaacacacca gagaaagaat tgcctgtaag     1740
   tcctggtcat cggaaaaccc cattcacaaa agacaaacat tcaagccgct tggaggctca     1800
   tctcacaaga gatgaactta gggcaaaagc tctccatatc ccattccctg tagaaaaaat     1860
   cattaacctc cctgttgttg acttcaacga aatgatgtcc aaagagcagt tcaatgaagc     1920
   tcaacttgca ttaattcggg atatacgtag gaggggtaag aataaagtgg ctgctcagaa     1980
   ttgcagaaaa agaaaactgg aaaatatagt agaactagag caagatttag atcatttgaa     2040
   agatgaaaaa gaaaaattgc tcaaagaaaa aggagaaaat gacaaaagcc ttcacctact     2100
   gaaaaaacaa ctcagcacct tatatctcga agttttcagc atgctacgtg atgaagatgg     2160
   aaaaccttat tctcctagtg aatactccct gcagcaaaca agagatggca atgttttcct     2220
   tgttcccaaa agtaagaagc cagatgttaa gaaaaactag atttaggagg atttgacctt     2280
   ttctgagcta gtttttttgt actattatac taaaagctcc tactgtgatg tgaaatgctc     2340
   atactttata agtaattcta tgcaaaatca tagccaaaac tagtatagaa aataatacga     2400
   aactttaaaa agcattggag tgtcagtatg ttgaatcagt agtttcactt taactgtaaa     2460
   caatttctta ggacaccatt tgggctagtt tctgtgtaag tgtaaatact acaaaaactt     2520
   atttatactg ttcttatgtc atttgttata ttcatagatt tatatgatga tatgacatct     2580
   ggctaaaaag aaattattgc aaaactaacc actatgtact tttttataaa tactgtatgg     2640
   acaaaaaatg gcatttttta tattaaattg tttagctctg gcaaaaaaaa aaaattttaa     2700
   gagctggtac taataaagga ttattatgac tgttaaatta aaaaaaaaaa aaaaaaaaaa     2760
   aaaaaaaaaa a                                                          2771
<210> 25
   <211> 2750
   <212> DNA
   <213> Homo sapiens
<400> 25
   aagcccagcc ccgggacgag ggaggagcgc cttaagtgcc cagcgggctc agaagccccg      60
   acgtgtggcg gctgagccgg gccccgcgca ctttctcggc cggggagggg ttcgggctcg     120
   ggcacccgga gttggcccct cgtaacgccg cgggaaagtg cgggcgaggg cagtggactc     180
   tgaggccgga gtcggcggca cccggggctt ctagttcgga cgcggtgccc cctggtggcg     240
   ctcaccgcgc gcgtggcctt ggcttccgtg acagcgctcg gttggccgtc acagcagccc     300
   tcggttggcc ctttcctgct ttatagcgtg caaacctcgc cgcgccaggg ccaagggaca     360
   ggttggagct gttgatctgt tgcgcaattg ctattttccc cagagcggct ttgtctttgg 420
   atttagcgtt tcagaattgc aattccaaaa tgtgtaagac gggatattct cttctgtgct 480
   gtcaagggac atggatttga ttgacatact ttggaggcaa gatatagatc ttggagtaag 540
   tcgagaagta tttgacttca gtcagcgacg gaaagagtat gagctggaaa aacagaaaaa 600
   acttgaaaag gaaagacaag aacaactcca aaaggagcaa gagaaagcct ttttcgctca 660
   gttacaacta gatgaagaga caggtgaatt tctcccaatt cagccagccc agcacatcca 720
   gtcagaaacc agtggatctg ccaactactc ccaggttgcc cacattccca aatcagatgc 780
   tttgtacttt gatgactgca tgcagctttt ggcgcagaca ttcccgtttg tagatgacaa 840
   tgagtcactt gttcctgata ttcccggtca catcgagagc ccagtcttca ttgctactaa 900
   tcaggctcag tcacctgaaa cttctgttgc tcaggtagcc cctgttgatt tagacggtat 960
   gcaacaggac attgagcaag tttgggagga gctattatcc attcctgagt tacagtgtct 1020
   taatattgaa aatgacaagc tggttgagac taccatggtt ccaagtccag aagccaaact 1080
   gacagaagtt gacaattatc atttttactc atctataccc tcaatggaaa aagaagtagg 1140
   taactgtagt ccacattttc ttaatgcttt tgaggattcc ttcagcagca tcctctccac 1200
   agaagacccc aaccagttga cagtgaactc attaaattca gatgccacag tcaacacaga 1260
   ttttggtgat gaattttatt ctgctttcat agctgagccc agtatcagca acagcatgcc 1320
   ctcacctgct actttaagcc attcactctc tgaacttcta aatgggccca ttgatgtttc 1380
   tgatctatca ctttgcaaag ctttcaacca aaaccaccct gaaagcacag cagaattcaa 1440
   tgattctgac tccggcattt cactaaacac aagtcccagt gtggcatcac cagaacactc 1500
   agtggaatct tccagctatg gagacacact acttggcctc agtgattctg aagtggaaga 1560
   gctagatagt gcccctggaa gtgtcaaaca gaatggtcct aaaacaccag tacattcttc 1620
   tggggatatg gtacaaccct tgtcaccatc tcaggggcag agcactcacg tgcatgatgc 1680
   ccaatgtgag aacacaccag agaaagaatt gcctgtaagt cctggtcatc ggaaaacccc 1740
   attcacaaaa gacaaacatt caagccgctt ggaggctcat ctcacaagag atgaacttag 1800
   ggcaaaagct ctccatatcc cattccctgt agaaaaaatc attaacctcc ctgttgttga 1860
   cttcaacgaa atgatgtcca aagagcagtt caatgaagct caacttgcat taattcggga 1920
   tatacgtagg aggggtaaga ataaagtggc tgctcagaat tgcagaaaaa gaaaactgga 1980
   aaatatagta gaactagagc aagatttaga tcatttgaaa gatgaaaaag aaaaattgct 2040
   caaagaaaaa ggagaaaatg acaaaagcct tcacctactg aaaaaacaac tcagcacctt 2100
   atatctcgaa gttttcagca tgctacgtga tgaagatgga aaaccttatt ctcctagtga 2160
   atactccctg cagcaaacaa gagatggcaa tgttttcctt gttcccaaaa gtaagaagcc 2220
   agatgttaag aaaaactaga tttaggagga tttgaccttt tctgagctag tttttttgta 2280
   ctattatact aaaagctcct actgtgatgt gaaatgctca tactttataa gtaattctat 2340
   gcaaaatcat agccaaaact agtatagaaa ataatacgaa actttaaaaa gcattggagt 2400
   gtcagtatgt tgaatcagta gtttcacttt aactgtaaac aatttcttag gacaccattt 2460
   gggctagttt ctgtgtaagt gtaaatacta caaaaactta tttatactgt tcttatgtca 2520
   tttgttatat tcatagattt atatgatgat atgacatctg gctaaaaaga aattattgca 2580
   aaactaacca ctatgtactt ttttataaat actgtatgga caaaaaatgg cattttttat 2640
   attaaattgt ttagctctgg caaaaaaaaa aaattttaag agctggtact aataaaggat 2700
   tattatgact gttaaattaa aaaaaaaaaa aaaaaaaaaa aaaaaaaaaa 2750
<210> 26
   <211> 2601
   <212> DNA
   <213> Homo sapiens
<400> 26
   ccgcccttgt aggctgtcca cctcaaacgg gccggacagg atatataaga gagaatgcac      60
   cgtgcactac acacgcgact cccacaaggt tgcagccgga gccgcccagc tcaccgagag     120
   cctagttccg gccagggtcg ccccggcaac cacgagccca gccaatcagc gccccggact     180
   gcaccagagc catggtcggc agaagagcac tgatcgtact ggctcactca gagaggacgt     240
   ccttcaacta tgccatgaag gaggctgctg cagcggcttt gaagaagaaa ggatgggagg     300
   tggtggagtc ggacctctat gccatgaact tcaatcccat catttccaga aaggacatca     360
   caggtaaact gaaggaccct gcgaactttc agtatcctgc cgagtctgtt ctggcttata     420
   aagaaggcca tctgagccca gatattgtgg ctgaacaaaa gaagctggaa gccgcagacc     480
   ttgtgatatt ccagttcccc ctgcagtggt ttggagtccc tgccattctg aaaggctggt     540
   ttgagcgagt gttcatagga gagtttgctt acacttacgc tgccatgtat gacaaaggac     600
   ccttccggag taagaaggca gtgctttcca tcaccactgg tggcagtggc tccatgtact     660
   ctctgcaagg gatccacggg gacatgaatg tcattctctg gccaattcag agtggcattc     720
   tgcatttctg tggcttccaa gtcttagaac ctcaactgac atatagcatt gggcacactc     780
   cagcagacgc ccgaattcaa atcctggaag gatggaagaa acgcctggag aatatttggg     840
   atgagacacc actgtatttt gctccaagca gcctctttga cctaaacttc caggcaggat     900
   tcttaatgaa aaaagaggta caggatgagg agaaaaacaa gaaatttggc ctttctgtgg     960
   gccatcactt gggcaagtcc atcccaactg acaaccagat caaagctaga aaatgagatt    1020
   ccttagcctg gatttccttc taacatgtta tcaaatctgg gtatctttcc aggcttccct    1080
   gacttgcttt agtttttaag atttgtgttt ttctttttcc acaaggaata aatgagaggg    1140
   aatcgactgt attcgtgcat ttttggatca tttttaactg attcttatga ttactatcat    1200
   ggcatataac caaaatccga ctgggctcaa gaggccactt agggaaagat gtagaaagat    1260
   gctagaaaaa tgttctttaa aggcatctac acaatttaat tcctcttttt agggctaaag 1320
   ttttagggta cagtttggct aggtatcatt caactctcca atgttctatt aatcacctct 1380
   ctgtagttta tggcagaagg gaattgctca gagaaggaaa agactgaatc tacctgccct 1440
   aagggactta acttgtttgg tagttagcca tctaatgctt gtttatgata tttcttgctt 1500
   tcaattacaa agcagttact aatatgccta gcacaagtac cactcttggt cagcttttgt 1560
   tgtttatata cagtacacag ataccttgaa aggaagagct aataaatctc ttctttgctg 1620
   cagtcatcta cttttttttt aattaaaaaa aatttttttt tgaagcagtc ttgctctgtt 1680
   acccaggctg gagtgcagtg gtgtgatctc ggctcactgc aacctctgcc tcccaggttc 1740
   cagcaattct cctgcctcag cctccctagt agctgggatg acaggcgcct gccatcatgc 1800
   ctgactaatt tttgtatttt tagtagagac ggcgtttcac catgttggcc aggctggtct 1860
   caaactcctg acctcaggtg atccgcctac ctcagcctcc caaagtgctg ggattacagg 1920
   cgtgatccac cacacctggc ccttgcaatc ttctacttta aggtttgcag agataaacca 1980
   ataaatccac accgtacatc tgcaatatga attcaagaaa ggaaatagta ccttcaatac 2040
   ttaaaaatag tcttccacaa aaaatacttt atttctgatc tatacaaatt ttcagaaggt 2100
   tattttcttt atcattgcta aactgatgac ttactatggg atggggtcca gtcccatgac 2160
   cttggggtac aattgtaaac ctagagtttt atcaactttg gtgaacagtt ttggcataat 2220
   agtcaatttc tacttctgga agtcatctca ttccactgtt ggtattatat aattcaagga 2280
   gaatatgata aaacactgcc ctcttgtggt gcattgaaag aagagatgag aaatgatgaa 2340
   aaggttgcct gaaaaatggg agacagcctc ttacttgcca agaaaatgaa gggattggac 2400
   cgagctggaa aacctccttt accagatgct gactggcact ggtggttttt gctctcgaca 2460
   gtatccacaa tagctgacgg ctgggtgttt cagtttgaaa atattttgtt gccttcatct 2520
   tcactgcaat tttgtgtaaa tttctcaaag atctgaatta aataaataaa attcatttct 2580
   acagacccac aaaaaaaaaa a 2601
<210> 27
   <211> 2499
   <212> DNA
   <213> Homo sapiens
<400> 27
   ccgcccttgt aggctgtcca cctcaaacgg gccggacagg atatataaga gagaatgcac        60
   cgtgcactac acacgcgact cccacaaggt tgcagccgga gccgcccagc tcaccgagag       120
   cctagttccg gccagggtcg ccccggcaac cacgagccca gccaatcagc gccccggact       180
   gcaccagagc catggtcggc agaagagcac tgatcgtact ggctcactca gagaggacgt       240
   ccttcaacta tgccatgaag gaggctgctg cagcggcttt gaagaagaaa ggatgggagg       300
   tggtggagtc ggacctctat gccatgaact tcaatcccat catttccaga aaggacatca       360
   caggtaaact gaaggaccct gcgaactttc agtatcctgc cgagtctgtt ctggcttata 420
   aagaaggcca tctgagccca gatattgtgg ctgaacaaaa gaagctggaa gccgcagacc 480
   ttgtgatatt ccagttcccc ctgcagtggt ttggagtccc tgccattctg aaaggctggt 540
   ttgagcgagt gttcatagga gagtttgctt acacttacgc tgccatgtat gacaaaggac 600
   ccttccggag tggcattctg catttctgtg gcttccaagt cttagaacct caactgacat 660
   atagcattgg gcacactcca gcagacgccc gaattcaaat cctggaagga tggaagaaac 720
   gcctggagaa tatttgggat gagacaccac tgtattttgc tccaagcagc ctctttgacc 780
   taaacttcca ggcaggattc ttaatgaaaa aagaggtaca ggatgaggag aaaaacaaga 840
   aatttggcct ttctgtgggc catcacttgg gcaagtccat cccaactgac aaccagatca 900
   aagctagaaa atgagattcc ttagcctgga tttccttcta acatgttatc aaatctgggt 960
   atctttccag gcttccctga cttgctttag tttttaagat ttgtgttttt ctttttccac 1020
   aaggaataaa tgagagggaa tcgactgtat tcgtgcattt ttggatcatt tttaactgat 1080
   tcttatgatt actatcatgg catataacca aaatccgact gggctcaaga ggccacttag 1140
   ggaaagatgt agaaagatgc tagaaaaatg ttctttaaag gcatctacac aatttaattc 1200
   ctctttttag ggctaaagtt ttagggtaca gtttggctag gtatcattca actctccaat 1260
   gttctattaa tcacctctct gtagtttatg gcagaaggga attgctcaga gaaggaaaag 1320
   actgaatcta cctgccctaa gggacttaac ttgtttggta gttagccatc taatgcttgt 1380
   ttatgatatt tcttgctttc aattacaaag cagttactaa tatgcctagc acaagtacca 1440
   ctcttggtca gcttttgttg tttatataca gtacacagat accttgaaag gaagagctaa 1500
   taaatctctt ctttgctgca gtcatctact ttttttttaa ttaaaaaaaa tttttttttg 1560
   aagcagtctt gctctgttac ccaggctgga gtgcagtggt gtgatctcgg ctcactgcaa 1620
   cctctgcctc ccaggttcca gcaattctcc tgcctcagcc tccctagtag ctgggatgac 1680
   aggcgcctgc catcatgcct gactaatttt tgtattttta gtagagacgg cgtttcacca 1740
   tgttggccag gctggtctca aactcctgac ctcaggtgat ccgcctacct cagcctccca 1800
   aagtgctggg attacaggcg tgatccacca cacctggccc ttgcaatctt ctactttaag 1860
   gtttgcagag ataaaccaat aaatccacac cgtacatctg caatatgaat tcaagaaagg 1920
   aaatagtacc ttcaatactt aaaaatagtc ttccacaaaa aatactttat ttctgatcta 1980
   tacaaatttt cagaaggtta ttttctttat cattgctaaa ctgatgactt actatgggat 2040
   ggggtccagt cccatgacct tggggtacaa ttgtaaacct agagttttat caactttggt 2100
   gaacagtttt ggcataatag tcaatttcta cttctggaag tcatctcatt ccactgttgg 2160
   tattatataa ttcaaggaga atatgataaa acactgccct cttgtggtgc attgaaagaa 2220
   gagatgagaa atgatgaaaa ggttgcctga aaaatgggag acagcctctt acttgccaag 2280
   aaaatgaagg gattggaccg agctggaaaa cctcctttac cagatgctga ctggcactgg 2340
   tggtttttgc tctcgacagt atccacaata gctgacggct gggtgtttca gtttgaaaat 2400
   attttgttgc cttcatcttc actgcaattt tgtgtaaatt tctcaaagat ctgaattaaa 2460
   taaataaaat tcatttctac agacccacaa aaaaaaaaa 2499
<210> 28
   <211> 2487
   <212> DNA
   <213> Homo sapiens
<400> 28
   ccgcccttgt aggctgtcca cctcaaacgg gccggacagg atatataaga gagaatgcac      60
   cgtgcactac acacgcgact cccacaaggt tgcagccgga gccgcccagc tcaccgagag     120
   cctagttccg gccagggtcg ccccggcaac cacgagccca gccaatcagc gccccggact     180
   gcaccagagc catggtcggc agaagagcac tgatcgtact ggctcactca gagaggacgt     240
   ccttcaacta tgccatgaag gaggctgctg cagcggcttt gaagaagaaa ggatgggagg     300
   tggtggagtc ggacctctat gccatgaact tcaatcccat catttccaga aaggacatca     360
   caggtaaact gaaggaccct gcgaactttc agtatcctgc cgagtctgtt ctggcttata     420
   aagaaggcca tctgagccca gatattgtgg ctgaacaaaa gaagctggaa gccgcagacc     480
   ttgtgatatt ccagagtaag aaggcagtgc tttccatcac cactggtggc agtggctcca     540
   tgtactctct gcaagggatc cacggggaca tgaatgtcat tctctggcca attcagagtg     600
   gcattctgca tttctgtggc ttccaagtct tagaacctca actgacatat agcattgggc     660
   acactccagc agacgcccga attcaaatcc tggaaggatg gaagaaacgc ctggagaata     720
   tttgggatga gacaccactg tattttgctc caagcagcct ctttgaccta aacttccagg     780
   caggattctt aatgaaaaaa gaggtacagg atgaggagaa aaacaagaaa tttggccttt     840
   ctgtgggcca tcacttgggc aagtccatcc caactgacaa ccagatcaaa gctagaaaat     900
   gagattcctt agcctggatt tccttctaac atgttatcaa atctgggtat ctttccaggc     960
   ttccctgact tgctttagtt tttaagattt gtgtttttct ttttccacaa ggaataaatg     1020
   agagggaatc gactgtattc gtgcattttt ggatcatttt taactgattc ttatgattac    1080
   tatcatggca tataaccaaa atccgactgg gctcaagagg ccacttaggg aaagatgtag    1140
   aaagatgcta gaaaaatgtt ctttaaaggc atctacacaa tttaattcct ctttttaggg    1200
   ctaaagtttt agggtacagt ttggctaggt atcattcaac tctccaatgt tctattaatc    1260
   acctctctgt agtttatggc agaagggaat tgctcagaga aggaaaagac tgaatctacc    1320
   tgccctaagg gacttaactt gtttggtagt tagccatcta atgcttgttt atgatatttc    1380
   ttgctttcaa ttacaaagca gttactaata tgcctagcac aagtaccact cttggtcagc    1440
   ttttgttgtt tatatacagt acacagatac cttgaaagga agagctaata aatctcttct    1500
   ttgctgcagt catctacttt ttttttaatt aaaaaaaatt tttttttgaa gcagtcttgc    1560
   tctgttaccc aggctggagt gcagtggtgt gatctcggct cactgcaacc tctgcctccc     1620
   aggttccagc aattctcctg cctcagcctc cctagtagct gggatgacag gcgcctgcca     1680
   tcatgcctga ctaatttttg tatttttagt agagacggcg tttcaccatg ttggccaggc      1740
   tggtctcaaa ctcctgacct caggtgatcc gcctacctca gcctcccaaa gtgctgggat     1800
   tacaggcgtg atccaccaca cctggccctt gcaatcttct actttaaggt ttgcagagat     1860
   aaaccaataa atccacaccg tacatctgca atatgaattc aagaaaggaa atagtacctt     1920
   caatacttaa aaatagtctt ccacaaaaaa tactttattt ctgatctata caaattttca     1980
   gaaggttatt ttctttatca ttgctaaact gatgacttac tatgggatgg ggtccagtcc     2040
   catgaccttg gggtacaatt gtaaacctag agttttatca actttggtga acagttttgg     2100
   cataatagtc aatttctact tctggaagtc atctcattcc actgttggta ttatataatt     2160
   caaggagaat atgataaaac actgccctct tgtggtgcat tgaaagaaga gatgagaaat     2220
   gatgaaaagg ttgcctgaaa aatgggagac agcctcttac ttgccaagaa aatgaaggga     2280
   ttggaccgag ctggaaaacc tcctttacca gatgctgact ggcactggtg gtttttgctc     2340
   tcgacagtat ccacaatagc tgacggctgg gtgtttcagt ttgaaaatat tttgttgcct     2400
   tcatcttcac tgcaattttg tgtaaatttc tcaaagatct gaattaaata aataaaattc     2460
   atttctacag acccacaaaa aaaaaaa                                         2487
<210> 29
   <211> 1039
   <212> DNA
   <213> Homo sapiens
<400> 29
   gctcgtccgc tccctccccc gcgccgtgca cgtcttggtt cgggccgggc ataaaaggct       60
   tcgcggccca gggctcactt ggcgctgaga acgcgggtcc acgcgtgtga tcgtccgtgc      120
   gtctagcctt tgcccacgca gctttcagtc atggcctccg gtaacgcgcg catcggaaag      180
   ccagcccctg acttcaaggc cacagcggtg gttgatggcg ccttcaaaga ggtgaagctg      240
   tcggactaca aagggaagta cgtggtcctc tttttctacc ctctggactt cacttttgtg      300
   tgccccaccg agatcatcgc gttcagcaac cgtgcagagg acttccgcaa gctgggctgt      360
   gaagtgctgg gcgtctcggt ggactctcag ttcacccacc tggcttggat caacaccccc      420
   cggaaagagg gaggcttggg ccccctgaac atccccctgc ttgctgacgt gaccagacgc      480
   ttgtctgagg attacggcgt gctgaaaaca gatgagggca ttgcctacag gggcctcttt      540
   atcatcgatg gcaagggtgt ccttcgccag atcactgtta atgatttgcc tgtgggacgc      600
   tccgtggatg aggctctgcg gctggtccag gccttccagt acacagacga gcatggggaa      660
   gtttgtcccg ctggctggaa gcctggcagt gacacgatta agcccaacgt ggatgacagc      720
   aaggaatatt tctccaaaca caattaggct ggctaacgga tagtgagctt gtgcccctgc      780
   ctaggtgcct gtgctgggtg tccacctgtg cccccacctg ggtgccctat gctgacccag      840
   gaaaggccag acctgcccct ccaaactcca cagtatggga ccctggaggg ctaggccaag      900
   gccttctcat gcctccacct agaagctgaa tagtgacgcc ctcccccaag cccacccagc      960
   cgcacacagg cctagaggta accaataaag tattagggaa aggtgtgaaa aaaaaaaaaa     1020
   aaaaaaaaaa aaaaaaaaa                                                  1039
<210> 30
   <211> 710
   <212> DNA
   <213> Homo sapiens
<400> 30
   gctcgtccgc tccctccccc gcgccgtgca cgtcttggtt cgggccgggc ataaaaggct      60
   tcgcggccca gggctcactt ggcgctgaga acgcgggtcc acgcgtgtga tcgtccgtgc     120
   gtctagcctt tgcccacgca gctttcagtc atggcctccg gtaacgcgcg catcggaaag     180
   ccagcccctg acttcaaggc cacagcggtg gttgatggcg ccttcaaaga ggtgaagctg     240
   tcggactaca aagggaagta cgtggtcctc tttttctacc ctctggactt cacttttgtg     300
   tgccccaccg agatcatcgc gttcagcaac cgtgcagagg acttccgcaa gctgggctgt     360
   gaagtgctgg gcgtctcggt ggactctcag ttcacccacc tggcttggta tgagcagggg     420
   ccaaagaggg aggttgcagc taagctcaca ccctcaggtc ctagcagtgt ggcttcgtgg     480
   ccattgctca acctctggaa cctgcgtttc cccatcgtga aaataatgga aacattgccg     540
   cccaagtctt taaggatgat gacagtaatt agcatttgac aactagttgc ctggtatata     600
   gagttgcaga tgcaactcag atgcaactct atctactcta tgtacttagt tcccaggagg     660
   gaggctgtgc tgccctattt catgaagatg gaaactccag ttcaccgaag                710
<210> 31
   <211> 6953
   <212> DNA
   <213> Homo sapiens
<400> 31
   ggcggctgtc gcggggaccg gaggcggagg ctgcagcggg cactgcggcg ccgaggcgcg      60
   gcggcaggac gggaaccacg cgagctgcag ccagctaggt cttgactcca ctaacgggga     120
   cttctcatac ggctttgaac ttggaccttg gtgcataagc aaatgtaact cagcgaattg     180
   taatggaaag catatggaca ggtctacgcc acatcactta ggtgctaaga gatgccgttt     240
   gcaggcctca tctctgcttg agaatcgggg gctccaggca ctagaagagc cggctgatcc     300
   tgggctccta gcttgaataa gccttcactt ccagctgctc tccccaacgg ctgtgtaaac     360
   tactcgtttc tcttaatgat gggtattggt aagaatacca catccaaatc aatggaggct     420
   ggaagttcaa cagaaggcaa atacgaagac gaggcaaagc acccagcttt cttcactctt     480
   ccggtggtga taaatggagg cgccacctcc agcggtgagc aggacaatga ggacactgag      540
   ctcatggcga tctacactac ggaaaacggc attgcagaaa agagctctct cgctgagacc      600
   ctggatagca ctggcagtct ggacccccag cgatcagaca tgatttatac catagaagat      660
   gttcctccct ggtacctgtg tatatttctg gggctacagc actacctgac atgcttcagc       720
   ggcacgatcg cagtgccctt cctgttggcc gatgccatgt gtgtggggta cgaccagtgg      780
   gccaccagcc agctcattgg gaccattttc ttctgtgtgg gaatcactac tttgctacag      840
   acaacgtttg gatgcaggtt acccctgttt caggccagtg cttttgcatt tttggcccct      900
   gctcgagcca tcctgtcttt agataaatgg aaatgtaaca ccacagatgt ttcagttgcc      960
   aatggaacag cagagctgtt gcacacagaa cacatctggt atccccggat ccgagagatc     1020
   cagggggcca tcatcatgtc ctcactgata gaagtagtca tcggcctcct cggcctgcct    1080
   ggggctctac tgaagtacat cggtcccttg accattacac ccacggtggc cctaattggc     1140
   ctctctggtt tccaggcagc gggggagaga gccgggaagc actggggcat tgccatgctg     1200
   acaatattcc tagtattact gttttctcaa tacgccagaa atgttaaatt tcctctcccg     1260
   atttataaat ccaagaaagg atggactgcg tacaagttac agctgttcaa aatgttccct     1320
   atcatcctgg ccatcctggt atcctggctg ctctgcttca tcttcacggt gacagatgtc     1380
   ttccctcccg acagcacaaa gtatggcttc tatgctcgca cagatgccag gcaaggcgtg     1440
   cttctggtag ccccgtggtt taaggttcca tacccatttc agtggggact gcccaccgtg     1500
   tctgcggccg gtgtcatcgg catgctcagt gccgtggtcg ccagcatcat cgagtctatt     1560
   ggtgactact acgcctgtgc acggctgtcc tgtgccccac ccccccccat ccacgcaata     1620
   aacaggggaa ttttcgtgga aggcctctcc tgtgttcttg atggcatatt tggtactggg     1680
   aatggctcta cttcatccag tcccaacatt ggagttttgg gaattacaaa ggtcggcagc     1740
   cgccgcgtga tacagtgcgg agcagccctc atgctcgctc tgggcatgat cgggaagttc     1800
   agcgccctct ttgcgtccct tccggatcct gtgctgggag ccctgttctg cacgctcttt     1860
   ggaatgatca cagctgttgg cctctctaac ctgcagttca ttgatttaaa ttcttcccgg     1920
   aacctctttg tgcttggatt ttcgatcttc tttgggctcg tccttccaag ttacctcaga     1980
   cagaaccctc tggtcacagg gataacagga atcgatcaag tgttgaacgt ccttctcaca     2040
   actgctatgt ttgtaggggg ctgtgtggct tttatcctgg ataacaccat cccaggcact     2100
   ccagaggaaa gaggaatccg gaaatggaag aagggtgtgg gcaaagggaa caaatcactc     2160
   gacggcatgg agtcgtacaa tttgccattt ggcatgaaca ttataaaaaa atacagatgc     2220
   ttcagctact tacccatcag cccaaccttt gtgggctaca catggaaagg cctcaggaag     2280
   agcgacaaca gccggagttc agatgaagac tcccaggcca cgggatagcc tttgctgtgc     2340
   cctgtggcct ggccgcagtg aggcatgtat ctgtagttcc ttgctgagta acaagaagta     2400
   aaatacatgt ttgtatatct acatttacat tctgcgcccc agagctaaga cagactgcga     2460
   atcacttttc tgctgtgagg gtgatggtgc ctgatgcggt gtctcgccta tctccttatt 2520
   taagccctta actccttgcc ctttgagagt gtccattgct ggccatggtc actaaatttg 2580
   aagttccagt cctccctttg gagtggacat ttaaaatctg cagccattcc ttgcttcagt 2640
   tcccttccac cacccagggc tggctttcct ataagccacc tccagggtcc aggggctttc 2700
   tctctctctc cgaagcccct ggacctgttt ccttgtcatt tgaccctcgt ctgtgcccgc 2760
   ggagacctcc tctgaccaag gggcactagt atgccttgcg ctatcatgac tgactggccc 2820
   agtatgattc tgttgtcgct gatctcccgg aggaggaggc tgttgacctg aaaacagtgg 2880
   cgtggctccc ctgtgcccag atctggccat gggtgaggcc tgcacagatg ttgaatgaac 2940
   atctgtttgg ggcattggag ggaaaaggaa aggaccctga agccaatatc tgattctcgg 3000
   ctgcaccttc tcttggccac cctggccctg accgggccag cacgtgcaca ctggcctctt 3060
   ttcatttcac tagtggccga gacaccctct ctggcattta tcactgttgg ccggtgtgat 3120
   gagtatagag tgccacccaa ggcaccagtc acacaagctg atgggcgtct tggctctgcc 3180
   gtcatgtgga tgttattgtc gcgcgcgtct ataattacaa ggttttattc ctatttaatg 3240
   ttattgacta tagcagattt tggaaatcag tgttttccat gtgaccctct ttccttgcat 3300
   ccctattcct tgtcccccac ccactcttcc ccccatcaag aaaagaacca gcatttgtaa 3360
   agctgtggac accatcaggg aagcttgttg taacggcttt tgaaggccag taaccattgt 3420
   tgtggttgtg ttttgtattg cttgatacca tgaaagtgta aatactgtaa tgcctaatct 3480
   atttatcaaa actgactact ggaccggagc ccagaaacca tgggtcaagt tacacgtgaa 3540
   tttgttttgt gaagaaggga agctggggca ggtaacacgc agagccgcca cgtggaacgg 3600
   tctgtccgcc ggtctgtccc gcttgccggg cttctgttgc aagtcttggc ttaaggagac 3660
   ttcctgtggg ttgccatgtc gcacgtccgt tagatcttga ttttacaggt gagggtggtt 3720
   gccaaaggtg ataagaaaag cagccaacag gctccctctc acttaagaga gttctaatta 3780
   ataacactaa attcttcctg agaaatgact cctcttggtt tggtttgaag tttttcagtg 3840
   aaggaaaagg cctgagatag gaagcagtgc cctcgcttca tgaggtggca catcttcggt 3900
   ggccgttggc ctggtcgagc tttggtcagc tgcgtgggtg cctgtctcct gtctccagcg 3960
   gcagggcatg gttcttggca gcctggtgtc cacacggtgc attcctgtcc ccctcggccc 4020
   caggctggct cagctctcca gtgctgcggc gctgtctttg cagcattgcc atgtcaccgt 4080
   agcccccttc actcttggaa gtttggggct tcaaggttta ttctggcaag aacacctcgg 4140
   agccactgca ttgttttgcc aaaaacttac tgcagattaa gtaaagaact tagatgctaa 4200
   attacatcta attttgtatg tttttttaaa tgtgtctttg atgcatactt tagaagcaca 4260
   ctggctcgcg gtttccatcc ctcttgcctt gggacactct tgaggctggc agccctcctg 4320
   caatgagttg ctctcagttc cttctgtttg gattttgctc aacatctcca gtttctctgc 4380
   tctgaaaaat ggggaaaact gctaaacatc ccagagattt gcaatcttct agctctccag 4440
   gtgagcagaa agaccaaata tccaaataaa agcctgaaag ccattggtga tccaggggca 4500
   ccttctcttc agcttgactg ggcgtcttcc gctgcataac actcaaggtg tctgctctag 4560
   tcatccagaa acccactccc ttctgaagcc cctccttggg ccgggttgta tcaggaggat 4620
   tcaaccccgc taacatgtgg ggtgccctct gtaggccttc acacagggaa ggaagcccta 4680
   ccctttcaac tcctgttgga gtgagagttt cctgttctga ttccactgtg gccaacccaa 4740
   taattctgtc cgaggagggg taagggaata ggaggtgggg agcaaagagg gcagcctgac 4800
   tagatgcccc tgctgaatct ggtactttct gcacagcctt gagtgattgg agtcccctaa 4860
   tgactaataa acttgaacat gtaacacaca cacacacaca cacacacacc attttaggaa 4920
   gtggttctcc tactggaaaa tatgctacag aatgaaagtt tgtaattgaa agaaaaagaa 4980
   tgtattcatc tgcttcttgg gtttaagcac acacctgaag gggagatatt gggggacacg 5040
   gtggccactc gcggtctgtg tcatgacact tcccattcac atcattggcc tgcgttgctg 5100
   gggctttgaa ccaccacccc gtactttgtt ttgcctgtag agtgttgtaa aatattttgg 5160
   atgtgaatgt ccttgcaggc cggttagtct ccagtttcca cagagaccac ctacccacca 5220
   ccttcccagg tcacctgcct ggtccctgaa agaaagcatt tgtgtgggca atcccaacat 5280
   gaaatgtaga cagcgtgaat acggacagga ggtgatgcca tattcacagt gggggatgga 5340
   gggcccccac ccctgcttca cagaataatg ggaagtgact ctagtctttg taagcaattt 5400
   tcattctcat taggaagtta aatgtagatt cacagataca tttactttca gaatcctcaa 5460
   gaggatttca aatgctaact tttagtttct tgggattgat caaggcaaga gaagtggctt 5520
   taaccatgcc agccagaatg aagcatcatg ggtcctgatg gtttcccgtt ttcatggtaa 5580
   caaattcgca cagaggtttt gctttatgct gtagaatacc gactttcatg atgacaactt 5640
   ggtttatgca tgaatattgc agtatttcca tatagcaaaa taacatttct acccaggata 5700
   atagtcacag ttttccaggg agggaatgat ataattttca tgacaatgtc atgtgatgat 5760
   agaatttctc cattgatgaa tctctagtat gtgtgtatac tttatttacc catacgtata 5820
   ttttatgacc atgagctaag ttgttatttc tttcattcat atccctgctg aaatattggt 5880
   actagcaact taaagttggc aagtctcatc ttcagtaata cggatgccat gggagtggcc 5940
   aggccagatt gaaggtgaat atggagcagt agcagaagcc tcattccaga acccatctgg 6000
   ccagagaagc agcagcatcc tgggggatgg ccgtgcatgg ggtgtacact cgctataggc 6060
   ataggcccgg catggctgtc gctggacgcc agctgtgcac acccagccac acctgctgca 6120
   cgccgcgtta gtgtgcggct ccgggcctga gcattcgcaa agctcgcttc tccagggagc 6180
   ctcctcttgg ctttggaaaa gagcccaggg acttaacgtg ctgcctgttg tactctgtcc 6240
   cctcatgact ttttagacac agggactagg acatcgacca ccctctgccc ctcctgcagt 6300
   cagaacccga gtagtgcaag aacggccacc cggttttgcc agcgttcagc gtctgcccat 6360
   ggacccataa ggcacattag cgttgggggt ctcttttcag cagcctcaca gacattcccg    6420
   tcctttctgg ttggtgttga ctgtgtttca gcatcactct accctcccat gtccttagca    6480
   ttgctgtgcg tgtttccagg cacttaggac acccttcccc acgttgccat cttactgaca    6540
   gttcaaatga ctctcctttt ttgttcgtta gtaacaatct tcatttgaca ccttaataca    6600
   tttctgactg ttcttaaaac agtggggatg cctatatttt ccttgagttt ttgtgactga    6660
   ctttggctat atataccttt ctggggtttt gtttgttttt tatttttatt ttgattaccg    6720
   agggtatttg gggtggggtt gttttttgag atgtgtaatt cttttatgga gtttttaaaa    6780
   agaattttca tattgttttt ctaacatggc ttcattaaac gtttaagtat tttaaaaata    6840
   tgtaatattt ggaccagatg ttgtgaataa tagtagagat aaagctattt tattctgtat    6900
   ttttaaaact gttccgtaca aataaaagct ctcctggaca cagtaaaaaa aaa           6953
<210> 32
   <211> 6971
   <212> DNA
   <213> Homo sapiens
<400> 32
   cattcatctt ctcctgccct tggacatcac agctccagga actcagatct tcagactcag      60
   ggtgaaggac accaccagct ttccaggttc cccagcttgc agacggcata cgctaggtct     120
   tgactccact aacggggact tctcatacgg ctttgaactt ggaccttggt gcataagcaa     180
   atgtaactca gcgaattgta atggaaagca tatggacagg tctacgccac atcacttagg     240
   tgctaagaga tgccgtttgc aggcctcatc tctgcttgag aatcgggggc tccaggcact     300
   agaagagccg gctgatcctg ggctcctagc ttgaataagc cttcacttcc agctgctctc     360
   cccaacggct gtgtaaacta ctcgtttctc ttaatgatgg gtattggtaa gaataccaca     420
   tccaaatcaa tggaggctgg aagttcaaca gaaggcaaat acgaagacga ggcaaagcac     480
   ccagctttct tcactcttcc ggtggtgata aatggaggcg ccacctccag cggtgagcag     540
   gacaatgagg acactgagct catggcgatc tacactacgg aaaacggcat tgcagaaaag     600
   agctctctcg ctgagaccct ggatagcact ggcagtctgg acccccagcg atcagacatg     660
   atttatacca tagaagatgt tcctccctgg tacctgtgta tatttctggg gctacagcac     720
   tacctgacat gcttcagcgg cacgatcgca gtgcccttcc tgttggccga tgccatgtgt     780
   gtggggtacg accagtgggc caccagccag ctcattggga ccattttctt ctgtgtggga     840
   atcactactt tgctacagac aacgtttgga tgcaggttac ccctgtttca ggccagtgct     900
   tttgcatttt tggcccctgc tcgagccatc ctgtctttag ataaatggaa atgtaacacc     960
   acagatgttt cagttgccaa tggaacagca gagctgttgc acacagaaca catctggtat    1020
   ccccggatcc gagagatcca gggggccatc atcatgtcct cactgataga agtagtcatc    1080
   ggcctcctcg gcctgcctgg ggctctactg aagtacatcg gtcccttgac cattacaccc    1140
   acggtggccc taattggcct ctctggtttc caggcagcgg gggagagagc cgggaagcac 1200
   tggggcattg ccatgctgac aatattccta gtattactgt tttctcaata cgccagaaat 1260
   gttaaatttc ctctcccgat ttataaatcc aagaaaggat ggactgcgta caagttacag 1320
   ctgttcaaaa tgttccctat catcctggcc atcctggtat cctggctgct ctgcttcatc 1380
   ttcacggtga cagatgtctt ccctcccgac agcacaaagt atggcttcta tgctcgcaca 1440
   gatgccaggc aaggcgtgct tctggtagcc ccgtggttta aggttccata cccatttcag 1500
   tggggactgc ccaccgtgtc tgcggccggt gtcatcggca tgctcagtgc cgtggtcgcc 1560
   agcatcatcg agtctattgg tgactactac gcctgtgcac ggctgtcctg tgccccaccc 1620
   ccccccatcc acgcaataaa caggggaatt ttcgtggaag gcctctcctg tgttcttgat 1680
   ggcatatttg gtactgggaa tggctctact tcatccagtc ccaacattgg agttttggga 1740
   attacaaagg tcggcagccg ccgcgtgata cagtgcggag cagccctcat gctcgctctg 1800
   ggcatgatcg ggaagttcag cgccctcttt gcgtcccttc cggatcctgt gctgggagcc 1860
   ctgttctgca cgctctttgg aatgatcaca gctgttggcc tctctaacct gcagttcatt 1920
   gatttaaatt cttcccggaa cctctttgtg cttggatttt cgatcttctt tgggctcgtc 1980
   cttccaagtt acctcagaca gaaccctctg gtcacaggga taacaggaat cgatcaagtg 2040
   ttgaacgtcc ttctcacaac tgctatgttt gtagggggct gtgtggcttt tatcctggat 2100
   aacaccatcc caggcactcc agaggaaaga ggaatccgga aatggaagaa gggtgtgggc 2160
   aaagggaaca aatcactcga cggcatggag tcgtacaatt tgccatttgg catgaacatt 2220
   ataaaaaaat acagatgctt cagctactta cccatcagcc caacctttgt gggctacaca 2280
   tggaaaggcc tcaggaagag cgacaacagc cggagttcag atgaagactc ccaggccacg 2340
   ggatagcctt tgctgtgccc tgtggcctgg ccgcagtgag gcatgtatct gtagttcctt 2400
   gctgagtaac aagaagtaaa atacatgttt gtatatctac atttacattc tgcgccccag 2460
   agctaagaca gactgcgaat cacttttctg ctgtgagggt gatggtgcct gatgcggtgt 2520
   ctcgcctatc tccttattta agcccttaac tccttgccct ttgagagtgt ccattgctgg 2580
   ccatggtcac taaatttgaa gttccagtcc tccctttgga gtggacattt aaaatctgca 2640
   gccattcctt gcttcagttc ccttccacca cccagggctg gctttcctat aagccacctc 2700
   cagggtccag ggcttttctc tctctctccg aagcccctgg acctgtttcc ttgtcatttg 2760
   accctcgtct gtgcccgcgg agacctcctc tgaccaaggg gcactagtat gccttgcgct 2820
   atcatgactg actggcccag tatgattctg ttgtcgctga tctcccggag gaggaggctg 2880
   ttgacctgaa aacagtggcg tggctcccct gtgcccagat ctggccatgg gtgaggcctg 2940
   cacagatgtt gaatgaacat ctgtttgggg cattggaggg aaaaggaaag gaccctgaag 3000
   ccaatatctg attctcggct gcaccttctc ttggccaccc tggccctgac cgggccagca 3060
   cgtgcacact ggcctctttt catttcacta gtggccgaga caccctctct ggcatttatc 3120
   actgttggcc ggtgtgatga gtatagagtg ccacccaagg caccagtcac acaagctgat 3180
   gggcgtcttg gctctgccgt catgtggatg ttattgtcgc gcgcgtctat aattacaagg 3240
   ttttattcct atttaatgtt attgactata gcagattttg gaaatcagtg ttttccatgt 3300
   gaccctcttt ccttgcatcc ctattccttg tcccccaccc actcttcccc ccatcaagaa 3360
   aagaaccagc atttgtaaag ctgtggacac catcagggaa gcttgttgta acggcttttg 3420
   aaggccagta accattgttg tggttgtgtt ttgtattgct tgataccatg aaagtgtaaa 3480
   tactgtaatg cctaatctat ttatcaaaac tgactactgg accggagccc agaaaccatg 3540
   ggtcaagtta cacgtgaatt tgttttgtga agaagggaag ctggggcagg taacacgcag 3600
   agccgccacg tggaacggtc tgtccgccgg tctgtcccgc ttgccgggct tctgttgcaa 3660
   gtcttggctt aaggagactt cctgtgggtt gccatgtcgc acgtccgtta gatcttgatt 3720
   ttacaggtga gggtggttgc caaaggtgat aagaaaagca gccaacaggc tccctctcac 3780
   ttaagagagt tctaattaat aacactaaat tcttcctgag aaatgactcc tcttggtttg 3840
   gtttgaagtt tttcagtgaa ggaaaaggcc tgagatagga agcagtgccc tcgcttcatg 3900
   aggtggcaca tcttcggtgg ccgttggcct ggtcgagctt tggtcagctg cgtgggtgcc 3960
   tgtctcctgt ctccagcggc agggcatggt tcttggcagc ctggtgtcca cacggtgcat 4020
   tcctgtcccc ctcggcccca ggctggctca gctctccagt gctgcggcgc tgtctttgca 4080
   gcattgccat gtcaccgtag cccccttcac tcttggaagt ttggggcttc aaggtttatt 4140
   ctggcaagaa cacctcggag ccactgcatt gttttgccaa aaacttactg cagattaagt 4200
   aaagaactta gatgctaaat tacatctaat tttgtatgtt tttttaaatg tgtctttgat 4260
   gcatacttta gaagcacact ggctcgcggt ttccatccct cttgccttgg gacactcttg 4320
   aggctggcag ccctcctgca atgagttgct ctcagttcct tctgtttgga ttttgctcaa 4380
   catctccagt ttctctgctc tgaaaaatgg ggaaaactgc taaacatccc agagatttgc 4440
   aatcttctag ctctccaggt gagcagaaag accaaatatc caaataaaag cctgaaagcc 4500
   attggtgatc caggggcacc ttctcttcag cttgactggg cgtcttccgc tgcataacac 4560
   tcaaggtgtc tgctctagtc atccagaaac ccactccctt ctgaagcccc tccttgggcc 4620
   gggttgtatc aggaggattc aaccccgcta acatgtgggg tgccctctgt aggccttcac 4680
   acagggaagg aagccctacc ctttcaactc ctgttggagt gagagtttcc tgttctgatt 4740
   ccactgtggc caacccaata attctgtccg aggaggggta agggaatagg aggtggggag 4800
   caaagagggc agcctgacta gatgcccctg ctgaatctgg tactttctgc acagccttga 4860
   gtgattggag tcccctaatg actaataaac ttgaacatgt aacacacaca cacacacaca 4920
   cacacaccat tttaggaagt ggttctccta ctggaaaata tgctacagaa tgaaagtttg 4980
   taattgaaag aaaaagaatg tattcatctg cttcttgggt ttaagcacac acctgaaggg 5040
   gagatattgg gggacacggt ggccactcgc ggtctgtgtc atgacacttc ccattcacat 5100
   cattggcctg cgttgctggg gctttgaacc accaccccgt actttgtttt gcctgtagag 5160
   tgttgtaaaa tattttggat gtgaatgtcc ttgcaggccg gttagtctcc agtttccaca 5220
   gagaccacct acccaccacc ttcccaggtc acctgcctgg tccctgaaag aaagcatttg 5280
   tgtgggcaat cccaacatga aatgtagaca gcgtgaatac ggacaggagg tgatgccata 5340
   ttcacagtgg gggatggagg gcccccaccc ctgcttcaca gaataatggg aagtgactct 5400
   agtctttgta agcaattttc attctcatta ggaagttaaa tgtagattca cagatacatt 5460
   tactttcaga atcctcaaga ggatttcaaa tgctaacttt tagtttcttg ggattgatca 5520
   aggcaagaga agtggcttta accatgccag ccagaatgaa gcatcatggg tcctgatggt 5580
   ttcccgtttt catggtaaca aattcgcaca gaggttttgc tttatgctgt agaataccga 5640
   ctttcatgat gacaacttgg tttatgcatg aatattgcag tatttccata tagcaaaata 5700
   acatttctac ccaggataat agtcacagtt ttccagggag ggaatgatat aattttcatg 5760
   acaatgtcat gtgatgatag aatttctcca ttgatgaatc tctagtatgt gtgtatactt 5820
   tatttaccca tacgtatatt ttatgaccat gagctaagtt gttatttctt tcattcatat 5880
   ccctgctgaa atattggtac tagcaactta aagttggcaa gtctcatctt cagtaatacg 5940
   gatgccatgg gagtggccag gccagattga aggtgaatat ggagcagtag cagaagcctc 6000
   attccagaac ccatctggcc agagaagcag cagcatcctg ggggatggcc gtgcatgggg 6060
   tgtacactcg ctataggcat aggcccggca tggctgtcgc tggacgccag ctgtgcacac 6120
   ccagccacac ctgctgcacg ccgcgttagt gtgcggctcc gggcctgagc attcgcaaag 6180
   ctcgcttctc cagggagcct cctcttggct ttggaaaaga gcccagggac ttaacgtgct 6240
   gcctgttgta ctctgtcccc tcatgacttt ttagacacag ggactaggac atcgaccacc 6300
   ctctgcccct cctgcagtca gaacccgagt agtgcaagaa cggccacccg gttttgccag 6360
   cgttcagcgt ctgcccatgg acccataagg cacattagcg ttgggggtct cttttcagca 6420
   gcctcacaga cattcccgtc ctttctggtt ggtgttgact gtgtttcagc atcactctac 6480
   cctcccatgt ccttagcatt gctgtgcgtg tttccaggca cttaggacac ccttccccac 6540
   gttgccatct tactgacagt tcaaatgact ctcctttttt gttcgttagt aacaatcttc 6600
   atttgacacc ttaatacatt tctgactgtt cttaaaacag tggggatgcc tatattttcc 6660
   ttgagttttt gtgactgact ttggctatat atacctttct ggggttttgt ttgtttttta 6720
   tttttatttt gattaccgag ggtatttggg gtggggttgt tttttgagat gtgtaattct 6780
   tttatggagt ttttaaaaag aattttcata ttgtttttct aacatggctt cattaaacgt 6840
   ttaagtattt taaaaatatg taatatttgg accagatgtt gtgaataata gtagagataa 6900
   agctatttta ttctgtattt ttaaaactgt tccgtacaaa taaaagctct cctggacaca 6960
   gtaaaaaaaa a 6971
<210> 33
   <211> 1593
   <212> DNA
   <213> Homo sapiens
<400> 33
   gcggtgccct tgcggcgcag ctggggtcgc ggccctgctc cccgcgcttt cttaaggccc      60
   gcgggcggcg caggagcggc actcgtggct gtggtggctt cggcagcggc ttcagcagat     120
   cggcggcatc agcggtagca ccagcactag cagcatgttg agccgggcag tgtgcggcac     180
   cagcaggcag ctggctccgg ttttggggta tctgggctcc aggcagaagc acagcctccc     240
   cgacctgccc tacgactacg gcgccctgga acctcacatc aacgcgcaga tcatgcagct     300
   gcaccacagc aagcaccacg cggcctacgt gaacaacctg aacgtcaccg aggagaagta     360
   ccaggaggcg ttggccaagg gagatgttac agcccagata gctcttcagc ctgcactgaa     420
   gttcaatggt ggtggtcata tcaatcatag cattttctgg acaaacctca gccctaacgg     480
   tggtggagaa cccaaagggg agttgctgga agccatcaaa cgtgactttg gttcctttga     540
   caagtttaag gagaagctga cggctgcatc tgttggtgtc caaggctcag gttggggttg     600
   gcttggtttc aataaggaac ggggacactt acaaattgct gcttgtccaa atcaggatcc     660
   actgcaagga acaacaggcc ttattccact gctggggatt gatgtgtggg agcacgctta     720
   ctaccttcag tataaaaatg tcaggcctga ttatctaaaa gctatttgga atgtaatcaa     780
   ctgggagaat gtaactgaaa gatacatggc ttgcaaaaag taaaccacga tcgttatgct     840
   gagtatgtta agctctttat gactgttttt gtagtggtat agagtactgc agaatacagt     900
   aagctgctct attgtagcat ttcttgatgt tgcttagtca cttatttcat aaacaactta     960
   atgttctgaa taatttctta ctaaacattt tgttattggg caagtgattg aaaatagtaa    1020
   atgctttgtg tgattgaatc tgattggaca ttttcttcag agagctaaat tacaattgtc    1080
   atttataaaa ccatcaaaaa tattccatcc atatactttg gggacttgta gggatgcctt     1140
   tctagtccta ttctattgca gttatagaaa atctagtctt ttgccccagt tacttaaaaa    1200
   taaaatatta acactttccc aagggaaaca ctcggctttc tatagaaaat tgcacttttt    1260
   gtcgagtaat cctctgcagt gatacttctg gtagatgtca cccagtggtt tttgttaggt    1320
   caaatgttcc tgtatagttt ttgcaaatag agctgtatac tgtttaaatg tagcaggtga    1380
   actgaactgg ggtttgctca cctgcacagt aaaggcaaac ttcaacagca aaactgcaaa    1440
   aaggtggttt ttgcagtagg agaaaggagg atgtttattt gcagggcgcc aagcaaggag    1500
   aattgggcag ctcatgcttg agacccaatc tccatgatga cctacaagct agagtattta    1560
   aaggcagtgg taaatttcag gaaagcagaa gtt                                 1593
<210> 34
   <211> 1035
   <212> DNA
   <213> Homo sapiens
<400> 34
   gcggtgccct tgcggcgcag ctggggtcgc ggccctgctc cccgcgcttt cttaaggccc 60
   gcgggcggcg caggagcggc actcgtggct gtggtggctt cggcagcggc ttcagcagat 120
   cggcggcatc agcggtagca ccagcactag cagcatgttg agccgggcag tgtgcggcac 180
   cagcaggcag ctggctccgg ttttggggta tctgggctcc aggcagaagc acagcctccc 240
   cgacctgccc tacgactacg gcgccctgga acctcacatc aacgcgcaga tcatgcagct 300
   gcaccacagc aagcaccacg cggcctacgt gaacaacctg aacgtcaccg aggagaagta 360
   ccaggaggcg ttggccaagg gagatgttac agcccagata gctcttcagc ctgcactgaa 420
   gttcaatggt ggtggtcata tcaatcatag cattttctgg acaaacctca gccctaacgg 480
   tggtggagaa cccaaagggg agttgctgga agccatcaaa cgtgactttg gttcctttga 540
   caagtttaag gagaagctga cggctgcatc tgttggtgtc caaggctcag gttggggttg 600
   gcttggtttc aataaggaac ggggacactt acaaattgct gcttgtccaa atcaggatcc 660
   actgcaagga acaacaggcc ttattccact gctggggatt gatgtgtggg agcacgctta 720
   ctaccttcag tataaaaatg tcaggcctga ttatctaaaa gctatttgga atgtaatcaa 780
   ctgggagaat gtaactgaaa gatacatggc ttgcaaaaag taaaccacga tcgttatgct 840
   gatcataccc taatgatccc agcaagataa tgtcctgtct tctaagatgt gcatcaagcc 900
   tggtacatac tgaaaaccct ataaggtcct ggataatttt tgtttgatta ttcattgaag 960
   aaacatttat tttccaattg tgtgaagttt ttgactgtta ataaaagaat ctgtcaacca 1020
   tcaaaaaaaa aaaaa 1035
<210> 35
   <211> 918
   <212> DNA
   <213> Homo sapiens
<400> 35
   gcggtgccct tgcggcgcag ctggggtcgc ggccctgctc cccgcgcttt cttaaggccc 60
   gcgggcggcg caggagcggc actcgtggct gtggtggctt cggcagcggc ttcagcagat 120
   cggcggcatc agcggtagca ccagcactag cagcatgttg agccgggcag tgtgcggcac 180
   cagcaggcag ctggctccgg ttttggggta tctgggctcc aggcagaagc acagcctccc 240
   cgacctgccc tacgactacg gcgccctgga acctcacatc aacgcgcaga tcatgcagct 300
   gcaccacagc aagcaccacg cggcctacgt gaacaacctg aacgtcaccg aggagaagta 360
   ccaggaggcg ttggccaagg gggagttgct ggaagccatc aaacgtgact ttggttcctt 420
   tgacaagttt aaggagaagc tgacggctgc atctgttggt gtccaaggct caggttgggg 480
   ttggcttggt ttcaataagg aacggggaca cttacaaatt gctgcttgtc caaatcagga 540
   tccactgcaa ggaacaacag gccttattcc actgctgggg attgatgtgt gggagcacgc    600
   ttactacctt cagtataaaa atgtcaggcc tgattatcta aaagctattt ggaatgtaat    660
   caactgggag aatgtaactg aaagatacat ggcttgcaaa aagtaaacca cgatcgttat    720
   gctgatcata ccctaatgat cccagcaaga taatgtcctg tcttctaaga tgtgcatcaa    780
   gcctggtaca tactgaaaac cctataaggt cctggataat ttttgtttga ttattcattg    840
   aagaaacatt tattttccaa ttgtgtgaag tttttgactg ttaataaaag aatctgtcaa    900
   ccatcaaaaa aaaaaaaa                                                   918
<210> 36
   <211> 1546
   <212> DNA
   <213> Homo sapiens
<400> 36
   ggggaggtct ggcctgcttt tcctccctga actggcccaa tgactggctc cctcacgctg 60
   accactcctc tgggctggcc tcctgcactc gcgctaacag cccaggctcc agggacagcc 120
   tgcgttcctg ggctggctgg gtgcagctct cttttcagga gagaaagctc tcttggagga 180
   gctggaaagg tgcccgactc cagccatgct ggcgctactg tgttcctgcc tgctcctggc 240
   agccggtgcc tcggacgcct ggacgggcga ggactcggcg gagcccaact ctgactcggc 300
   ggagtggatc cgagacatgt acgccaaggt cacggagatc tggcaggagg tcatgcagcg 360
   gcgggacgac gacggcgcgc tccacgccgc ctgccaggtg cagccgtcgg ccacgctgga 420
   cgccgcgcag ccccgggtga ccggcgtcgt cctcttccgg cagcttgcgc cccgcgccaa 480
   gctcgacgcc ttcttcgccc tggagggctt cccgaccgag ccgaacagct ccagccgcgc 540
   catccacgtg caccagttcg gggacctgag ccagggctgc gagtccaccg ggccccacta 600
   caacccgctg gccgtgccgc acccgcagca cccgggcgac ttcggcaact tcgcggtccg 660
   cgacggcagc ctctggaggt accgcgccgg cctggccgcc tcgctcgcgg gcccgcactc 720
   catcgtgggc cgggccgtgg tcgtccacgc tggcgaggac gacctgggcc gcggcggcaa 780
   ccaggccagc gtggagaacg ggaacgcggg ccggcggctg gcctgctgcg tggtgggcgt 840
   gtgcgggccc gggctctggg agcgccaggc gcgggagcac tcagagcgca agaagcggcg 900
   gcgcgagagc gagtgcaagg ccgcctgagc gcggccccca cccggcggcg gccagggacc 960
   cccgaggccc ccctctgcct ttgagcttct cctctgctcc aacagacacc ctccactctg 1020
   aggtctcacc ttcgcctttg ctgaagtctc cccgcagccc tctccaccca gaggtctccc 1080
   tataccgaga cccaccatcc ttccatcctg aggaccgccc caaccctcgg agccccccac 1140
   tcagtaggtc tgaaggcctc catttgtacc gaaacacccc gctcacgctg acagcctcct 1200
   aggctccctg aggtaccttt ccacccagac cctccttccc caccccataa gccctgagac 1260
   tcccgccttt gacctgacga tcttccccct tcccgccttc aggttcctcc taggcgctca 1320
   gaggccgctc tggggggttg cctcgagtcc ccccacccct ccccacccac caccgctccc 1380
   gcggcaagcc agcccgtgca acggaagcca ggccaactgc cccgcgtctt cagctgtttc 1440
   gcatccaccg ccaccccact gagagctgct cctttggggg aatgtttggc aacctttgtg 1500
   ttacagatta aaaattcagc aattcagtaa aaaaaaaaaa aaaaaa 1546
<210> 37
   <211> 5667
   <212> DNA
   <213> Homo sapiens
<400> 37
   ctcttgctgt ttctttagcc actggtctgc aggcgttttc ttcttctaac ttcctctcct 60
   gtgacaaaag agataactat tagagaaaca aaagtccaga atgctaaggt tgccgctttc 120
   acttcctctc accctttagc ccagaactgc tttgaataca ccaattgctg tggggcggct 180
   cgaggaagag aagacaccag tgcctcagaa actgctcggt cagacggtga tagcgagcca 240
   cgcattcaca gggccactgc tgctcacaga agcagtgagg atgatgccag gatgatgtct 300
   gcctcgcgcc tggctgggac tctgatccca gccatggcct tcctctcctg cgtgagacca 360
   gaaagctggg agccctgcgt ggaggtggtt cctaatatta cttatcaatg catggagctg 420
   aatttctaca aaatccccga caacctcccc ttctcaacca agaacctgga cctgagcttt 480
   aatcccctga ggcatttagg cagctatagc ttcttcagtt tcccagaact gcaggtgctg 540
   gatttatcca ggtgtgaaat ccagacaatt gaagatgggg catatcagag cctaagccac 600
   ctctctacct taatattgac aggaaacccc atccagagtt tagccctggg agccttttct 660
   ggactatcaa gtttacagaa gctggtggct gtggagacaa atctagcatc tctagagaac 720
   ttccccattg gacatctcaa aactttgaaa gaacttaatg tggctcacaa tcttatccaa 780
   tctttcaaat tacctgagta tttttctaat ctgaccaatc tagagcactt ggacctttcc 840
   agcaacaaga ttcaaagtat ttattgcaca gacttgcggg ttctacatca aatgccccta 900
   ctcaatctct ctttagacct gtccctgaac cctatgaact ttatccaacc aggtgcattt 960
   aaagaaatta ggcttcataa gctgacttta agaaataatt ttgatagttt aaatgtaatg 1020
   aaaacttgta ttcaaggtct ggctggttta gaagtccatc gtttggttct gggagaattt 1080
   agaaatgaag gaaacttgga aaagtttgac aaatctgctc tagagggcct gtgcaatttg 1140
   accattgaag aattccgatt agcatactta gactactacc tcgatgatat tattgactta 1200
   tttaattgtt tgacaaatgt ttcttcattt tccctggtga gtgtgactat tgaaagggta 1260
   aaagactttt cttataattt cggatggcaa catttagaat tagttaactg taaatttgga 1320
   cagtttccca cattgaaact caaatctctc aaaaggctta ctttcacttc caacaaaggt 1380
   gggaatgctt tttcagaagt tgatctacca agccttgagt ttctagatct cagtagaaat 1440
   ggcttgagtt tcaaaggttg ctgttctcaa agtgattttg ggacaaccag cctaaagtat 1500
   ttagatctga gcttcaatgg tgttattacc atgagttcaa acttcttggg cttagaacaa 1560
   ctagaacatc tggatttcca gcattccaat ttgaaacaaa tgagtgagtt ttcagtattc 1620
   ctatcactca gaaacctcat ttaccttgac atttctcata ctcacaccag agttgctttc 1680
   aatggcatct tcaatggctt gtccagtctc gaagtcttga aaatggctgg caattctttc 1740
   caggaaaact tccttccaga tatcttcaca gagctgagaa acttgacctt cctggacctc 1800
   tctcagtgtc aactggagca gttgtctcca acagcattta actcactctc cagtcttcag 1860
   gtactaaata tgagccacaa caacttcttt tcattggata cgtttcctta taagtgtctg 1920
   aactccctcc aggttcttga ttacagtctc aatcacataa tgacttccaa aaaacaggaa 1980
   ctacagcatt ttccaagtag tctagctttc ttaaatctta ctcagaatga ctttgcttgt 2040
   acttgtgaac accagagttt cctgcaatgg atcaaggacc agaggcagct cttggtggaa 2100
   gttgaacgaa tggaatgtgc aacaccttca gataagcagg gcatgcctgt gctgagtttg 2160
   aatatcacct gtcagatgaa taagaccatc attggtgtgt cggtcctcag tgtgcttgta 2220
   gtatctgttg tagcagttct ggtctataag ttctattttc acctgatgct tcttgctggc 2280
   tgcataaagt atggtagagg tgaaaacatc tatgatgcct ttgttatcta ctcaagccag 2340
   gatgaggact gggtaaggaa tgagctagta aagaatttag aagaaggggt gcctccattt 2400
   cagctctgcc ttcactacag agactttatt cccggtgtgg ccattgctgc caacatcatc 2460
   catgaaggtt tccataaaag ccgaaaggtg attgttgtgg tgtcccagca cttcatccag 2520
   agccgctggt gtatctttga atatgagatt gctcagacct ggcagtttct gagcagtcgt 2580
   gctggtatca tcttcattgt cctgcagaag gtggagaaga ccctgctcag gcagcaggtg 2640
   gagctgtacc gccttctcag caggaacact tacctggagt gggaggacag tgtcctgggg 2700
   cggcacatct tctggagacg actcagaaaa gccctgctgg atggtaaatc atggaatcca 2760
   gaaggaacag tgggtacagg atgcaattgg caggaagcaa catctatctg aagaggaaaa 2820
   ataaaaacct cctgaggcat ttcttgccca gctgggtcca acacttgttc agttaataag 2880
   tattaaatgc tgccacatgt caggccttat gctaagggtg agtaattcca tggtgcacta 2940
   gatatgcagg gctgctaatc tcaaggagct tccagtgcag agggaataaa tgctagacta 3000
   aaatacagag tcttccaggt gggcatttca accaactcag tcaaggaacc catgacaaag 3060
   aaagtcattt caactcttac ctcatcaagt tgaataaaga cagagaaaac agaaagagac 3120
   attgttcttt tcctgagtct tttgaatgga aattgtatta tgttatagcc atcataaaac 3180
   cattttggta gttttgactg aactgggtgt tcactttttc ctttttgatt gaatacaatt 3240
   taaattctac ttgatgactg cagtcgtcaa ggggctcctg atgcaagatg ccccttccat 3300
   tttaagtctg tctccttaca gaggttaaag tctagtggct aattcctaag gaaacctgat 3360
   taacacatgc tcacaaccat cctggtcatt ctcgagcatg ttctattttt taactaatca 3420
   cccctgatat atttttattt ttatatatcc agttttcatt tttttacgtc ttgcctataa 3480
   gctaatatca taaataaggt tgtttaagac gtgcttcaaa tatccatatt aaccactatt 3540
   tttcaaggaa gtatggaaaa gtacactctg tcactttgtc actcgatgtc attccaaagt 3600
   tattgcctac taagtaatga ctgtcatgaa agcagcattg aaataatttg tttaaagggg 3660
   gcactctttt aaacgggaag aaaatttccg cttcctggtc ttatcatgga caatttgggc 3720
   tagaggcagg aaggaagtgg gatgacctca ggaggtcacc ttttcttgat tccagaaaca 3780
   tatgggctga taaacccggg gtgacctcat gaaatgagtt gcagcagaag tttatttttt 3840
   tcagaacaag tgatgtttga tggacctctg aatctcttta gggagacaca gatggctggg 3900
   atccctcccc tgtacccttc tcactgccag gagaactacg tgtgaaggta ttcaaggcag 3960
   ggagtataca ttgctgtttc ctgttgggca atgctccttg accacatttt gggaagagtg 4020
   gatgttatca ttgagaaaac aatgtgtctg gaattaatgg ggttcttata aagaaggttc 4080
   ccagaaaaga atgttcatcc agcctcctca gaaacagaac attcaagaaa aggacaatca 4140
   ggatgtcatc agggaaatga aaataaaaac cacaatgaga tatcacctta taccaggtag 4200
   aatggctact ataaaaaaat gaagtgtcat caaggatata gagaaattgg aacccttctt 4260
   cactgctgga gggaatggaa aatggtgtag ccgttatgaa aaacagtacg gaggtttctc 4320
   aaaaattaaa aatagaactg ctatatgatc cagcaatctc acttctgtat atatacccaa 4380
   aataattgaa atcagaattt caagaaaata tttacactcc catgttcatt gtggcactct 4440
   tcacaatcac tgtttccaaa gttatggaaa caacccaaat ttccattgaa aaataaatgg 4500
   acaaagaaaa tgtgcatata cgtacaatgg gatattattc agcctaaaaa aagggggaat 4560
   cctgttattt atgacaacat gaataaaccc ggaggccatt atgctatgta aaatgagcaa 4620
   gtaacagaaa gacaaatact gcctgatttc atttatatga ggttctaaaa tagtcaaact 4680
   catagaagca gagaatagaa cagtggttcc tagggaaaag gaggaaggga gaaatgagga 4740
   aatagggagt tgtctaattg gtataaaatt atagtatgca agatgaatta gctctaaaga 4800
   tcagctgtat agcagagttc gtataatgaa caatactgta ttatgcactt aacattttgt 4860
   taagagggta cctctcatgt taagtgttct taccatatac atatacacaa ggaagctttt 4920
   ggaggtgatg gatatattta ttaccttgat tgtggtgatg gtttgacagg tatgtgacta 4980
   tgtctaaact catcaaattg tatacattaa atatatgcag ttttataata tcaattatgt 5040
   ctgaatgaag ctataaaaaa gaaaagacaa caaaattcag ttgtcaaaac tggaaatatg 5100
   accacagtca gaagtgtttg ttactgagtg tttcagagtg tgtttggttt gagcaggtct 5160
   agggtgattg aacatccctg ggtgtgtttc catgtctcat gtactagtga aagtagatgt 5220
   gtgcatttgt gcacatatcc ctatgtatcc ctatcagggc tgtgtgtatt tgaaagtgtg 5280
   tgtgtccgca tgatcatatc tgtatagaag agagtgtgat tatatttctt gaagaataca 5340
   tccatttgaa atggatgtct atggctgttt gagatgagtt ctctactctt gtgcttgtac 5400
   agtagtctcc ccttatccct tatgcttggt ggatacgttc ttagacccca agtggatctc 5460
   tgagaccgca gatggtacca aacctcatat atgcaatatt ttttcctata cataaatacc 5520
   taagataaag ttcatcttct gaattaggca cagtaagaga ttaacaataa ctaacaataa 5580
   aattgaatag ttataataat atattgtaat aaaagttatg tgaatgtgat ctctttcttt 5640
   ctctctctca aaaaaaaaaa aaaaaaa 5667
<210> 38
   <211> 5787
   <212> DNA
   <213> Homo sapiens
<400> 38
   ctcttgctgt ttctttagcc actggtctgc aggcgttttc ttcttctaac ttcctctcct      60
   gtgacaaaag agataactat tagagaaaca aaagtccaga atgctaaggt tgccgctttc     120
   acttcctctc accctttagc ccagaactgc tttgaataca ccaattgctg tggggcggct     180
   cgaggaagag aagacaccag tgcctcagaa actgctcggt cagacggtga tagcgagcca     240
   cgcattcaca gggccactgc tgctcacaga agcagtgagg atgatgccag gatgatgtct     300
   gcctcgcgcc tggctgggac tctgatccca gccatggcct tcctctcctg cgtgagacca     360
   gaaagctggg agccctgcgt ggagacttgg ccctaaacca cacagaagag ctggcatgaa     420
   acccagagct ttcagactcc ggagcctcag cccttcaccc cgattccatt gcttcttgct     480
   aaatgctgcc gttttatcac ggaggtggtt cctaatatta cttatcaatg catggagctg     540
   aatttctaca aaatccccga caacctcccc ttctcaacca agaacctgga cctgagcttt     600
   aatcccctga ggcatttagg cagctatagc ttcttcagtt tcccagaact gcaggtgctg     660
   gatttatcca ggtgtgaaat ccagacaatt gaagatgggg catatcagag cctaagccac     720
   ctctctacct taatattgac aggaaacccc atccagagtt tagccctggg agccttttct     780
   ggactatcaa gtttacagaa gctggtggct gtggagacaa atctagcatc tctagagaac     840
   ttccccattg gacatctcaa aactttgaaa gaacttaatg tggctcacaa tcttatccaa     900
   tctttcaaat tacctgagta tttttctaat ctgaccaatc tagagcactt ggacctttcc     960
   agcaacaaga ttcaaagtat ttattgcaca gacttgcggg ttctacatca aatgccccta    1020
   ctcaatctct ctttagacct gtccctgaac cctatgaact ttatccaacc aggtgcattt    1080
   aaagaaatta ggcttcataa gctgacttta agaaataatt ttgatagttt aaatgtaatg    1140
   aaaacttgta ttcaaggtct ggctggttta gaagtccatc gtttggttct gggagaattt    1200
   agaaatgaag gaaacttgga aaagtttgac aaatctgctc tagagggcct gtgcaatttg    1260
   accattgaag aattccgatt agcatactta gactactacc tcgatgatat tattgactta    1320
   tttaattgtt tgacaaatgt ttcttcattt tccctggtga gtgtgactat tgaaagggta    1380
   aaagactttt cttataattt cggatggcaa catttagaat tagttaactg taaatttgga    1440
   cagtttccca cattgaaact caaatctctc aaaaggctta ctttcacttc caacaaaggt 1500
   gggaatgctt tttcagaagt tgatctacca agccttgagt ttctagatct cagtagaaat 1560
   ggcttgagtt tcaaaggttg ctgttctcaa agtgattttg ggacaaccag cctaaagtat 1620
   ttagatctga gcttcaatgg tgttattacc atgagttcaa acttcttggg cttagaacaa 1680
   ctagaacatc tggatttcca gcattccaat ttgaaacaaa tgagtgagtt ttcagtattc 1740
   ctatcactca gaaacctcat ttaccttgac atttctcata ctcacaccag agttgctttc 1800
   aatggcatct tcaatggctt gtccagtctc gaagtcttga aaatggctgg caattctttc 1860
   caggaaaact tccttccaga tatcttcaca gagctgagaa acttgacctt cctggacctc 1920
   tctcagtgtc aactggagca gttgtctcca acagcattta actcactctc cagtcttcag 1980
   gtactaaata tgagccacaa caacttcttt tcattggata cgtttcctta taagtgtctg 2040
   aactccctcc aggttcttga ttacagtctc aatcacataa tgacttccaa aaaacaggaa 2100
   ctacagcatt ttccaagtag tctagctttc ttaaatctta ctcagaatga ctttgcttgt 2160
   acttgtgaac accagagttt cctgcaatgg atcaaggacc agaggcagct cttggtggaa 2220
   gttgaacgaa tggaatgtgc aacaccttca gataagcagg gcatgcctgt gctgagtttg 2280
   aatatcacct gtcagatgaa taagaccatc attggtgtgt cggtcctcag tgtgcttgta 2340
   gtatctgttg tagcagttct ggtctataag ttctattttc acctgatgct tcttgctggc 2400
   tgcataaagt atggtagagg tgaaaacatc tatgatgcct ttgttatcta ctcaagccag 2460
   gatgaggact gggtaaggaa tgagctagta aagaatttag aagaaggggt gcctccattt 2520
   cagctctgcc ttcactacag agactttatt cccggtgtgg ccattgctgc caacatcatc 2580
   catgaaggtt tccataaaag ccgaaaggtg attgttgtgg tgtcccagca cttcatccag 2640
   agccgctggt gtatctttga atatgagatt gctcagacct ggcagtttct gagcagtcgt 2700
   gctggtatca tcttcattgt cctgcagaag gtggagaaga ccctgctcag gcagcaggtg 2760
   gagctgtacc gccttctcag caggaacact tacctggagt gggaggacag tgtcctgggg 2820
   cggcacatct tctggagacg actcagaaaa gccctgctgg atggtaaatc atggaatcca 2880
   gaaggaacag tgggtacagg atgcaattgg caggaagcaa catctatctg aagaggaaaa 2940
   ataaaaacct cctgaggcat ttcttgccca gctgggtcca acacttgttc agttaataag 3000
   tattaaatgc tgccacatgt caggccttat gctaagggtg agtaattcca tggtgcacta 3060
   gatatgcagg gctgctaatc tcaaggagct tccagtgcag agggaataaa tgctagacta 3120
   aaatacagag tcttccaggt gggcatttca accaactcag tcaaggaacc catgacaaag 3180
   aaagtcattt caactcttac ctcatcaagt tgaataaaga cagagaaaac agaaagagac 3240
   attgttcttt tcctgagtct tttgaatgga aattgtatta tgttatagcc atcataaaac 3300
   cattttggta gttttgactg aactgggtgt tcactttttc ctttttgatt gaatacaatt 3360
   taaattctac ttgatgactg cagtcgtcaa ggggctcctg atgcaagatg ccccttccat     3420
   tttaagtctg tctccttaca gaggttaaag tctagtggct aattcctaag gaaacctgat     3480
   taacacatgc tcacaaccat cctggtcatt ctcgagcatg ttctattttt taactaatca     3540
   cccctgatat atttttattt ttatatatcc agttttcatt tttttacgtc ttgcctataa     3600
   gctaatatca taaataaggt tgtttaagac gtgcttcaaa tatccatatt aaccactatt     3660
   tttcaaggaa gtatggaaaa gtacactctg tcactttgtc actcgatgtc attccaaagt     3720
   tattgcctac taagtaatga ctgtcatgaa agcagcattg aaataatttg tttaaagggg     3780
   gcactctttt aaacgggaag aaaatttccg cttcctggtc ttatcatgga caatttgggc     3840
   tagaggcagg aaggaagtgg gatgacctca ggaggtcacc ttttcttgat tccagaaaca     3900
   tatgggctga taaacccggg gtgacctcat gaaatgagtt gcagcagaag tttatttttt     3960
   tcagaacaag tgatgtttga tggacctctg aatctcttta gggagacaca gatggctggg     4020
   atccctcccc tgtacccttc tcactgccag gagaactacg tgtgaaggta ttcaaggcag     4080
   ggagtataca ttgctgtttc ctgttgggca atgctccttg accacatttt gggaagagtg     4140
   gatgttatca ttgagaaaac aatgtgtctg gaattaatgg ggttcttata aagaaggttc     4200
   ccagaaaaga atgttcatcc agcctcctca gaaacagaac attcaagaaa aggacaatca     4260
   ggatgtcatc agggaaatga aaataaaaac cacaatgaga tatcacctta taccaggtag     4320
   aatggctact ataaaaaaat gaagtgtcat caaggatata gagaaattgg aacccttctt     4380
   cactgctgga gggaatggaa aatggtgtag ccgttatgaa aaacagtacg gaggtttctc     4440
   aaaaattaaa aatagaactg ctatatgatc cagcaatctc acttctgtat atatacccaa     4500
   aataattgaa atcagaattt caagaaaata tttacactcc catgttcatt gtggcactct     4560
   tcacaatcac tgtttccaaa gttatggaaa caacccaaat ttccattgaa aaataaatgg     4620
   acaaagaaaa tgtgcatata cgtacaatgg gatattattc agcctaaaaa aagggggaat     4680
   cctgttattt atgacaacat gaataaaccc ggaggccatt atgctatgta aaatgagcaa     4740
   gtaacagaaa gacaaatact gcctgatttc atttatatga ggttctaaaa tagtcaaact     4800
   catagaagca gagaatagaa cagtggttcc tagggaaaag gaggaaggga gaaatgagga     4860
   aatagggagt tgtctaattg gtataaaatt atagtatgca agatgaatta gctctaaaga     4920
   tcagctgtat agcagagttc gtataatgaa caatactgta ttatgcactt aacattttgt     4980
   taagagggta cctctcatgt taagtgttct taccatatac atatacacaa ggaagctttt     5040
   ggaggtgatg gatatattta ttaccttgat tgtggtgatg gtttgacagg tatgtgacta     5100
   tgtctaaact catcaaattg tatacattaa atatatgcag ttttataata tcaattatgt     5160
   ctgaatgaag ctataaaaaa gaaaagacaa caaaattcag ttgtcaaaac tggaaatatg     5220
   accacagtca gaagtgtttg ttactgagtg tttcagagtg tgtttggttt gagcaggtct     5280
   agggtgattg aacatccctg ggtgtgtttc catgtctcat gtactagtga aagtagatgt     5340
   gtgcatttgt gcacatatcc ctatgtatcc ctatcagggc tgtgtgtatt tgaaagtgtg 5400
   tgtgtccgca tgatcatatc tgtatagaag agagtgtgat tatatttctt gaagaataca 5460
   tccatttgaa atggatgtct atggctgttt gagatgagtt ctctactctt gtgcttgtac 5520
   agtagtctcc ccttatccct tatgcttggt ggatacgttc ttagacccca agtggatctc 5580
   tgagaccgca gatggtacca aacctcatat atgcaatatt ttttcctata cataaatacc 5640
   taagataaag ttcatcttct gaattaggca cagtaagaga ttaacaataa ctaacaataa 5700
   aattgaatag ttataataat atattgtaat aaaagttatg tgaatgtgat ctctttcttt 5760
   ctctctctca aaaaaaaaaa aaaaaaa 5787
<210> 39
   <211> 5500
   <212> DNA
   <213> Homo sapiens
<400> 39
   ctcttgctgt ttctttagcc actggtctgc aggcgttttc ttcttctaac ttcctctcct      60
   gtgacaaaag agataactat tagagaaaca aaagtccaga atgctaaggt tgccgctttc     120
   acttcctctc accctttagc ccagaactgc tttgaataca ccaattgctg tggggcggct     180
   cgaggaagag aagacaccag tgcctcagaa actgctcggt cagacggtga tagcgagcca     240
   cgcattcaca gggccactgc tgctcacaga agcagtgagg atgatgccag gatgatgtct     300
   gcctcgcgcc tggctgggac tctgatccca gccatggcct tcctctcctg cgtgagacca     360
   gaaagctggg agccctgcgt ggaggtgtga aatccagaca attgaagatg gggcatatca     420
   gagcctaagc cacctctcta ccttaatatt gacaggaaac cccatccaga gtttagccct     480
   gggagccttt tctggactat caagtttaca gaagctggtg gctgtggaga caaatctagc     540
   atctctagag aacttcccca ttggacatct caaaactttg aaagaactta atgtggctca     600
   caatcttatc caatctttca aattacctga gtatttttct aatctgacca atctagagca     660
   cttggacctt tccagcaaca agattcaaag tatttattgc acagacttgc gggttctaca     720
   tcaaatgccc ctactcaatc tctctttaga cctgtccctg aaccctatga actttatcca     780
   accaggtgca tttaaagaaa ttaggcttca taagctgact ttaagaaata attttgatag     840
   tttaaatgta atgaaaactt gtattcaagg tctggctggt ttagaagtcc atcgtttggt     900
   tctgggagaa tttagaaatg aaggaaactt ggaaaagttt gacaaatctg ctctagaggg     960
   cctgtgcaat ttgaccattg aagaattccg attagcatac ttagactact acctcgatga    1020
   tattattgac ttatttaatt gtttgacaaa tgtttcttca ttttccctgg tgagtgtgac    1080
   tattgaaagg gtaaaagact tttcttataa tttcggatgg caacatttag aattagttaa    1140
   ctgtaaattt ggacagtttc ccacattgaa actcaaatct ctcaaaaggc ttactttcac    1200
   ttccaacaaa ggtgggaatg ctttttcaga agttgatcta ccaagccttg agtttctaga    1260
   tctcagtaga aatggcttga gtttcaaagg ttgctgttct caaagtgatt ttgggacaac 1320
   cagcctaaag tatttagatc tgagcttcaa tggtgttatt accatgagtt caaacttctt 1380
   gggcttagaa caactagaac atctggattt ccagcattcc aatttgaaac aaatgagtga 1440
   gttttcagta ttcctatcac tcagaaacct catttacctt gacatttctc atactcacac 1500
   cagagttgct ttcaatggca tcttcaatgg cttgtccagt ctcgaagtct tgaaaatggc 1560
   tggcaattct ttccaggaaa acttccttcc agatatcttc acagagctga gaaacttgac 1620
   cttcctggac ctctctcagt gtcaactgga gcagttgtct ccaacagcat ttaactcact 1680
   ctccagtctt caggtactaa atatgagcca caacaacttc ttttcattgg atacgtttcc 1740
   ttataagtgt ctgaactccc tccaggttct tgattacagt ctcaatcaca taatgacttc 1800
   caaaaaacag gaactacagc attttccaag tagtctagct ttcttaaatc ttactcagaa 1860
   tgactttgct tgtacttgtg aacaccagag tttcctgcaa tggatcaagg accagaggca 1920
   gctcttggtg gaagttgaac gaatggaatg tgcaacacct tcagataagc agggcatgcc 1980
   tgtgctgagt ttgaatatca cctgtcagat gaataagacc atcattggtg tgtcggtcct 2040
   cagtgtgctt gtagtatctg ttgtagcagt tctggtctat aagttctatt ttcacctgat 2100
   gcttcttgct ggctgcataa agtatggtag aggtgaaaac atctatgatg cctttgttat 2160
   ctactcaagc caggatgagg actgggtaag gaatgagcta gtaaagaatt tagaagaagg 2220
   ggtgcctcca tttcagctct gccttcacta cagagacttt attcccggtg tggccattgc 2280
   tgccaacatc atccatgaag gtttccataa aagccgaaag gtgattgttg tggtgtccca 2340
   gcacttcatc cagagccgct ggtgtatctt tgaatatgag attgctcaga cctggcagtt 2400
   tctgagcagt cgtgctggta tcatcttcat tgtcctgcag aaggtggaga agaccctgct 2460
   caggcagcag gtggagctgt accgccttct cagcaggaac acttacctgg agtgggagga 2520
   cagtgtcctg gggcggcaca tcttctggag acgactcaga aaagccctgc tggatggtaa     2580
   atcatggaat ccagaaggaa cagtgggtac aggatgcaat tggcaggaag caacatctat 2640
   ctgaagagga aaaataaaaa cctcctgagg catttcttgc ccagctgggt ccaacacttg 2700
   ttcagttaat aagtattaaa tgctgccaca tgtcaggcct tatgctaagg gtgagtaatt 2760
   ccatggtgca ctagatatgc agggctgcta atctcaagga gcttccagtg cagagggaat 2820
   aaatgctaga ctaaaataca gagtcttcca ggtgggcatt tcaaccaact cagtcaagga 2880
   acccatgaca aagaaagtca tttcaactct tacctcatca agttgaataa agacagagaa 2940
   aacagaaaga gacattgttc ttttcctgag tcttttgaat ggaaattgta ttatgttata 3000
   gccatcataa aaccattttg gtagttttga ctgaactggg tgttcacttt ttcctttttg 3060
   attgaataca atttaaattc tacttgatga ctgcagtcgt caaggggctc ctgatgcaag 3120
   atgccccttc cattttaagt ctgtctcctt acagaggtta aagtctagtg gctaattcct 3180
   aaggaaacct gattaacaca tgctcacaac catcctggtc attctcgagc atgttctatt 3240
   ttttaactaa tcacccctga tatattttta tttttatata tccagttttc atttttttac 3300
   gtcttgccta taagctaata tcataaataa ggttgtttaa gacgtgcttc aaatatccat 3360
   attaaccact atttttcaag gaagtatgga aaagtacact ctgtcacttt gtcactcgat 3420
   gtcattccaa agttattgcc tactaagtaa tgactgtcat gaaagcagca ttgaaataat 3480
   ttgtttaaag ggggcactct tttaaacggg aagaaaattt ccgcttcctg gtcttatcat 3540
   ggacaatttg ggctagaggc aggaaggaag tgggatgacc tcaggaggtc accttttctt 3600
   gattccagaa acatatgggc tgataaaccc ggggtgacct catgaaatga gttgcagcag 3660
   aagtttattt ttttcagaac aagtgatgtt tgatggacct ctgaatctct ttagggagac 3720
   acagatggct gggatccctc ccctgtaccc ttctcactgc caggagaact acgtgtgaag 3780
   gtattcaagg cagggagtat acattgctgt ttcctgttgg gcaatgctcc ttgaccacat 3840
   tttgggaaga gtggatgtta tcattgagaa aacaatgtgt ctggaattaa tggggttctt 3900
   ataaagaagg ttcccagaaa agaatgttca tccagcctcc tcagaaacag aacattcaag 3960
   aaaaggacaa tcaggatgtc atcagggaaa tgaaaataaa aaccacaatg agatatcacc 4020
   ttataccagg tagaatggct actataaaaa aatgaagtgt catcaaggat atagagaaat 4080
   tggaaccctt cttcactgct ggagggaatg gaaaatggtg tagccgttat gaaaaacagt 4140
   acggaggttt ctcaaaaatt aaaaatagaa ctgctatatg atccagcaat ctcacttctg 4200
   tatatatacc caaaataatt gaaatcagaa tttcaagaaa atatttacac tcccatgttc 4260
   attgtggcac tcttcacaat cactgtttcc aaagttatgg aaacaaccca aatttccatt 4320
   gaaaaataaa tggacaaaga aaatgtgcat atacgtacaa tgggatatta ttcagcctaa 4380
   aaaaaggggg aatcctgtta tttatgacaa catgaataaa cccggaggcc attatgctat 4440
   gtaaaatgag caagtaacag aaagacaaat actgcctgat ttcatttata tgaggttcta 4500
   aaatagtcaa actcatagaa gcagagaata gaacagtggt tcctagggaa aaggaggaag 4560
   ggagaaatga ggaaataggg agttgtctaa ttggtataaa attatagtat gcaagatgaa 4620
   ttagctctaa agatcagctg tatagcagag ttcgtataat gaacaatact gtattatgca 4680
   cttaacattt tgttaagagg gtacctctca tgttaagtgt tcttaccata tacatataca 4740
   caaggaagct tttggaggtg atggatatat ttattacctt gattgtggtg atggtttgac 4800
   aggtatgtga ctatgtctaa actcatcaaa ttgtatacat taaatatatg cagttttata 4860
   atatcaatta tgtctgaatg aagctataaa aaagaaaaga caacaaaatt cagttgtcaa 4920
   aactggaaat atgaccacag tcagaagtgt ttgttactga gtgtttcaga gtgtgtttgg 4980
   tttgagcagg tctagggtga ttgaacatcc ctgggtgtgt ttccatgtct catgtactag 5040
   tgaaagtaga tgtgtgcatt tgtgcacata tccctatgta tccctatcag ggctgtgtgt 5100
   atttgaaagt gtgtgtgtcc gcatgatcat atctgtatag aagagagtgt gattatattt 5160
   cttgaagaat acatccattt gaaatggatg tctatggctg tttgagatga gttctctact     5220
   cttgtgcttg tacagtagtc tccccttatc ccttatgctt ggtggatacg ttcttagacc     5280
   ccaagtggat ctctgagacc gcagatggta ccaaacctca tatatgcaat attttttcct      5340
   atacataaat acctaagata aagttcatct tctgaattag gcacagtaag agattaacaa     5400
   taactaacaa taaaattgaa tagttataat aatatattgt aataaaagtt atgtgaatgt     5460
   gatctctttc tttctctctc tcaaaaaaaa aaaaaaaaaa                           5500

## Description

The present invention relates gene panels, biomarkers, DNA microarrays, and transcriptional profiling technologies relating to intrinsic and extrinsic aging-based oxidative stress of skin. More particularly, the invention provides methods for identifying and evaluating agents for antioxidant efficacy in skin cells and cosmetic compositions comprising safe and effective amounts of identified agents formulated for application to the skin.

The skin is the largest and most visible organ of the mammalian organism. The signs of aging are therefore most readily apparent in skin and result from a complex process involving various genetic, environmental and hormonal mechanisms. One can differentiate generally between intrinsic, chronological aging and extrinsic, "environmental" aging; both processes occur in conjunction with the effects superimposed on one another. Free radicals play a central role in the course of both intrinsic and extrinsic aging. In chronological aging, free radicals derive from normal human metabolism, while in extrinsic aging; free radicals are produced by environmental insults/stress such as UV-radiation exposure, cigarette smoking, alcohol consumption and air pollution. Indeed, it is estimated that over half of UV radiation-induced damage to skin is attributable to the UV-induced formation of free radicals. The free radicals known as reactive oxygen species (ROS) play a significant role in skin photo-aging and melanogenesis. The "free radical theory of aging" was first proposed by Harman et al. in 1956 and an imbalance between formation and neutralization of free radicals remains the most widely accepted theory to account for the effects of aging on skin.

Oxidative stress is caused by an excess in the production of reactive oxygen species (ROS) in comparison with the ability of the cellular response to detoxify, repair and defend. The cell seeks to maintain a reducing environment which is preserved by enzymes that maintain the desired reduced state through a constant input of metabolic energy. Disturbances in this normal redox state cause toxic effects through production of free radicals including ROS such as peroxides and superoxides. Free radicals are highly reactive molecules with unpaired electrons that can damage various cellular structural membranes, lipids, proteins and nucleic acids. Free radicals also lead to inflammation, which is considered to play an additional role in skin aging. The production of free radicals increases with age, while the integrity and ability of endogenous defense mechanisms to counter them decreases. An imbalance leads to progressive and cumulative damage to cellular structures resulting in aging and accelerated aging where environmental aging is superimposed on the natural aging process.

Antioxidants are substances which provide protection from both endogenous and exogenous oxidative stress. It seems intuitive that topical application of antioxidants to skin may neutralize or detoxify free radicals and ameliorate or prevent some of their aging effects. Cosmetic compositions comprising antioxidants and intended for topical administration to the skin have been used for centuries and formulations have been found in preserved states among relics of ancient cultures. Traditionally, however, the cosmetic industry has focused on the provision of antioxidants that act by free-radical scavenging and neutralizing, such as Vitamins C and E. Cosmetic formulations comprising direct-acting antioxidants suffer from the fact that such agents are naturally unstable and easily oxidized in an oxidizing environment so that they may be rendered inactive by the same environmental insult causing the free radical imbalance sought to be treated. Further, absorption into the skin is difficult to assess and overall ability to evaluate efficacy has been fraught with subjectivity and reliance based generally on presumptions of a nexus between measurement of *in vitro* scavenging potential effectuated scavenging in the skin, and on visual inspection of skin across treatment regimens and on consumer self-report.

Hence, the cosmetic industry is in need of antioxidant agents and cosmetic formulations comprising them which act through mechanisms other than direct scavenging, and is further in need of improved objective methods for evaluating the antioxidant efficacy of proposed agents and cosmetic treatment regimens.

Accordingly, the present invention addresses the deficiencies in the art by providing methods for identifying and objectively evaluating cosmetic agents which are effective for reversing or preventing oxidative stress in skin cells or tissue, or which are effective for restoring the skin to a desirable redox state. Methods are provided which enable identification and evaluation of agents which act to maintain or restore oxidative balance to the skin through multiple internal and external antioxidant mechanisms.

According to the invention a screening method in accordance with claim 1 is provided.

Technologies relating to DNA microarray transcriptional analysis and specific bioinformatic and statistical treatments were employed to discover a unique set of 22 genes, wherein inspection of the transcription profiles of these genes and the expression profiles of the expression products of these genes provides information about redox status of skin and response of skin cells to proposed antioxidant agents.

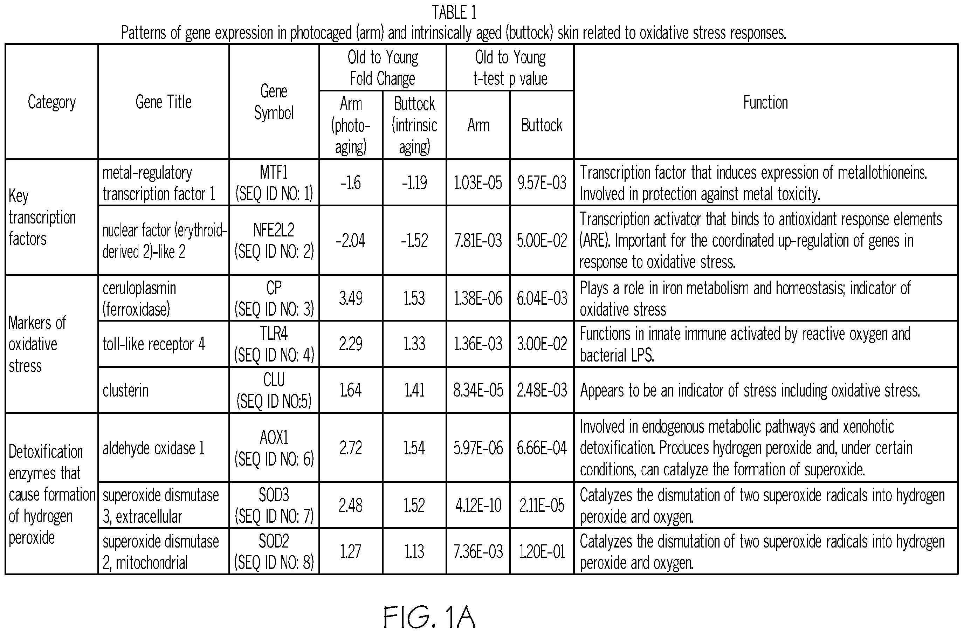

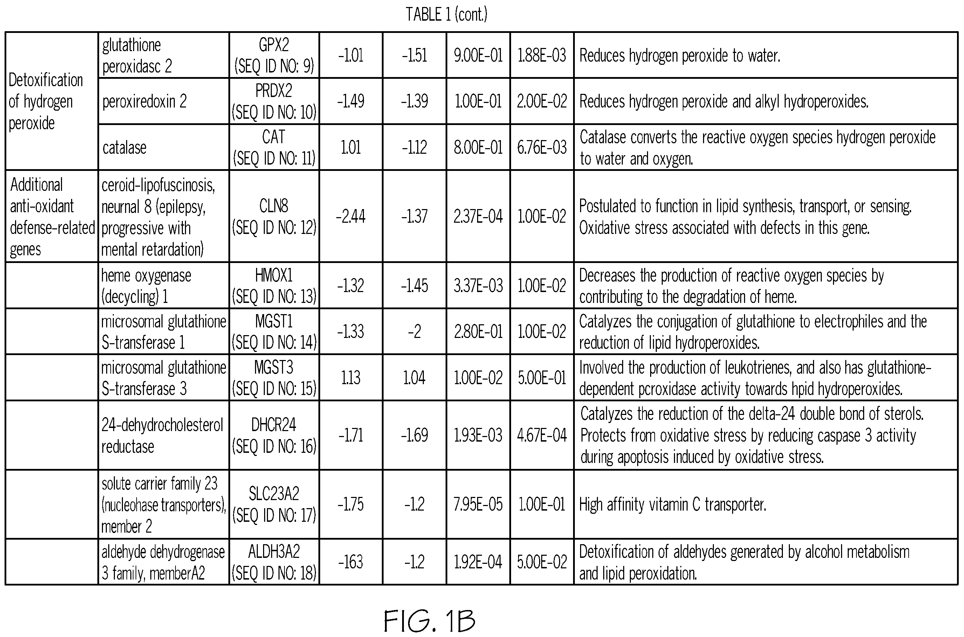

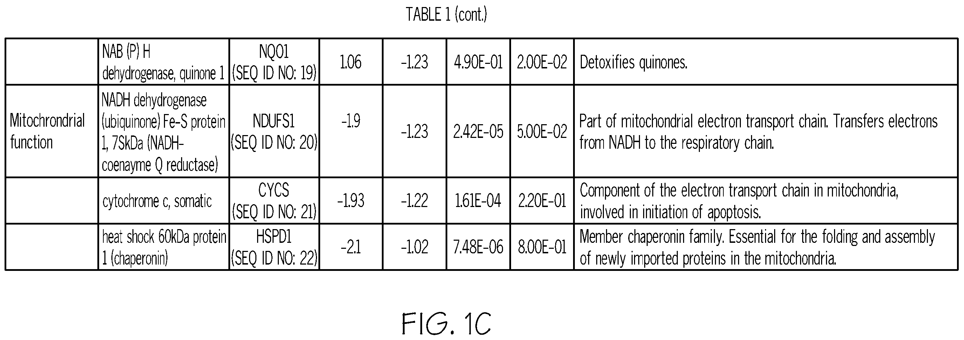

Reference is made to a gene panel comprising genes which are transcriptionally regulated in human skin as a function of oxidative stress, the panel including the genes selected from the group consisting of the genes set forth in Table 1 as **Figure 1****.** Reference is also made to a biomarker panel which comprises expression products of the genes constituting the inventive gene panel. Use may be made of a microarray which comprises immobilized oligonucleotides which hybridize specifically to nucleic acids corresponding to the genes constituting the gene panel. The microarray is particularly useful in effectuating screening methods according to the invention.

Features and benefits of the various embodiments of the present invention will become apparent from the following description, which includes figures and examples of specific embodiments intended to give a broad representation of the invention. Various modifications will be apparent to those skilled in the art from this description and from practice of the invention. The scope is not intended to be limited to the particular forms disclosed and the invention covers all modifications, equivalents and alternatives falling within the spirit and scope of the invention as defined by the claims.

The accompanying Figures, which are incorporated into and constitute part of specification, illustrate specific embodiments of the invention and together with the general and detailed descriptions, serve to explain the principles of aspects of the invention and should not be construed as limiting the scope of the invention as defined by the claims.
- **Figure 1**: A table of the 22 genes identified by DNA microarray transcriptional analysis combined with application of specific bioinformatic and statistical techniques, as being transcriptionally regulated in aging skin as a function of oxidative stress. The table includes gene expression product, gene symbol, known function as it relates to oxidative stress, and array-based fold change data for each identified gene.
- **Figure 2**: Illustrates the cellular detox system which is triggered by presence of oxidizing radicals. In particular, expression of the Phase 2 family of enzymes as up-regulated in response to presence of reactive electrophilic species is illustrated.
- **Figure 3**: Illustrates the general mechanism of Nfr2-mediated activation of the Antioxidant Response Element (ARE).
- **Figure 4**: Illustrates the ARE activation assay and dose-dependent regulation of Nrf2 mediated transcription by Olivem™460 (Olive Oil DFFAP), Signaline™ (Olive Oil BJO) and GFF (yeast ferment filtrate).
- **Figure 5**: Illustrates synergism between GFF and Olivem™460 (Olive Oil DFFAP) with respect to increase in HO-1 as a measure of antioxidant response efficacy.
- **Figure 6**: Illustrates validity of the HO-1 biomarker assay in human skin explants.
- **Figure 7**: HO-1 biomarker assay: Illustrates confirmation of ARE results with ELISA for HO-1, a Phase 2 enzyme, for each agent in Figure 4.
- **Figure 8**: Table of genes not previously predicted or determined to be regulated in aging skin as a function of oxidative stress.
- **Figure 9**: Transcriptional profile data illustrating one embodiment of the inventive methods to evaluate antioxidant profile for cosmetic agents Olivem™ 460 (Olive Oil DFFAP) and GFF (yeast ferment filtrate).
- **Figure 10**: Tables of ARE activation assay results for proposed cosmetic agents GFF (yeast ferment filtrate), Signaline™ (Olive Oil BJO), and Olivem™460 (Olive Oil DFFAP).
- **Figure 11**: Tables of HO-1 ELISA results for proposed cosmetic agents GFF (yeast ferment filtrate, Signaline™(Olive Oil BJO) and Olivem™ 460 (Olive Oil DFFAP).
- **Figure 12**: Sets forth compounds identified as "hits" for potential to provide an antioxidant benefit to the skin by regulating Nrf2-mediated transcription of an antioxidant response element (ARE) in accordance with the inventive methods.

Optimum health of cells, including skin cells, requires maintenance of a delicate balance of oxidants and antioxidants. When this balance is disturbed so that oxidants overwhelm the antioxidant capacity of the cell, significant damage can occur. This damage affects the function of cellular proteins, membrane lipids, and DNA and if unchecked will result in impaired health of the organism. Damaging effects of oxidative stress are seen most readily in skin, oral and respiratory cells.

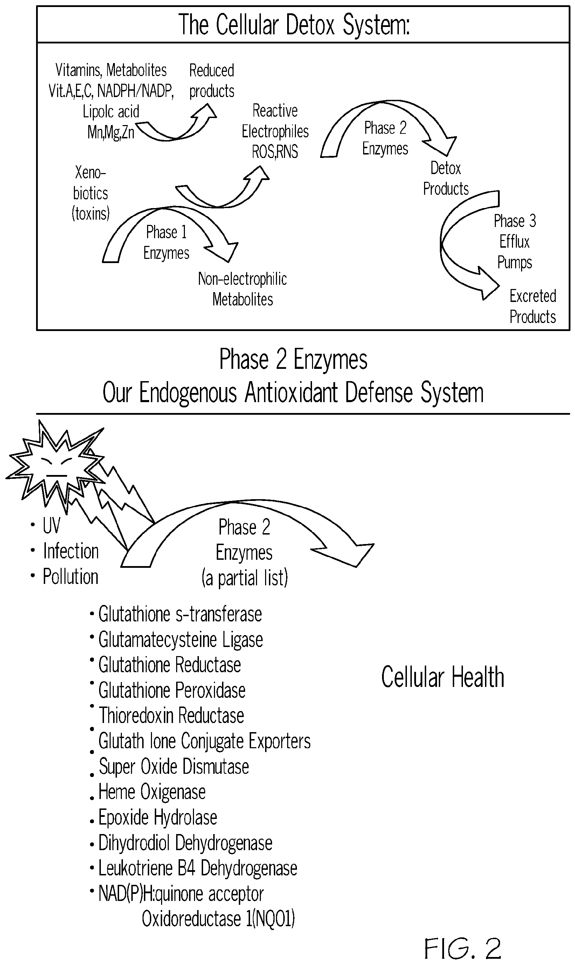

Reactive Oxygen Species (ROS) are particularly destructive free radicals generated in skin cells from environmental insults such as UV radiation skin or from internal processes such as metabolism and inflammation. ROS are a major factor in the process of photo-aging resulting in hyper-pigmented spots, wrinkles, and DNA damage and account for much of the damage seen in and associated with photo-aged skin. The ROS theory of aging was first proposed in the late 1950s and remains the most widely accepted theory of aging today. According to the ROS theory, accumulated environmental and metabolic processes lead to oxidative stress. Oxidative stress induces inflammation characterized by MMP upregulation and breakdown of the dermal matrix, reduction in repair functioning capacity characterized by lowered enzyme function and protein crosslinking. These manifest in skin by decreased collagen content, decreased GAGs and increased elastosis of the skin. The initial phase 1 cellular response to oxidative stress is provided by reducing vitamins such as vitamins A, C and E and metabolites. Upon continuous assault, however, these may be depleted and a second phase cellular response ensues which includes up-regulation and increased expression and secretion of an endogenous system of defensive proteins, the Phase 2 enzymes. A schematic of the Cellular Detox System, including the initial phase 1 response and the heightened Phase 2 response, is set forth as **Figure 2**, which also includes a partial list of the Phase 2 enzymes exhibiting increased expression in response to Nfr2-mediated transcription off the Antioxidant Response Element (ARE), a promoter region linked to the genes which encode the Phase 2 enzymes.

The cosmetic industry provides numerous skin care products aimed at reducing, preventing or reversing the visible signs of aging. With the insights gained from the growth of the sciences of genomics and proteomics, the industry's underpinning focus on improving appearance has shifted from one of the addressing effects of aging to addressing the processes which are reflected by those effects. The present inventors sought to apply genomic technology to cull a focused panel of genes related to oxidative stress and antioxidant defenses that are differentially expressed between young, aged, and photo-aged skin. The resulting panel of 22 genes (Figure 1) provides evidence of oxidative stress in extrinsically and intrinsically aged skin and includes a substantial number which encode for transcriptional factors and enzymes having particular relevance to maintaining an optimal redox balance in skin. The results of this analysis demonstrate a dysregulation of anti-oxidant defenses in aging skin, which will give rise to an increase in levels of hydrogen peroxide, and provide evidence of oxidative stress.

One example therefore provides a gene panel comprising genes which are regulated in human skin as a function of age-related oxidative stress. The panel comprises genes selected from the group consisting of the genes set forth in Table 1, **Figure 1****.** Transcriptional analysis revealed age-related down-regulation of key transcription factors among the 22 tabled genes and gene products that regulate antioxidant protective mechanisms, including NRFT which encodes Nrf2, the DNA binding protein that activates the Antioxidant Response Element (ARE), and MTF1 which encodes Mtf1, a regulator of metallothioneins which are important defense elements against metal toxicity. Accordingly, in a specific embodiment, the regulation comprises transcriptional regulation and the selected genes include one or more (a) NRF2, MTF1, and GPX2, wherein the transcriptional regulation comprises down-regulation; and one or more of (b) CP, CLU, TLR4, SOD3 and AOX1, wherein the transcriptional regulation comprises up-regulation. The oxidative stress may be experienced as a result of either or both chronological age of the skin and/or environmental factors impacting the skin. Well-known environmental factors which cause oxidative stress to skin include exposure of the skin to UV radiation, both UVA and UVB, direct and second hand smoking, consumption of alcohol, and air pollution. In another specific embodiment the oxidative stress derives from H₂O₂ production and the selected genes include: (a) GPX2, PRDX2, and CAT, wherein the regulation comprises transcriptional down-regulation; and (b) SOD3 and AOX1, wherein the regulation comprises transcriptional up-regulation.

The genes set forth in Table 1 each encode a particular protein implicated in age-related oxidative stress. Measurement of expression of the gene products in skin cells or skin tissue also provides information about the redox status of the skin. The gene products form a clinically relevant biomarker panel. The present inventors discovered that genes which encode certain markers for oxidative stress, such as CP (ceruloplasmin) and CLU (clusterin) are substantially up-regulated in aging skin.

Clinical utility of the gene panel includes use of standard molecular biological techniques, such as RT-PCR, and provision of a low density DNA chip designed for high throughput transcriptional profiling of an entire 22 gene panel or some relevant subset thereof. Hence, the invention may use a set of specific oligonucleotide primers for RT-PCR analysis of the gene panel and a microarray comprising immobilized oligonucleotides which hybridize specifically to nucleic acids corresponding to the genes constituting any of the gene panels according to the invention. Production of a low density DNA microarray / chip capable of profiling a given set of genes is well-within the capability of a person of skill in the art using methods known in the art. This panel can also be utilized by screening at the level of protein expression by standard immunochemical methods, such a ELISA, bead-based assays with immobilized antibodies and Western blotting, as well as proteomic analysis with mass spectroscopic detection.

The invention provides a screening method for identifying or evaluating an agent effective for restoring a desired redox balance in skin under oxidative stress, the method comprising providing a reference transcriptional profile or biomarker profile for skin under oxidative stress; contacting test skin, skin cells or a skin equivalent with a proposed agent for a time period; generating a test transcriptional profile or biomarker profile for the test skin; comparing the test profile to the reference profile; determining that a proposed agent is effective for restoring a desired redox balance if the test profile substantially overlays the reference profile. In this case agents which mimic the cellular detoxification and defense/repair response are sought and identified. Cosmetic compositions comprising the identified agents or those evaluated as efficacious may be formulated for topical application to the skin.

Increased expression of the Phase 2 enzymes in response to a continued presence of electrophiles and reactive oxygen species is driven by transcription off the Antioxidant Response Element (ARE) which is common to this family of proteins. The transcriptional up-regulation results in increased numbers of antioxidant-implicated proteins such as catalase, superoxide dismutase (SOD), and heme-oxigenase (HO-1). These enzymes catalyze reduction of oxidative intermediates such as O₂- and H₂O₂ to less harmful end products such as H₂O. In addition to those responsible for metabolizing the damaging toxins, other proteins are up-regulated that actually identify and repair the damage caused by the ROS.

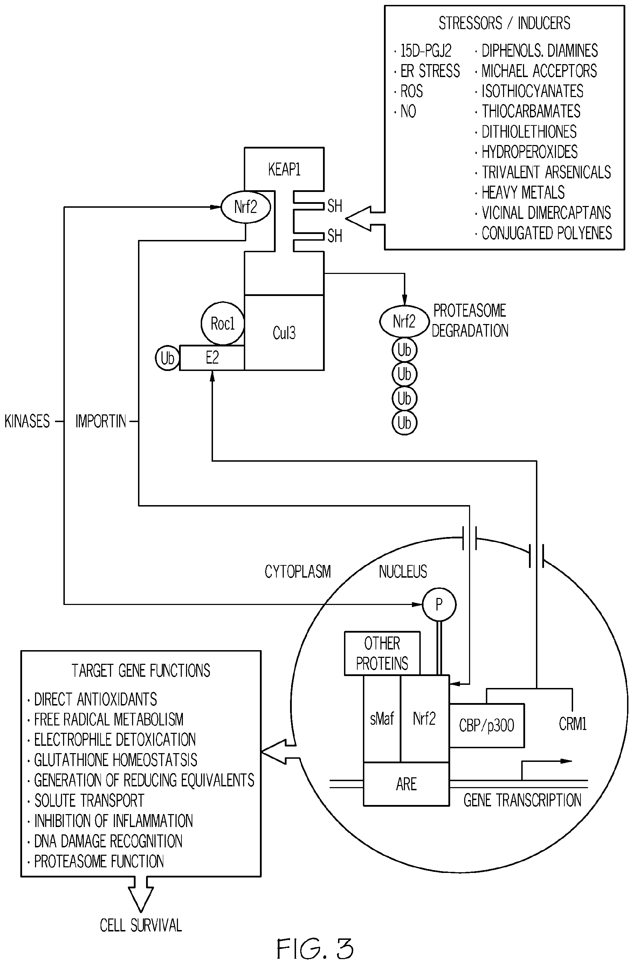

The ARE is a unique cis-acting regulatory element activated by redox-cycling phenols and electrophiles. Recent studies suggest that the DNA-binding protein Nrf2 is the principal transcription factor necessary for ARE activation even though many other transcription factors bind to the ARE sequence. Nuclear factor (erythroid-derived 2)-like 2, also known as NFE2L2 or Nrf2, is a transcription factor which in humans is encoded by the NFR2 gene. Nrf2 is therefore a master regulator of the antioxidant response and oxidative stress is the main driving force for ARE activation. **Figure 3** sets forth a schematic of the Nrf2-mediated ARE activation. Oxidative stress or electrophiles induce oxidation of Keap1 and a conformational change results in a release of Nrf2 from the Nrf2-Keap1 complex resulting in nuclear translocation of Nrf2 through an importin-mediated transport with subsequent activation of multiple AREs and an increase in transcription of the genes linked to the AREs.

Up-regulation of transcription off the ARE results in expression of Phase 2 enzymes which catalyze generation of over 30 proteins that detoxify electrophiles and oxidants into harmless metabolites, build up the store of glutathione, a major antioxidant in the cell, and repair damage by increasing breakdown of damaged proteins and identifying and treating DNA damage. Accordingly, an active identified as an Nrf2 activator may be considered as capable of boosting the cell's own defense mechanisms to provide the tissue with an increased endogenous antioxidant capacity. On the other hand, direct antioxidant agents which work via ROS-scavenging provide a direct but more transient benefit by directly reducing oxidants in the cell and cellular environment.

An ARE reporter cell line may be developed and used to screen for compounds that increase transcription off the ARE in response to oxidative stress, resulting in an increase in cellular antioxidant defense proteins. An ARE32 reporter cell line was adapted to provide an assay for cosmetic agents which may increase transcription off the ARE and increase expression of Phase 2 enzymes, thus augmenting the body's own antioxidant response. ARE32 is a stable reporter cell line produced by CXR Biosciences by transfecting MCF7 cells with pGL8x-ARE (8 copies of the rat GST ARE linked to the luciferase gene) and pCDNA3.1 which contains the neomycin selectable marker. Selection is performed in the presence of G418 and resistant clones are isolated and screened for induction of luciferase. An exemplary standard is tert-butyl hydroquinone (tBHQ), a known activator of Nrf2.

A cosmetic composition may be formulated for topical application to skin to deliver at least one agent that provides an antioxidant benefit to the skin by regulating Nrf2-mediated transcription off an antioxidant response element (ARE). The composition may further comprise at least one additional agent that provides an antioxidant benefit to the skin at least partially through inducing an internal cellular antioxidant response mechanism independent of ARE regulation, wherein efficacy of a combined antioxidant effect is synergistic, and /or may comprise at least one additional agent that provides a direct antioxidant benefit to the skin by direct scavenging of free radicals. Non-limiting examples of agents which are free radical scavengers include vitamins A, E and C.

Non-limiting examples of other skin care actives which may be included in the compositions are amino sugars, vitamin B3, retinoids, peptides, phytosterols, hexamidine, dialkanoyl hydroxyproline compounds, salicylic acid compounds, N-acyl amino acid compounds, sunscreen actives, water-soluble vitamins, oil-soluble vitamins, and dermatologically acceptable carriers.

Hexamidine is understood to include isomers, tautomers, salts and derivatives including but not limited to organic acids such as carboxylic acids and mineral acids such sulfonic acid. A technical name for Hexamidine is 4,4'-(hexamethylenedioxy)dibenzenecarboximidamide. Dermatologically acceptable salts include alkali metal salts such as potassium and sodium; alkaline earth metal salts, such as calcium and magnesium; non-toxic heavy metal salts, and ammonium and trialkylammonium salts. Alternatively, Hexamidine is hexamidine isethionate, which is commercially available under the trade name ELASTAB HP100 from Laboratories Serobiologiques of Pulnoy, France.

The compositions may comprise Hexamidine from about 0.0001% to about 25%, alternatively from about 0.001% to about 10%, alternatively from about 0.01% to about 5%, and alternatively from about 0.02% to about 2.5% by weight of the composition.

Signaline™ from ISP-Vincience is a proprietary ingredient composed mainly of 1,2-diacylglycerol (1,2-DAG) obtained from Olea europaea (olive) oil by enzyme hydrolysis with about 10% of fatty alcohols extracted from Simmondsia chinensis seed (jojoba) wax.

Olivem® is a registered trademark of the B&T Company. The commercially available Olivem 400 series as of the filing date of this application includes Olivem 400, 450 and 460 and all are identified as extremely mild anionic surfactants derived from olive oil fatty acids and are generally characterized by the proprietor as Sodium PEG-7 Olive Oil carboxylates. The Olivem series comprises approximately 81% oleic acid, 8% palmitic acid, 7% linoleic acid, and 2% steric acid. Olivem 400, 450 and 460 and have 36%, 50% and 60% active concentration respectively. Use in cosmetic formulations is generally at 3-10% concentration by weight. Throughout this application where "Olivem" appears it is understood to be Olivem460 unless otherwise indicated. The acronym DFFAP is intended to mean "derived from fatty acid, pegylated." GFF, as used herein, is understood to refer to "galactomyces ferment filtrate", a ferment filtrate of the yeast like fungal genus, Galactomyces.

A screening assay may be used for identifying or evaluating a cosmetic agent as an Nrf2 activator capable of inducing or augmenting an endogenous cellular response to oxidative stress in skin, skin cells, or a skin equivalent, wherein the endogenous cellular response is an up-regulation in transcription off the antioxidant response element (ARE). The assay comprises: providing an ARE reporter cell line wherein the ARE is linked to a reporter gene and transcription off the ARE is mediated by Nrf2 in response to an Nrf2 activator; contacting the ARE reporter cells with a cosmetic agent proposed as an Nrf2 activator; detecting signal generated by transcription of the reporter gene off the ARE; determining that the cosmetic agent is an Nrf2 activator capable of inducing or augmenting the endogenous cellular response if signal is detected. In a specific embodiment the reporter gene is luciferase. The signal may be detected according to any known reporter system. In a particular embodiment the signal is luminescence.

Screening may also include a confirmation step comprising contacting skin, skin cells or a skin equivalent with the proposed Nrf2 activator and measuring a biomarker for Phase 2 enzymatic activity on skin, skin cells or a skin equivalent wherein the biomarker is selected from the group consisting of catalase, superoxide dismutase, heme-oxygenase and catalytic products thereof.

One example of a method of manufacturing a cosmetic composition effective for providing an antioxidant benefit to skin comprises formulating the composition with one or more agents identified in accordance with one or more of the screening assays. For example, the method of manufacture may include formulation with one or more agents identified according to the screening assay for identifying a cosmetic agent as an Nrf2 activator capable of inducing or augmenting an endogenous cellular response to oxidative stress in skin, skin cells, or a skin equivalent, or it may include formulation with one or more agents identified as providing an antioxidant benefit to the skin through transcriptional regulation of genes constituting a gene panel according to the invention. Use of a microarray complementary to a gene panel is also included in certain embodiments. In other methods, the method of manufacture may include formulation with one or more agents identified from some combination of the novel screening assays.

The following Examples are provided to illustrate certain aspects of the instant invention and should not be construed as limiting the scope thereof as defined by the claims.

### Example 1: Generation of the unique panel of 22 genes relating to oxidative stress in intrinsically and photo-aged skin.

A comparison of transcriptional expression in young sun-protected, old sun-protected, young sun-damaged and old sun-damaged skin. Subjects included ten young (ages 18-20) and ten aged (ages 60-67) females. Three 4mm punch biopsies were obtained from each of two skin sites on each subject: sun protected (buttocks "YB" and "OB") and sun-exposed (outer forearm, "YA" and "OA"). The older subjects were selected partly on the basis of having visible moderate to severe photo-damage on the forearm. RNA was extracted and purified from the biopsies. Labeled target cRNA was synthesized and analyzed using Affymetrix® HG-U133 Plus 2.0 microarrays containing 54613 probe sets covering the entire human genome.

Identification of genes related to antioxidant defenses or oxidative stress was according to the following protocol: (1) Functional annotations for genes on the HG-U133 Plus 2.0 GeneChip™ from the Gene Ontology, EntrezGene and Swiss Prot databases were searched using the keyword protocol [oxidative stress OR antioxidant OR free radical OR hydrogen peroxide OR organic peroxide OR superoxide OR reactive oxygen species]. From this a total of 245 unique annotated genes were identified. (2) The array data for the list of 245 oxidative stress-related genes were subjected to the following filtering protocol in order to identify genes regulated in either intrinsic or photo-aging:
- At least 10% present calls across all samples (>=4 present calls)
- t-test p value <0.05 for the Arm OR Buttock Older to Younger comparisons
- Fold change < -1.5 OR >1.5 for the Arm OR Buttock Older to Younger comparisons

A total of 27 genes met the filtering criteria up to this stage. An additional bioinformatics step based on strength of association with both aging and antioxidant activity was conducted which resulted in the elimination of 11 of these genes as not sufficiently correlated, and inclusion of six additional genes (CAT, HMOX1, MGST3, NQO1, PRDX2 and SOD2), which although they failed to meet the fold change criteria set forth above, nonetheless exhibited statistically significant correlations to both aging and oxidative stress.

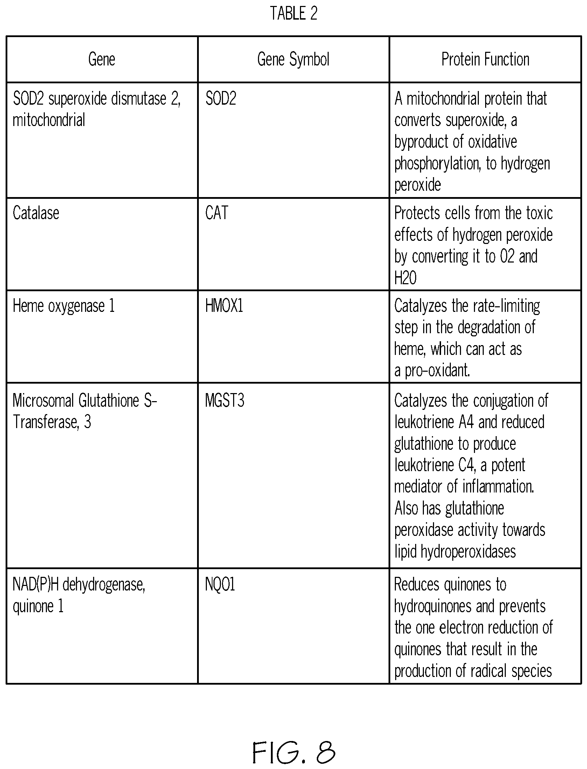

A panel of 22 genes emerged as the end product of the entire filtering protocol and transcriptional expression data for these genes is set forth in Table 1, set forth in **Figure 1****.** As indicated by inspection of the Tabled data set forth as **Figure 1**, the patterns of expression of oxidative stress-related genes in aging skin are substantially similar as between photo-protected and photo-damaged sites with respect to directionality. Key transcription factors that regulate protective mechanisms are down-regulated with aging, indicating a loss in cytoplasmic protective response with age. There is substantial up-regulation of genes related to H₂O₂ production (superoxide dismutase 3 "SOD3" and aldehyde oxidase 1 "AOX1") without concomitant increases in genes which encode enzymes necessary to detoxify H₂O₂ (e.g. catalase and glutathione peroxidase). H₂O₂ in the presence of transition metals can form extremely damaging hydroxy radicals. H₂O₂ can also induce matrix metalloproteinase 2 (MMP2) which was significantly up-regulated in this study. Increased MMP activity may contribute to the loss of dermal matrix associated with aging. This pattern of gene expression may contribute to oxidative stress in aging skin. Also notable is that glutathione peroxidase 2 (GPX2) was significantly down-regulated in intrinsically aged skin. **Figure 8** sets forth a subset of the genes set forth in Table 1 which were found to be transcriptionally regulated in accordance with the instant methods, however which code for proteins which have not been previously identified or suggested as implicated in age-related changes in skin. In particular embodiments, a gene panel according to the invention comprises a select subset of genes which are regulated in human skin as a function of age-related oxidative stress, the panel comprising at least two genes selected from the group consisting of the genes set forth in Table 1, wherein at least one of the selected genes is SOD2, CAT, HMOX1, MGST3 or NQO1.

Increased ceruloplasmin and SOD in view of decreased glutathione peroxidase resembles the pattern of gene expression previously reported for these proteins in the autoimmune disease rheumatoid arthritis. Up-regulation of toll-like receptor 4 (TLR4) suggests activation of the innate immune response in aging skin, possibly associated with oxidative stress. Other ROS-protective genes which demonstrate decreased expression in aging skin include ALDH3A2, DHCR24, MGST1, and SLC23A2.

### Example 2: Application of Transcriptional Profiling Methods; Generation of Transcriptional Profiles for Skin Explants Treated with Olivem™ 460 and GFF

Skin explants were collected from surgical waste (abdominoplasty, 46 year old female) and processed the same day. Processing was done by rinsing the skin sample in cold PBS, the subcutaneous fat was removed with a scalpel, the edges were re-cut, and then 1.3 cm squares were collected. Each square was placed on a transwell filter in a 6 well plate and 80ul of treatment was applied topically. Explants were treated with Olivem™460 (Olive Oil DFFAP) (0.1%) plus GFF (20%) in water, incubated at 33°C at 95% humidity for 3 days then the treatment was removed. The tissue was incubated an additional 3 days and 4 mm punches were taken and quick frozen in liquid N₂ for later RNA isolation and PCR analysis with primers specific for the genes. Control skin was untreated.

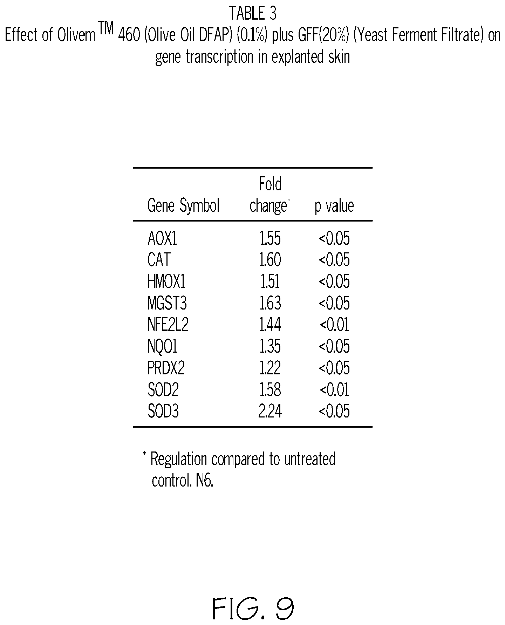

A table of the genes transcriptionally regulated by treatment with a cosmetic composition comprising Olivem™460 (Olive Oil DFFAP) and GFF is set forth as **Figure 9****,** and constitutes a gene panel according to the invention. Notably, up regulation of the expression of AOX1, SOD2 and SOD3, which are detoxifying enzymes that produce hydrogen peroxide, along with CAT and PRDX2, which detoxify hydrogen peroxide, a balanced and beneficial antioxidant response, is observed. This contrasts to the pattern in aged skin, where AOX1, SOD2 and SOD3 are up-regulated, but CAT and PRDX2, along with GPX2, are down-regulated.

### Example 3: Application of ARE Activation Assay and hemi-oxygenasel ELISA to assess antioxidant activity of proposed agents.

The antioxidant properties of Olive Oil DFAP, Signaline and GFF are demonstrated by results derived from the ARE transcription assay and an ELISA biomarker assay wherein the biomarker is a phase 2 enzyme, HO-1. Results are also set forth for the heme-oxygenase 1 (HO-1) biomarker test using human skin explant cultures.

Primary cultures of human skin keratinocytes and fibroblasts and ex vivo human skin cultures (explants) were treated for 24 hours with specific olive oil derivatives (Olive Oil-Derived Fatty Acids modified with PEG-7 "Olive Oil DFAP"), olive oil blended with jojoba oil (Signaline™) and Galactomyces Ferment Filtrate (GFF). ARE was assayed using the ARE-32 reporter cell line available from CXR-Biosciences and set forth in detail, below:

### ARE assay

ARE32 is a stable MCF7 cell line containing pGL8x-ARE (8 copies of the rat GST ARE linked to the luciferase gene) and pCDNA3.1 which contains the neomycin selectable marker, which was licensed from CXRbiosciences. Selection was performed in the presence of G418 and resistant clones were isolated. Clones were screened for induction of luciferase in response to tBHQ.

### Reagents and instruments

- Dulbecco's Modified Eagle Medium (DMEM) (Gibco, Cat #11054-020, lot#1361242)
- Fetal Bovine Serum Heat Inactivated (FBS) ((Gibco, Cat # 16140-063, lot#1345764)
- Geneticin G418 sulphate (G418) (Gibco, Cat # 11811-031)
- Steady Glo System (Promega, Cat # E2510)
- 96-well plate (Costar, Cat # 3917)
- Penicillin-Streptomycin (Gibco, Cat # 15070-063, Lot # 109733)
- Tert-Butylhydroquinone (tBHQ) (Aldrich, Cat # 11,294-1)
- Perkin Elmer Envision reader

### Maintenance of AREC32 cells

ARE32 cells are maintained routinely in the DMEM (phenol red free) containing:
10% FBS
50 units/ml penicillin & 50 µg/ml streptomycin
0.8 mg/ml G418
Cells are subcultured every 3-4 days. Cells were frozen in medium contains 90%
FBS and 10% DMSO.

### Induction of luciferase with tBHQ

1. In a 96 well-plate, seed 1x104 cells/well in 100 µl DMEM containing 50 units/ml penicillin, 50 µg/ml streptomycin, 0.8 mg/ml G418 and 10% FBS.
2. Incubate the cells at 37°C in a 5% CO2 incubator for 24 hrs.
3. Replace the medium with 100 µl fresh media and treat with test compounds 2ul per well, positive control was 25uM TBHQ. (10 mM tBHQ of stock solution was freshly prepared in DMSO)
4. Add 100ul of media after treatment, final assay volume 200uL.
5. Incubate the cells at 37°C in CO2 incubator for another 24 hrs.
6. Assay for luciferase activity with Steady-glo Assay System by following the manufacture's instruction:
   a. Remove 100uL of media
   b. Add 100ul of reconstituted substrate directly to well
   c. Incubate 5-10 min shaking.
   d. Read on Envision with Promega Luciferase assay protocol. (half life of steady-glo is 5 hours)
   e. Read plate for 5 seconds per well.

Cell type and tissue confirmation of ARE were confirmed via an HO-1 ELISA normalized to total protein per sample. Statistical analysis was conducted using the Student's t-test, comparing treated to control samples. Error bars indicate the Standard Deviation.

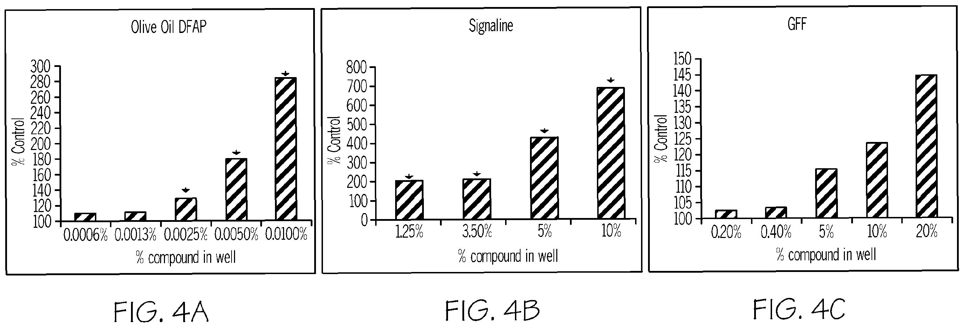

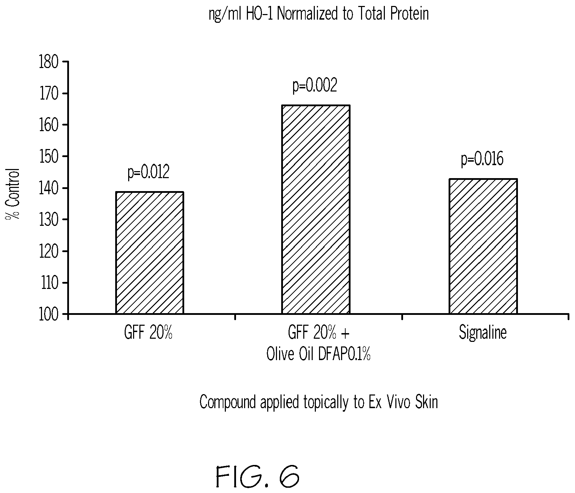

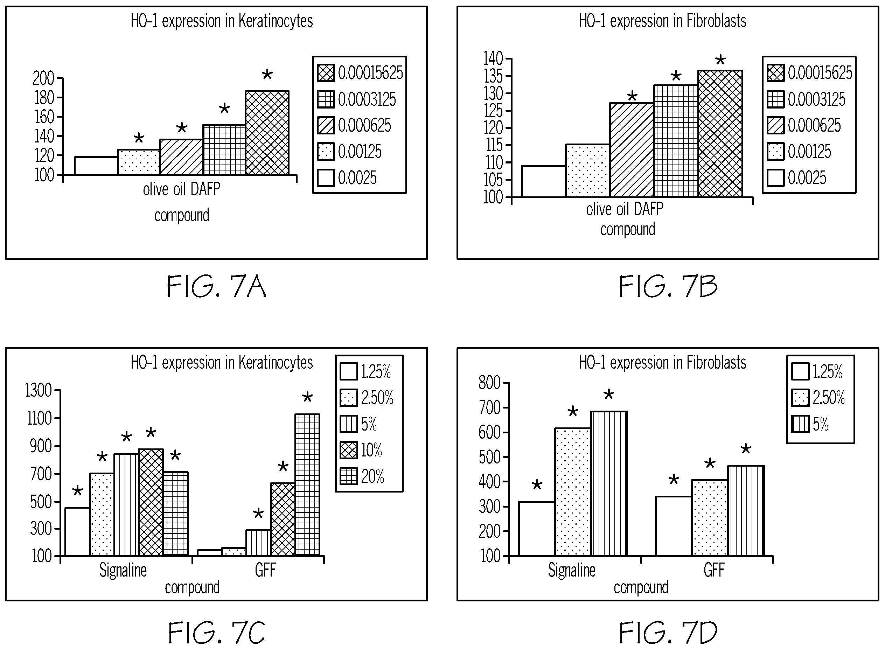

A general schematic for how the ARE reporter assay operates to identify agents that promote transcription off the ARE is set forth in **Figure 3****.** The ARE assay results for Olivem™460 (Olive Oil DFFAP), Signaline and GFF are set forth in **Figure 4****.** All three agents produced significant dose-dependent up-regulation of transcription off the ARE. Results for Olivem™460 (Olive Oil DFFAP) triggered expression of HO-1 in Keratinocytes are set forth in **Figure 7A**, results for Olivem™460 (Olive Oil DFFAP) in Fibroblasts are set forth in **Figure 7B****,** while results for Signaline and GFF are set forth in **Figure 7C** and **7D**. All three agents produced significant dose-dependent increases in HO-1 (biomarker of cellular antioxidant capacity) in human skin keratinocytes and fibroblasts. **Figure 9** sets forth the HO-1 data for human skin explants. Signaline, GFF and a GFF/ Olivem™460 (Olive Oil DFFAP) combination applied topically to human skin ex vivo produced a significant increased expression of HO-1. The combination of GFF and Signaline in human skin keratinocytes yielded a synergistic increase in HO-1 expression, as set forth in **Figure 6**. Close inspection of the results reveals that transcription off the ARE was less affected by GFF than would be expected by the HO-1 biomarker result. This suggests that an alternative pathway, such as HIF-1 (hypoxia inducible factor1, transcription factor which responds to conditions of low oxygen), may be involved. Further, human skin keratinocytes treated with a level of Olivem™460 (Olive Oil DFFAP) too low to affect transcription off ARE nonetheless behaved synergistically with GFF to increase HO-1 over what would be expected with GFF alone, indicating a synergistic anti-oxidant effect as well as support for independent pathways.

### Example 4: Application of ARE Activation Assay and hemi-oxygenasel ELISA to assess antioxidant activity of additional proposed agents: Olivem™ 460 (Olive Oil DFFAP), Signaline™(Olive Oil BJO) and GFF (Yeast Ferment Filtrate).

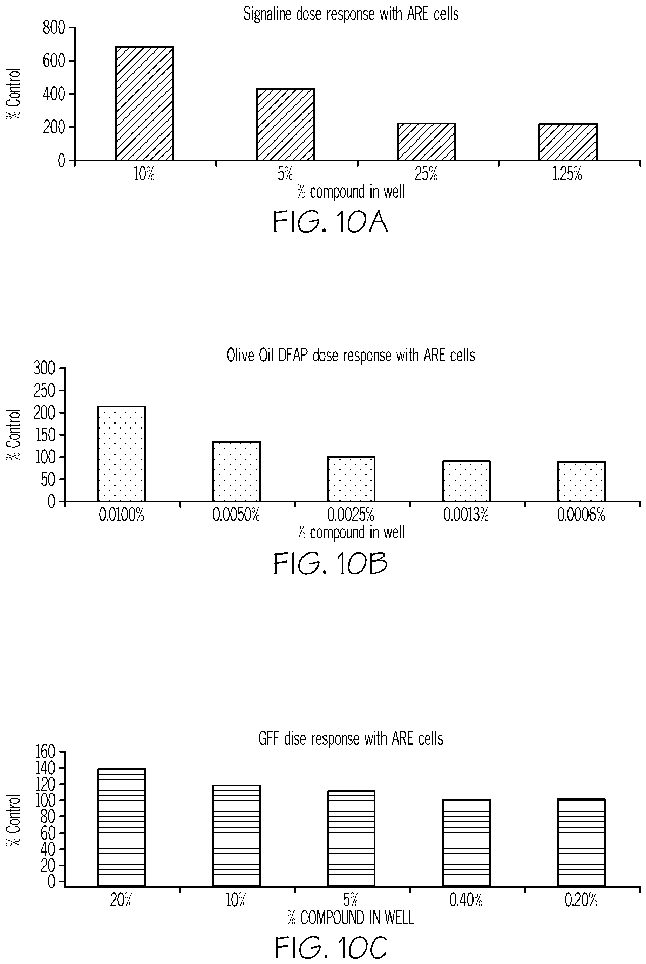

The ARE activation assay described in Example 3 is employed. To test for transcription off the antioxidant response element, the reporter line is grown, compounds are added, each compound in varying doses, and allowed to incubate for 24 hours. Cells are then lysed and luminescent output is detected and quantified. A dose-response output for Signaline is set forth in **Figure 10A**. Inspection reveals that up-regulation is dose-dependent. A dose-response output for Olivem™460 (Olive Oil DFFAP) is set forth in **Figure 10B**. Inspection reveals that up-regulation is dose-dependent. A dose-response output for GFF is set forth in **Figure 10C****.** Inspection reveals that up-regulation by GFF is not significant at any dose.

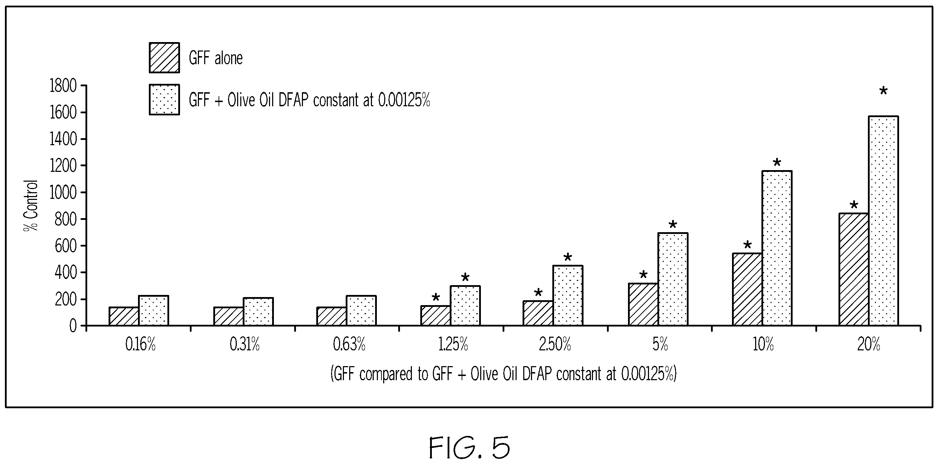

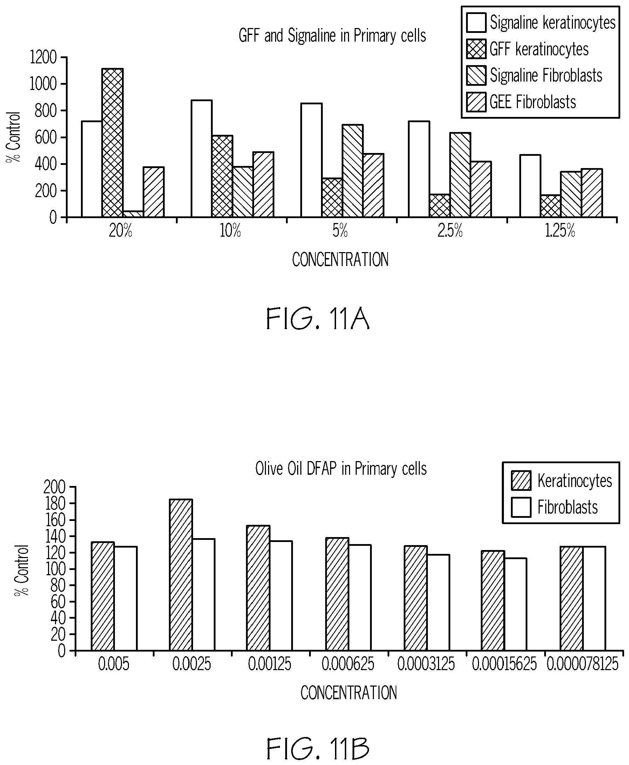

The heme-oxygenase 1 (HO-1) ELISA described in Example is also employed for each agent in both keratinocytes and fibroblasts in order to confirm activation of transcription off the ARE and to further explore possible antioxidant mechanisms. Hemi-oxygenasel (HO-1) is a Phase 2 enzyme and a commercial ELISA was selected to quantify its production in the cells. As set forth in **Figure 11A****,** both GFF and Signaline demonstrate a dose-dependent response in keratinocytes but not in fibroblasts. **Figure 11B** indicates that Olivem™460 (Olive Oil DFFAP) exhibits dose-dependent up-regulation in both keratinocytes and fibroblasts. The highest dose is thought to be toxic, more so to fibroblasts than keratinocytes, which accounts for the apparently inconsistent result at the highest concentration. As set forth in **Figure 5**, when Olivem™460 (Olive Oil DFFAP) in a dose that did not result in an up-regulation of HO-1 is combined with GFF, an unpredicted synergistic increase in HO-1 expression results. Signaline and GFF, alone or in combination with Olivem™460 (Olive Oil DFFAP), up-regulate HO-1 expression in human skin explants.

### Example 5: Illustrates an embodiment of the invention whereby proposed compounds are screened for potential efficacy in regulating the nrf2-mediated triggering of the ARE.

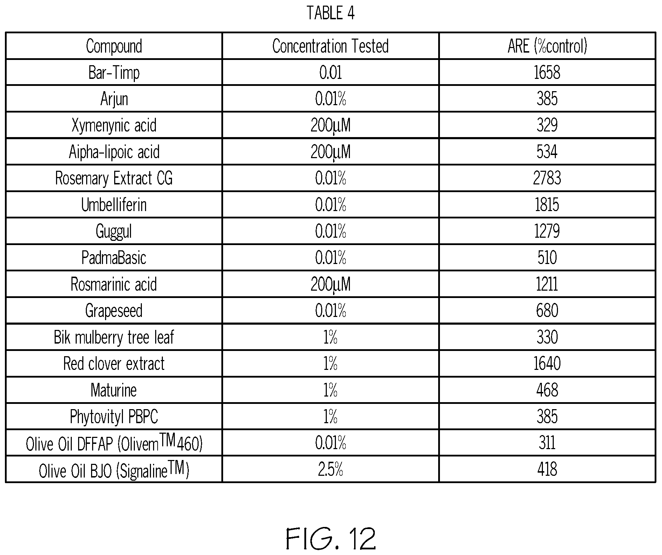

Proposed compounds are screened using the ARE assay set forth in Example 3, above. **Figure 12** sets forth a Table of "ARE screening hits" along with concentrations tested for each compounds and the observed percent increase in ARE. Generally, compounds that up-regulate transcription off the ARE will increase the amount of protective Phase 2 Enzymes in the cell providing increased antioxidant capacity.

### SEQUENCE IDENTIFICATION INFORMATION

SEQ ID NO:1 Homo sapiens aldehyde dehydrogenase 3 family, member A2 (ALDH3A2), transcript variant 2
SEQ ID NO:2 Homo sapiens aldehyde dehydrogenase 3 family, member A2 (ALDH3A2), transcript variant 1
SEQ ID NO:3 Homo sapiens aldehyde oxidase **1** (AOX1)
SEQ ID NO:4 Homo sapiens catalase (CAT)
SEQ ID NO:5 Homo sapiens ceroid-lipofuscinosis, neuronal 8 (epilepsy, progressive with mental retardation) (CLN8)
SEQ ID NO:6 Homo sapiens clusterin (CLU), transcript variant **1**
SEQ ID NO:7 Homo sapiens clusterin (CLU), transcript variant 2
SEQ ID NO:8 Homo sapiens ceruloplasmin (ferroxidase) (CP)
SEQ ID NO:9 Homo sapiens cytochrome c, somatic (CYCS), nuclear gene encoding mitochondrial protein
SEQ ID NO:10 Homo sapiens 24-dehydrocholesterol reductase (DHCR24)
SEQ ID NO:11 Homo sapiens glutathione peroxidase 2 (gastrointestinal) (GPX2)
SEQ ID NO:12 Homo sapiens heme oxygenase (decycling) 1 (HMOX1)
SEQ ID NO:13 Homo sapiens heat shock 60kDa protein 1 (chaperonin) (HSPD1), nuclear gene encoding mitochondrial protein, transcript variant **1**
SEQ ID NO:14 Homo sapiens heat shock 60kDa protein **1** (chaperonin) (HSPD**1**), nuclear gene encoding mitochondrial protein, transcript variant 2
SEQ ID NO:15 Homo sapiens heat shock 60kDa protein **1** (chaperonin) (HSPD**1**), nuclear gene encoding mitochondrial protein, transcript variant 2
SEQ ID NO:16 Homo sapiens microsomal glutathione S-transferase 1 (MGST**1**), transcript variant **1**a
SEQ ID NO:17 Homo sapiens microsomal glutathione S-transferase 1 (MGST**1**), transcript variant **1**b
SEQ ID NO:18 Homo sapiens microsomal glutathione S-transferase 1 (MGST**1**), transcript variant **1**c
SEQ ID NO:19 Homo sapiens microsomal glutathione S-transferase 1 (MGST**1**), transcript variant **1**d
SEQ ID NO:20 Homo sapiens microsomal glutathione S-transferase 3 (MGST3)
SEQ ID NO:21 Homo sapiens metal-regulatory transcription factor 1 (MTF1)
SEQ ID NO:22 Homo sapiens NADH dehydrogenase (ubiquinone) Fe-S protein **1**, 75kDa (NADH-coenzyme Q reductase) (NDUFS1), nuclear gene encoding mitochondrial protein
SEQ ID NO:23 Homo sapiens nuclear factor (erythroid-derived 2)-like 2 (NFE2L2), transcript variant 1
SEQ ID NO:24 Homo sapiens nuclear factor (erythroid-derived 2)-like 2 (NFE2L2), transcript variant 2
SEQ ID NO:25 Homo sapiens nuclear factor (erythroid-derived 2)-like 2 (NFE2L2), transcript variant 3
SEQ ID NO:26 Homo sapiens NAD(P)H dehydrogenase, quinone **1** (NQO1), transcript variant **1**
SEQ ID NO:27 Homo sapiens NAD(P)H dehydrogenase, quinone **1** (NQO1), transcript variant 2
SEQ ID NO:28 Homo sapiens NAD(P)H dehydrogenase, quinone **1** (NQO1), transcript variant 3
SEQ ID NO:29 Homo sapiens peroxiredoxin 2 (PRDX2), nuclear gene encoding mitochondrial protein, transcript variant **1**
SEQ ID NO:30 Homo sapiens peroxiredoxin 2 (PRDX2), nuclear gene encoding mitochondrial protein, transcript variant 3
SEQ ID NO:31 Homo sapiens solute carrier family 23 (nucleobase transporters),member 2 (SLC23A2), transcript variant 1
SEQ ID NO:32 Homo sapiens solute carrier family 23 (nucleobase transporters), member 2 (SLC23A2),transcript variant 2
SEQ ID NO:33 Homo sapiens superoxide dismutase 2, mitochondrial (SOD2), nuclear gene encoding mitochondrial protein, transcript variant 1
SEQ ID NO:34 Homo sapiens superoxide dismutase 2, mitochondrial (SOD2), nuclear gene encoding mitochondrial protein, transcript variant 2
SEQ ID NO:35 Homo sapiens superoxide dismutase 2, mitochondrial (SOD2), nuclear gene encoding mitochondrial protein, transcript variant 3
SEQ ID NO:36 Homo sapiens superoxide dismutase 3, extracellular (SOD3)
SEQ ID NO:37 Homo sapiens toll-like receptor 4 (TLR4), transcript variant 1
SEQ ID NO:38 Homo sapiens toll-like receptor 4 (TLR4), transcript variant 3, non-coding
SEQ ID NO:39 Homo sapiens toll-like receptor 4 (TLR4), transcript variant 4

## Claims

1. A screening method for identifying or evaluating an agent effective for restoring a desired redox balance in skin under oxidative stress, the method comprising providing a reference transcriptional profile or biomarker profile for skin under oxidative stress, wherein the reference transcriptional profile includes the genes in Table 1 and the biomarker profile includes a gene product from each of the genes in Table 1; contacting test skin, skin cells or a skin equivalent with a proposed agent for a time period; generating a test transcriptional profile or biomarker profile for the test skin; comparing the test profile to the reference profile; and determining that a proposed agent is effective for restoring a desired redox balance if the test profile substantially mimics the reference profile.

## Patentansprüche

1. Screening-Verfahren zum Identifizieren oder Bewerten eines Mittels, das zum Wiederherstellen eines gewünschten Redox-Gleichgewichts in Haut unter oxidativem Stress wirksam ist, wobei das Verfahren Folgendes umfasst:
Bereitstellen eines Referenztranskriptionsprofils oder Biomarkerprofils für Haut unter oxidativem Stress, wobei das Referenztranskriptionsprofil die Gene in Tabelle 1 einschließt und das Biomarkerprofil ein Genprodukt von jedem der Gene in Tabelle 1 einschließt; Inkontaktbringen von Testhaut, Hautzellen oder einem Hautäquivalent mit einem vorgeschlagenen Mittel für einen Zeitraum; Erzeugen eines Testtranskriptionsprofils oder Biomarkerprofils für die Testhaut; Vergleichen des Testprofils mit dem Referenzprofil; und Bestimmen, dass ein vorgeschlagenes Mittel zum Wiederherstellen eines gewünschten Redox-Gleichgewichts wirksam ist, wenn das Testprofil dem Referenzprofil im Wesentlichen gleichkommt.

## Revendications

1. Procédé de criblage pour l'identification ou l'évaluation d'un agent efficace pour restaurer un équilibre redox souhaité dans une peau sous stress oxydatif, le procédé comprenant la fourniture d'un profil transcriptionnel de référence ou d'un profil de biomarqueur pour une peau sous stress oxydatif, dans lequel le profil transcriptionnel de référence inclut les gènes dans le Tableau 1 et le profil de biomarqueur inclut un produit génique provenant de chacun des gènes dans le Tableau 1 ; la mise en contact de peau de test, de cellules de peau ou d'un équivalent de peau avec un agent proposé pendant une période de temps ; la génération d'un profil transcriptionnel ou profil de biomarqueur de test pour la peau de test ; la comparaison du profil de test au profil de référence ; et le fait de déterminer qu'un agent proposé est efficace pour restaurer un équilibre redox souhaité si le profil de test reproduit essentiellement le profil de référence.
